# EUROPEAN PATENT APPLICATION

(11) **EP 3 476 860 A1**
(43) Date of publication of application: **01.05.2019**
(21) Application number: 17815743.4
(22) Date of filing: 23.06.2017
(51) Int. Cl.: C07K 14/755, C07K 1/113, C07K 19/00, C12P 21/00

(54) **RECOMBINANT SINGLE-CHAIN FVIII AND CHEMICAL CONJUGATE THEREOF**

(30) Priority: 24.06.2016 KR 20160079442
(71) Applicant: Mogam Institute for Biomedical Research, Gyeonggi-do 16924 (KR); Green Cross Corporation, Yongin-si, Gyeonggi-do 16924 (KR)
(72) Inventor: KANG, Kwan-Yub, Yongin-si Gyeonggi-do 16924 (KR); LEE, Seung-Hoon, Yongin-si Gyeonggi-do 16924 (KR); OH, Injae, Yongin-si Gyeonggi-do 16924 (KR); OH, Mee Sook, Yongin-si Gyeonggi-do 16924 (KR); RYU, Jae Hwan, Yongin-si Gyeonggi-do 16924 (KR); JO, Eui-Cheol, Yongin-si Gyeonggi-do 16924 (KR); LEE, Ki-Nam, Yongin-si Gyeonggi-do 16924 (KR); YANG, Sun-Ah, Yongin-si Gyeonggi-do 16924 (KR)
(74) Representative: dompatent von Kreisler Selting Werner - Partnerschaft von Patent- und Rechtsanwälten mbB
(86) International application number: PCT/KR2017/006633
(87) International publication number: WO 2017/222330

(57) **Abstract**

The present application provides a single-chain blood coagulation factor VIII including a B region which is partially deleted to contain at least four glycosylation sites, but not a heavy chain, a light-chain, and a region to be cleaved by proteases, or a single-chain blood coagulation factor VIII in which the A or B region has some residues pegylated. A single-chain blood coagulation factor VIII according to the present application not only retains intact therapeutic efficacy and can be easily produced on mass scale because of its single chain form, but also has an increased half-life in vivo through pegylation, thus enhancing convenience for patients as a therapeutic agent for hemophilia A and bringing about a reduction of medical expenses through reduction in production cost.

<110>    Mogam Institute for Biomedical Research

<120>    Recombinant single chain Factor VIII and chemical conjugates
         thereof

<130>    PCT201705003

<150>    KR 2016-0079442
<151>    2016-06-24

<160>    48

<170>    KopatentIn 2.0

<210>    1
<211>    2332
<212>    PRT
<213>    Homo sapiens

<400>    1
Ala Thr Arg Arg Tyr Tyr Leu Gly Ala Val Glu Leu Ser Trp Asp Tyr
  1               5                  10                  15

Met Gln Ser Asp Leu Gly Glu Leu Pro Val Asp Ala Arg Phe Pro Pro
             20                  25                  30

Arg Val Pro Lys Ser Phe Pro Phe Asn Thr Ser Val Val Tyr Lys Lys
         35                  40                  45

Thr Leu Phe Val Glu Phe Thr Asp His Leu Phe Asn Ile Ala Lys Pro
     50                  55                  60

Arg Pro Pro Trp Met Gly Leu Leu Gly Pro Thr Ile Gln Ala Glu Val
 65                  70                  75                  80

Tyr Asp Thr Val Val Ile Thr Leu Lys Asn Met Ala Ser His Pro Val
                 85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg

225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe

565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His

            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Ser Thr Glu Ser Gly Arg Leu Phe Lys Gly Lys
                965                 970                 975

Arg Ala His Gly Pro Ala Leu Leu Thr Lys Asp Asn Ala Leu Phe Lys
            980                 985                 990

Val Ser Ile Ser Leu Leu Lys Thr Asn Lys Thr Ser Asn Asn Ser Ala
        995                 1000                1005

Thr Asn Arg Lys Thr His Ile Asp Gly Pro Ser Leu Leu Ile Glu Asn
    1010                1015                1020

Ser Pro Ser Val Trp Gln Asn Ile Leu Glu Ser Asp Thr Glu Phe Lys
1025                1030                1035                1040

Lys Val Thr Pro Leu Ile His Asp Arg Met Leu Met Asp Lys Asn Ala
                1045                1050                1055

Thr Ala Leu Arg Leu Asn His Met Ser Asn Lys Thr Thr Ser Ser Lys
            1060                1065                1070

Asn Met Glu Met Val Gln Gln Lys Lys Glu Gly Pro Ile Pro Pro Asp
        1075                1080                1085

Ala Gln Asn Pro Asp Met Ser Phe Phe Lys Met Leu Phe Leu Pro Glu
    1090                1095                1100

Ser Ala Arg Trp Ile Gln Arg Thr His Gly Lys Asn Ser Leu Asn Ser
1105                1110                1115                1120

Gly Gln Gly Pro Ser Pro Lys Gln Leu Val Ser Leu Gly Pro Glu Lys
                1125                1130                1135

Ser Val Glu Gly Gln Asn Phe Leu Ser Glu Lys Asn Lys Val Val Val
            1140                1145                1150

Gly Lys Gly Glu Phe Thr Lys Asp Val Gly Leu Lys Glu Met Val Phe
        1155                1160                1165

Pro Ser Ser Arg Asn Leu Phe Leu Thr Asn Leu Asp Asn Leu His Glu
    1170                1175                1180

Asn Asn Thr His Asn Gln Glu Lys Lys Ile Gln Glu Glu Ile Glu Lys
1185                1190                1195                1200

Lys Glu Thr Leu Ile Gln Glu Asn Val Val Leu Pro Gln Ile His Thr
                1205                1210                1215

Val Thr Gly Thr Lys Asn Phe Met Lys Asn Leu Phe Leu Leu Ser Thr
            1220                1225                1230

Arg Gln Asn Val Glu Gly Ser Tyr Asp Gly Ala Tyr Ala Pro Val Leu

1235                1240                1245

Gln Asp Phe Arg Ser Leu Asn Asp Ser Thr Asn Arg Thr Lys Lys His
    1250                1255                1260

Thr Ala His Phe Ser Lys Lys Gly Glu Glu Glu Asn Leu Glu Gly Leu
1265                1270                1275                1280

Gly Asn Gln Thr Lys Gln Ile Val Glu Lys Tyr Ala Cys Thr Thr Arg
                1285                1290                1295

Ile Ser Pro Asn Thr Ser Gln Gln Asn Phe Val Thr Gln Arg Ser Lys
            1300                1305                1310

Arg Ala Leu Lys Gln Phe Arg Leu Pro Leu Glu Glu Thr Glu Leu Glu
        1315                1320                1325

Lys Arg Ile Ile Val Asp Asp Thr Ser Thr Gln Trp Ser Lys Asn Met
    1330                1335                1340

Lys His Leu Thr Pro Ser Thr Leu Thr Gln Ile Asp Tyr Asn Glu Lys
1345                1350                1355                1360

Glu Lys Gly Ala Ile Thr Gln Ser Pro Leu Ser Asp Cys Leu Thr Arg
                1365                1370                1375

Ser His Ser Ile Pro Gln Ala Asn Arg Ser Pro Leu Pro Ile Ala Lys
            1380                1385                1390

Val Ser Ser Phe Pro Ser Ile Arg Pro Ile Tyr Leu Thr Arg Val Leu
        1395                1400                1405

Phe Gln Asp Asn Ser Ser His Leu Pro Ala Ala Ser Tyr Arg Lys Lys
    1410                1415                1420

Asp Ser Gly Val Gln Glu Ser Ser His Phe Leu Gln Gly Ala Lys Lys
1425                1430                1435                1440

Asn Asn Leu Ser Leu Ala Ile Leu Thr Leu Glu Met Thr Gly Asp Gln
                1445                1450                1455

Arg Glu Val Gly Ser Leu Gly Thr Ser Ala Thr Asn Ser Val Thr Tyr
            1460                1465                1470

Lys Lys Val Glu Asn Thr Val Leu Pro Lys Pro Asp Leu Pro Lys Thr
        1475                1480                1485

Ser Gly Lys Val Glu Leu Leu Pro Lys Val His Ile Tyr Gln Lys Asp
    1490                1495                1500

Leu Phe Pro Thr Glu Thr Ser Asn Gly Ser Pro Gly His Leu Asp Leu
1505                1510                1515                1520

Val Glu Gly Ser Leu Leu Gln Gly Thr Glu Gly Ala Ile Lys Trp Asn
                1525                1530                1535

Glu Ala Asn Arg Pro Gly Lys Val Pro Phe Leu Arg Val Ala Thr Glu
            1540                1545                1550

Ser Ser Ala Lys Thr Pro Ser Lys Leu Leu Asp Pro Leu Ala Trp Asp
        1555                1560                1565

Asn His Tyr Gly Thr Gln Ile Pro Lys Glu Glu Trp Lys Ser Gln Glu

1570                1575                1580

Lys Ser Pro Glu Lys Thr Ala Phe Lys Lys Lys Asp Thr Ile Leu Ser
1585                1590                1595                1600

Leu Asn Ala Cys Glu Ser Asn His Ala Ile Ala Ala Ile Asn Glu Gly
                1605                1610                1615

Gln Asn Lys Pro Glu Ile Glu Val Thr Trp Ala Lys Gln Gly Arg Thr
            1620                1625                1630

Glu Arg Leu Cys Ser Gln Asn Pro Pro Val Leu Lys Arg His Gln Arg
        1635                1640                1645

Glu Ile Thr Arg Thr Thr Leu Gln Ser Asp Gln Glu Glu Ile Asp Tyr
    1650                1655                1660

Asp Asp Thr Ile Ser Val Glu Met Lys Lys Glu Asp Phe Asp Ile Tyr
1665                1670                1675                1680

Asp Glu Asp Glu Asn Gln Ser Pro Arg Ser Phe Gln Lys Lys Thr Arg
                1685                1690                1695

His Tyr Phe Ile Ala Ala Val Glu Arg Leu Trp Asp Tyr Gly Met Ser
            1700                1705                1710

Ser Ser Pro His Val Leu Arg Asn Arg Ala Gln Ser Gly Ser Val Pro
        1715                1720                1725

Gln Phe Lys Lys Val Val Phe Gln Glu Phe Thr Asp Gly Ser Phe Thr
    1730                1735                1740

Gln Pro Leu Tyr Arg Gly Glu Leu Asn Glu His Leu Gly Leu Leu Gly
1745                1750                1755                1760

Pro Tyr Ile Arg Ala Glu Val Glu Asp Asn Ile Met Val Thr Phe Arg
                1765                1770                1775

Asn Gln Ala Ser Arg Pro Tyr Ser Phe Tyr Ser Ser Leu Ile Ser Tyr
            1780                1785                1790

Glu Glu Asp Gln Arg Gln Gly Ala Glu Pro Arg Lys Asn Phe Val Lys
        1795                1800                1805

Pro Asn Glu Thr Lys Thr Tyr Phe Trp Lys Val Gln His His Met Ala
    1810                1815                1820

Pro Thr Lys Asp Glu Phe Asp Cys Lys Ala Trp Ala Tyr Phe Ser Asp
1825                1830                1835                1840

Val Asp Leu Glu Lys Asp Val His Ser Gly Leu Ile Gly Pro Leu Leu
                1845                1850                1855

Val Cys His Thr Asn Thr Leu Asn Pro Ala His Gly Arg Gln Val Thr
            1860                1865                1870

Val Gln Glu Phe Ala Leu Phe Phe Thr Ile Phe Asp Glu Thr Lys Ser
        1875                1880                1885

Trp Tyr Phe Thr Glu Asn Met Glu Arg Asn Cys Arg Ala Pro Cys Asn
    1890                1895                1900

Ile Gln Met Glu Asp Pro Thr Phe Lys Glu Asn Tyr Arg Phe His Ala

1905                1910                1915                1920

Ile Asn Gly Tyr Ile Met Asp Thr Leu Pro Gly Leu Val Met Ala Gln
                1925                1930                1935

Asp Gln Arg Ile Arg Trp Tyr Leu Leu Ser Met Gly Ser Asn Glu Asn
            1940                1945                1950

Ile His Ser Ile His Phe Ser Gly His Val Phe Thr Val Arg Lys Lys
        1955                1960                1965

Glu Glu Tyr Lys Met Ala Leu Tyr Asn Leu Tyr Pro Gly Val Phe Glu
    1970                1975                1980

Thr Val Glu Met Leu Pro Ser Lys Ala Gly Ile Trp Arg Val Glu Cys
1985                1990                1995                2000

Leu Ile Gly Glu His Leu His Ala Gly Met Ser Thr Leu Phe Leu Val
                2005                2010                2015

Tyr Ser Asn Lys Cys Gln Thr Pro Leu Gly Met Ala Ser Gly His Ile
            2020                2025                2030

Arg Asp Phe Gln Ile Thr Ala Ser Gly Gln Tyr Gly Gln Trp Ala Pro
        2035                2040                2045

Lys Leu Ala Arg Leu His Tyr Ser Gly Ser Ile Asn Ala Trp Ser Thr
    2050                2055                2060

Lys Glu Pro Phe Ser Trp Ile Lys Val Asp Leu Leu Ala Pro Met Ile
2065                2070                2075                2080

Ile His Gly Ile Lys Thr Gln Gly Ala Arg Gln Lys Phe Ser Ser Leu
                2085                2090                2095

Tyr Ile Ser Gln Phe Ile Ile Met Tyr Ser Leu Asp Gly Lys Lys Trp
            2100                2105                2110

Gln Thr Tyr Arg Gly Asn Ser Thr Gly Thr Leu Met Val Phe Phe Gly
        2115                2120                2125

Asn Val Asp Ser Ser Gly Ile Lys His Asn Ile Phe Asn Pro Pro Ile
    2130                2135                2140

Ile Ala Arg Tyr Ile Arg Leu His Pro Thr His Tyr Ser Ile Arg Ser
2145                2150                2155                2160

Thr Leu Arg Met Glu Leu Met Gly Cys Asp Leu Asn Ser Cys Ser Met
                2165                2170                2175

Pro Leu Gly Met Glu Ser Lys Ala Ile Ser Asp Ala Gln Ile Thr Ala
            2180                2185                2190

Ser Ser Tyr Phe Thr Asn Met Phe Ala Thr Trp Ser Pro Ser Lys Ala
        2195                2200                2205

Arg Leu His Leu Gln Gly Arg Ser Asn Ala Trp Arg Pro Gln Val Asn
    2210                2215                2220

Asn Pro Lys Glu Trp Leu Gln Val Asp Phe Gln Lys Thr Met Lys Val
2225                2230                2235                2240

Thr Gly Val Thr Thr Gln Gly Val Lys Ser Leu Leu Thr Ser Met Tyr

2245                2250                2255

Val Lys Glu Phe Leu Ile Ser Ser Ser Gln Asp Gly His Gln Trp Thr
            2260                2265                2270

Leu Phe Phe Gln Asn Gly Lys Val Lys Val Phe Gln Gly Asn Gln Asp
        2275                2280                2285

Ser Phe Thr Pro Val Val Asn Ser Leu Asp Pro Pro Leu Leu Thr Arg
    2290                2295                2300

Tyr Leu Arg Ile His Pro Gln Ser Trp Val His Gln Ile Ala Leu Arg
2305                2310                2315                2320

Met Glu Val Leu Gly Cys Glu Ala Gln Asp Leu Tyr
                2325                2330

<210>   2
<211>   1467
<212>   PRT
<213>   Artificial Sequence

<220>
<223>   G2 (scF2)

<400>   2
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Thr Leu Gln Ser Asp Gln Glu Glu Ile Asp Tyr Asp
785                 790                 795                 800

Asp Thr Ile Ser Val Glu Met Lys Lys Glu Asp Phe Asp Ile Tyr Asp
                805                 810                 815

Glu Asp Glu Asn Gln Ser Pro Arg Ser Phe Gln Lys Lys Thr Arg His
            820                 825                 830

Tyr Phe Ile Ala Ala Val Glu Arg Leu Trp Asp Tyr Gly Met Ser Ser
        835                 840                 845

Ser Pro His Val Leu Arg Asn Arg Ala Gln Ser Gly Ser Val Pro Gln
    850                 855                 860

Phe Lys Lys Val Val Phe Gln Glu Phe Thr Asp Gly Ser Phe Thr Gln
865                 870                 875                 880

Pro Leu Tyr Arg Gly Glu Leu Asn Glu His Leu Gly Leu Leu Gly Pro
                885                 890                 895

Tyr Ile Arg Ala Glu Val Glu Asp Asn Ile Met Val Thr Phe Arg Asn
            900                 905                 910

Gln Ala Ser Arg Pro Tyr Ser Phe Tyr Ser Ser Leu Ile Ser Tyr Glu
        915                 920                 925

Glu Asp Gln Arg Gln Gly Ala Glu Pro Arg Lys Asn Phe Val Lys Pro
    930                 935                 940

Asn Glu Thr Lys Thr Tyr Phe Trp Lys Val Gln His His Met Ala Pro
945                 950                 955                 960

Thr Lys Asp Glu Phe Asp Cys Lys Ala Trp Ala Tyr Phe Ser Asp Val
                965                 970                 975

Asp Leu Glu Lys Asp Val His Ser Gly Leu Ile Gly Pro Leu Leu Val
            980                 985                 990

Cys His Thr Asn Thr Leu Asn Pro Ala His Gly Arg Gln Val Thr Val
        995                 1000                1005

Gln Glu Phe Ala Leu Phe Phe Thr Ile Phe Asp Glu Thr Lys Ser Trp
    1010                1015                1020

Tyr Phe Thr Glu Asn Met Glu Arg Asn Cys Arg Ala Pro Cys Asn Ile
1025                1030                1035                1040

Gln Met Glu Asp Pro Thr Phe Lys Glu Asn Tyr Arg Phe His Ala Ile
                1045                1050                1055

Asn Gly Tyr Ile Met Asp Thr Leu Pro Gly Leu Val Met Ala Gln Asp
            1060                1065                1070

Gln Arg Ile Arg Trp Tyr Leu Leu Ser Met Gly Ser Asn Glu Asn Ile
        1075                1080                1085

His Ser Ile His Phe Ser Gly His Val Phe Thr Val Arg Lys Lys Glu
    1090                1095                1100

Glu Tyr Lys Met Ala Leu Tyr Asn Leu Tyr Pro Gly Val Phe Glu Thr
1105                1110                1115                1120

Val Glu Met Leu Pro Ser Lys Ala Gly Ile Trp Arg Val Glu Cys Leu
                1125                1130                1135

Ile Gly Glu His Leu His Ala Gly Met Ser Thr Leu Phe Leu Val Tyr
            1140                1145                1150

Ser Asn Lys Cys Gln Thr Pro Leu Gly Met Ala Ser Gly His Ile Arg
        1155                1160                1165

Asp Phe Gln Ile Thr Ala Ser Gly Gln Tyr Gly Gln Trp Ala Pro Lys
    1170                1175                1180

Leu Ala Arg Leu His Tyr Ser Gly Ser Ile Asn Ala Trp Ser Thr Lys
1185                1190                1195                1200

Glu Pro Phe Ser Trp Ile Lys Val Asp Leu Leu Ala Pro Met Ile Ile
                1205                1210                1215

His Gly Ile Lys Thr Gln Gly Ala Arg Gln Lys Phe Ser Ser Leu Tyr
            1220                1225                1230

Ile Ser Gln Phe Ile Ile Met Tyr Ser Leu Asp Gly Lys Lys Trp Gln
        1235                1240                1245

Thr Tyr Arg Gly Asn Ser Thr Gly Thr Leu Met Val Phe Phe Gly Asn
    1250                1255                1260

Val Asp Ser Ser Gly Ile Lys His Asn Ile Phe Asn Pro Pro Ile Ile
1265                1270                1275                1280

Ala Arg Tyr Ile Arg Leu His Pro Thr His Tyr Ser Ile Arg Ser Thr
                1285                1290                1295

Leu Arg Met Glu Leu Met Gly Cys Asp Leu Asn Ser Cys Ser Met Pro
            1300                1305                1310

Leu Gly Met Glu Ser Lys Ala Ile Ser Asp Ala Gln Ile Thr Ala Ser
        1315                1320                1325

Ser Tyr Phe Thr Asn Met Phe Ala Thr Trp Ser Pro Ser Lys Ala Arg
    1330                1335                1340

Leu His Leu Gln Gly Arg Ser Asn Ala Trp Arg Pro Gln Val Asn Asn
1345                1350                1355                1360

Pro Lys Glu Trp Leu Gln Val Asp Phe Gln Lys Thr Met Lys Val Thr
                1365                1370                1375

Gly Val Thr Thr Gln Gly Val Lys Ser Leu Leu Thr Ser Met Tyr Val
            1380                1385                1390

Lys Glu Phe Leu Ile Ser Ser Ser Gln Asp Gly His Gln Trp Thr Leu
        1395                1400                1405

Phe Phe Gln Asn Gly Lys Val Lys Val Phe Gln Gly Asn Gln Asp Ser
    1410                1415                1420

Phe Thr Pro Val Val Asn Ser Leu Asp Pro Pro Leu Leu Thr Arg Tyr
1425                1430                1435                1440

Leu Arg Ile His Pro Gln Ser Trp Val His Gln Ile Ala Leu Arg Met
                1445                1450                1455

Glu Val Leu Gly Cys Glu Ala Gln Asp Leu Tyr
            1460                1465


<210>    3
<211>    1511
<212>    PRT
<213>    Artificial Sequence

<220>
<223>    G3 (scF3)


<400>    3

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Thr Leu Gln Ser Asp Gln Glu Glu Ile Asp Tyr Asp Asp Thr Ile Ser
        835                 840                 845

Val Glu Met Lys Lys Glu Asp Phe Asp Ile Tyr Asp Glu Asp Glu Asn
    850                 855                 860

Gln Ser Pro Arg Ser Phe Gln Lys Lys Thr Arg His Tyr Phe Ile Ala
865                 870                 875                 880

Ala Val Glu Arg Leu Trp Asp Tyr Gly Met Ser Ser Ser Pro His Val
                885                 890                 895

Leu Arg Asn Arg Ala Gln Ser Gly Ser Val Pro Gln Phe Lys Lys Val
            900                 905                 910

Val Phe Gln Glu Phe Thr Asp Gly Ser Phe Thr Gln Pro Leu Tyr Arg
        915                 920                 925

Gly Glu Leu Asn Glu His Leu Gly Leu Leu Gly Pro Tyr Ile Arg Ala
    930                 935                 940

Glu Val Glu Asp Asn Ile Met Val Thr Phe Arg Asn Gln Ala Ser Arg
945                 950                 955                 960

Pro Tyr Ser Phe Tyr Ser Ser Leu Ile Ser Tyr Glu Glu Asp Gln Arg
                965                 970                 975

Gln Gly Ala Glu Pro Arg Lys Asn Phe Val Lys Pro Asn Glu Thr Lys
            980                 985                 990

Thr Tyr Phe Trp Lys Val Gln His His Met Ala Pro Thr Lys Asp Glu
        995                 1000                1005

Phe Asp Cys Lys Ala Trp Ala Tyr Phe Ser Asp Val Asp Leu Glu Lys
    1010                1015                1020

Asp Val His Ser Gly Leu Ile Gly Pro Leu Leu Val Cys His Thr Asn
1025                1030                1035                1040

Thr Leu Asn Pro Ala His Gly Arg Gln Val Thr Val Gln Glu Phe Ala
                1045                1050                1055

Leu Phe Phe Thr Ile Phe Asp Glu Thr Lys Ser Trp Tyr Phe Thr Glu
            1060                1065                1070

Asn Met Glu Arg Asn Cys Arg Ala Pro Cys Asn Ile Gln Met Glu Asp
        1075                1080                1085

Pro Thr Phe Lys Glu Asn Tyr Arg Phe His Ala Ile Asn Gly Tyr Ile
    1090                1095                1100

Met Asp Thr Leu Pro Gly Leu Val Met Ala Gln Asp Gln Arg Ile Arg
1105                1110                1115                1120

Trp Tyr Leu Leu Ser Met Gly Ser Asn Glu Asn Ile His Ser Ile His
                1125                1130                1135

Phe Ser Gly His Val Phe Thr Val Arg Lys Lys Glu Glu Tyr Lys Met
            1140                1145                1150

Ala Leu Tyr Asn Leu Tyr Pro Gly Val Phe Glu Thr Val Glu Met Leu
        1155                1160                1165

Pro Ser Lys Ala Gly Ile Trp Arg Val Glu Cys Leu Ile Gly Glu His
    1170                1175                1180

Leu His Ala Gly Met Ser Thr Leu Phe Leu Val Tyr Ser Asn Lys Cys
1185                1190                1195                1200

Gln Thr Pro Leu Gly Met Ala Ser Gly His Ile Arg Asp Phe Gln Ile
                1205                1210                1215

Thr Ala Ser Gly Gln Tyr Gly Gln Trp Ala Pro Lys Leu Ala Arg Leu
            1220                1225                1230

His Tyr Ser Gly Ser Ile Asn Ala Trp Ser Thr Lys Glu Pro Phe Ser
        1235                1240                1245

Trp Ile Lys Val Asp Leu Leu Ala Pro Met Ile Ile His Gly Ile Lys
    1250                1255                1260

Thr Gln Gly Ala Arg Gln Lys Phe Ser Ser Leu Tyr Ile Ser Gln Phe
1265                1270                1275                1280

Ile Ile Met Tyr Ser Leu Asp Gly Lys Lys Trp Gln Thr Tyr Arg Gly
                1285                1290                1295

Asn Ser Thr Gly Thr Leu Met Val Phe Phe Gly Asn Val Asp Ser Ser
            1300                1305                1310

Gly Ile Lys His Asn Ile Phe Asn Pro Pro Ile Ile Ala Arg Tyr Ile
        1315                1320                1325

Arg Leu His Pro Thr His Tyr Ser Ile Arg Ser Thr Leu Arg Met Glu
    1330                1335                1340

Leu Met Gly Cys Asp Leu Asn Ser Cys Ser Met Pro Leu Gly Met Glu
1345                1350                1355                1360

Ser Lys Ala Ile Ser Asp Ala Gln Ile Thr Ala Ser Ser Tyr Phe Thr
                1365                1370                1375

Asn Met Phe Ala Thr Trp Ser Pro Ser Lys Ala Arg Leu His Leu Gln
            1380                1385                1390

Gly Arg Ser Asn Ala Trp Arg Pro Gln Val Asn Asn Pro Lys Glu Trp
        1395                1400                1405

Leu Gln Val Asp Phe Gln Lys Thr Met Lys Val Thr Gly Val Thr Thr
    1410                1415                1420

Gln Gly Val Lys Ser Leu Leu Thr Ser Met Tyr Val Lys Glu Phe Leu
1425                1430                1435                1440

Ile Ser Ser Ser Gln Asp Gly His Gln Trp Thr Leu Phe Phe Gln Asn
                1445                1450                1455

Gly Lys Val Lys Val Phe Gln Gly Asn Gln Asp Ser Phe Thr Pro Val
            1460                1465                1470

Val Asn Ser Leu Asp Pro Pro Leu Leu Thr Arg Tyr Leu Arg Ile His
        1475                1480                1485

Pro Gln Ser Trp Val His Gln Ile Ala Leu Arg Met Glu Val Leu Gly
    1490                1495                1500

Cys Glu Ala Gln Asp Leu Tyr
1505                1510


<210>    4
<211>    1581
<212>    PRT
<213>    Artificial Sequence

<220>
<223>    G4 (scF4)


<400>    4
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala

            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile

435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn

770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val

1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys

1445                1450                1455

Ala Arg Leu His Leu Gln Gly Arg Ser Asn Ala Trp Arg Pro Gln Val
        1460                1465                1470

Asn Asn Pro Lys Glu Trp Leu Gln Val Asp Phe Gln Lys Thr Met Lys
    1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
        1540                1545                1550

Arg Tyr Leu Arg Ile His Pro Gln Ser Trp Val His Gln Ile Ala Leu
    1555                1560                1565

Arg Met Glu Val Leu Gly Cys Glu Ala Gln Asp Leu Tyr
    1570                1575                1580

<210>    5
<211>    1644
<212>    PRT
<213>    Artificial Sequence

<220>
<223>    G6 (scF5)

<400>    5
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn

130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile

465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe

805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Thr Leu Gln Ser Asp Gln Glu Glu Ile Asp Tyr
                965                 970                 975

Asp Asp Thr Ile Ser Val Glu Met Lys Lys Glu Asp Phe Asp Ile Tyr
            980                 985                 990

Asp Glu Asp Glu Asn Gln Ser Pro Arg Ser Phe Gln Lys Lys Thr Arg
        995                 1000                1005

His Tyr Phe Ile Ala Ala Val Glu Arg Leu Trp Asp Tyr Gly Met Ser
    1010                1015                1020

Ser Ser Pro His Val Leu Arg Asn Arg Ala Gln Ser Gly Ser Val Pro
1025                1030                1035                1040

Gln Phe Lys Lys Val Val Phe Gln Glu Phe Thr Asp Gly Ser Phe Thr
                1045                1050                1055

Gln Pro Leu Tyr Arg Gly Glu Leu Asn Glu His Leu Gly Leu Leu Gly
            1060                1065                1070

Pro Tyr Ile Arg Ala Glu Val Glu Asp Asn Ile Met Val Thr Phe Arg
        1075                1080                1085

Asn Gln Ala Ser Arg Pro Tyr Ser Phe Tyr Ser Ser Leu Ile Ser Tyr
    1090                1095                1100

Glu Glu Asp Gln Arg Gln Gly Ala Glu Pro Arg Lys Asn Phe Val Lys
1105                1110                1115                1120

Pro Asn Glu Thr Lys Thr Tyr Phe Trp Lys Val Gln His His Met Ala
                1125                1130                1135

Pro Thr Lys Asp Glu Phe Asp Cys Lys Ala Trp Ala Tyr Phe Ser Asp

1140                1145                1150

Val Asp Leu Glu Lys Asp Val His Ser Gly Leu Ile Gly Pro Leu Leu
        1155                1160                1165

Val Cys His Thr Asn Thr Leu Asn Pro Ala His Gly Arg Gln Val Thr
    1170                1175                1180

Val Gln Glu Phe Ala Leu Phe Phe Thr Ile Phe Asp Glu Thr Lys Ser
1185                1190                1195                1200

Trp Tyr Phe Thr Glu Asn Met Glu Arg Asn Cys Arg Ala Pro Cys Asn
                1205                1210                1215

Ile Gln Met Glu Asp Pro Thr Phe Lys Glu Asn Tyr Arg Phe His Ala
            1220                1225                1230

Ile Asn Gly Tyr Ile Met Asp Thr Leu Pro Gly Leu Val Met Ala Gln
        1235                1240                1245

Asp Gln Arg Ile Arg Trp Tyr Leu Leu Ser Met Gly Ser Asn Glu Asn
    1250                1255                1260

Ile His Ser Ile His Phe Ser Gly His Val Phe Thr Val Arg Lys Lys
1265                1270                1275                1280

Glu Glu Tyr Lys Met Ala Leu Tyr Asn Leu Tyr Pro Gly Val Phe Glu
                1285                1290                1295

Thr Val Glu Met Leu Pro Ser Lys Ala Gly Ile Trp Arg Val Glu Cys
            1300                1305                1310

Leu Ile Gly Glu His Leu His Ala Gly Met Ser Thr Leu Phe Leu Val
        1315                1320                1325

Tyr Ser Asn Lys Cys Gln Thr Pro Leu Gly Met Ala Ser Gly His Ile
    1330                1335                1340

Arg Asp Phe Gln Ile Thr Ala Ser Gly Gln Tyr Gly Gln Trp Ala Pro
1345                1350                1355                1360

Lys Leu Ala Arg Leu His Tyr Ser Gly Ser Ile Asn Ala Trp Ser Thr
                1365                1370                1375

Lys Glu Pro Phe Ser Trp Ile Lys Val Asp Leu Leu Ala Pro Met Ile
            1380                1385                1390

Ile His Gly Ile Lys Thr Gln Gly Ala Arg Gln Lys Phe Ser Ser Leu
        1395                1400                1405

Tyr Ile Ser Gln Phe Ile Ile Met Tyr Ser Leu Asp Gly Lys Lys Trp
    1410                1415                1420

Gln Thr Tyr Arg Gly Asn Ser Thr Gly Thr Leu Met Val Phe Phe Gly
1425                1430                1435                1440

Asn Val Asp Ser Ser Gly Ile Lys His Asn Ile Phe Asn Pro Pro Ile
                1445                1450                1455

Ile Ala Arg Tyr Ile Arg Leu His Pro Thr His Tyr Ser Ile Arg Ser
            1460                1465                1470

Thr Leu Arg Met Glu Leu Met Gly Cys Asp Leu Asn Ser Cys Ser Met

1475                1480                1485

Pro Leu Gly Met Glu Ser Lys Ala Ile Ser Asp Ala Gln Ile Thr Ala
    1490                1495                1500

Ser Ser Tyr Phe Thr Asn Met Phe Ala Thr Trp Ser Pro Ser Lys Ala
1505                1510                1515                1520

Arg Leu His Leu Gln Gly Arg Ser Asn Ala Trp Arg Pro Gln Val Asn
                1525                1530                1535

Asn Pro Lys Glu Trp Leu Gln Val Asp Phe Gln Lys Thr Met Lys Val
            1540                1545                1550

Thr Gly Val Thr Thr Gln Gly Val Lys Ser Leu Leu Thr Ser Met Tyr
        1555                1560                1565

Val Lys Glu Phe Leu Ile Ser Ser Ser Gln Asp Gly His Gln Trp Thr
    1570                1575                1580

Leu Phe Phe Gln Asn Gly Lys Val Lys Val Phe Gln Gly Asn Gln Asp
1585                1590                1595                1600

Ser Phe Thr Pro Val Val Asn Ser Leu Asp Pro Pro Leu Leu Thr Arg
                1605                1610                1615

Tyr Leu Arg Ile His Pro Gln Ser Trp Val His Gln Ile Ala Leu Arg
            1620                1625                1630

Met Glu Val Leu Gly Cys Glu Ala Gln Asp Leu Tyr
        1635                1640

<210>   6
<211>   1587
<212>   PRT
<213>   Artificial Sequence

<220>
<223>   G4_flex (scF6)

<400>   6
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
        450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Gln Asn Pro Pro Val Thr Leu Gln Ser
            900                 905                 910

Asp Gln Glu Glu Ile Asp Tyr Asp Asp Thr Ile Ser Val Glu Met Lys
        915                 920                 925

Lys Glu Asp Phe Asp Ile Tyr Asp Glu Asp Glu Asn Gln Ser Pro Arg
    930                 935                 940

Ser Phe Gln Lys Lys Thr Arg His Tyr Phe Ile Ala Ala Val Glu Arg
945                 950                 955                 960

Leu Trp Asp Tyr Gly Met Ser Ser Ser Pro His Val Leu Arg Asn Arg
                965                 970                 975

Ala Gln Ser Gly Ser Val Pro Gln Phe Lys Lys Val Val Phe Gln Glu
            980                 985                 990

Phe Thr Asp Gly Ser Phe Thr Gln Pro Leu Tyr Arg Gly Glu Leu Asn
        995                 1000                1005

Glu His Leu Gly Leu Leu Gly Pro Tyr Ile Arg Ala Glu Val Glu Asp
    1010                1015                1020

Asn Ile Met Val Thr Phe Arg Asn Gln Ala Ser Arg Pro Tyr Ser Phe
1025                1030                1035                1040

Tyr Ser Ser Leu Ile Ser Tyr Glu Glu Asp Gln Arg Gln Gly Ala Glu
                1045                1050                1055

Pro Arg Lys Asn Phe Val Lys Pro Asn Glu Thr Lys Thr Tyr Phe Trp
            1060                1065                1070

Lys Val Gln His His Met Ala Pro Thr Lys Asp Glu Phe Asp Cys Lys
        1075                1080                1085

Ala Trp Ala Tyr Phe Ser Asp Val Asp Leu Glu Lys Asp Val His Ser
    1090                1095                1100

Gly Leu Ile Gly Pro Leu Leu Val Cys His Thr Asn Thr Leu Asn Pro
1105                1110                1115                1120

Ala His Gly Arg Gln Val Thr Val Gln Glu Phe Ala Leu Phe Phe Thr
                1125                1130                1135

Ile Phe Asp Glu Thr Lys Ser Trp Tyr Phe Thr Glu Asn Met Glu Arg
            1140                1145                1150

Asn Cys Arg Ala Pro Cys Asn Ile Gln Met Glu Asp Pro Thr Phe Lys
        1155                1160                1165

Glu Asn Tyr Arg Phe His Ala Ile Asn Gly Tyr Ile Met Asp Thr Leu
    1170                1175                1180

Pro Gly Leu Val Met Ala Gln Asp Gln Arg Ile Arg Trp Tyr Leu Leu
1185                1190                1195                1200

Ser Met Gly Ser Asn Glu Asn Ile His Ser Ile His Phe Ser Gly His
                1205                1210                1215

Val Phe Thr Val Arg Lys Lys Glu Glu Tyr Lys Met Ala Leu Tyr Asn
            1220                1225                1230

Leu Tyr Pro Gly Val Phe Glu Thr Val Glu Met Leu Pro Ser Lys Ala
        1235                1240                1245

Gly Ile Trp Arg Val Glu Cys Leu Ile Gly Glu His Leu His Ala Gly
    1250                1255                1260

Met Ser Thr Leu Phe Leu Val Tyr Ser Asn Lys Cys Gln Thr Pro Leu
1265                1270                1275                1280

Gly Met Ala Ser Gly His Ile Arg Asp Phe Gln Ile Thr Ala Ser Gly
                1285                1290                1295

Gln Tyr Gly Gln Trp Ala Pro Lys Leu Ala Arg Leu His Tyr Ser Gly
            1300                1305                1310

Ser Ile Asn Ala Trp Ser Thr Lys Glu Pro Phe Ser Trp Ile Lys Val
        1315                1320                1325

Asp Leu Leu Ala Pro Met Ile Ile His Gly Ile Lys Thr Gln Gly Ala
    1330                1335                1340

Arg Gln Lys Phe Ser Ser Leu Tyr Ile Ser Gln Phe Ile Ile Met Tyr
1345                1350                1355                1360

Ser Leu Asp Gly Lys Lys Trp Gln Thr Tyr Arg Gly Asn Ser Thr Gly
                1365                1370                1375

Thr Leu Met Val Phe Phe Gly Asn Val Asp Ser Ser Gly Ile Lys His
            1380                1385                1390

Asn Ile Phe Asn Pro Pro Ile Ile Ala Arg Tyr Ile Arg Leu His Pro
        1395                1400                1405

Thr His Tyr Ser Ile Arg Ser Thr Leu Arg Met Glu Leu Met Gly Cys
    1410                1415                1420

Asp Leu Asn Ser Cys Ser Met Pro Leu Gly Met Glu Ser Lys Ala Ile
1425                1430                1435                1440

Ser Asp Ala Gln Ile Thr Ala Ser Ser Tyr Phe Thr Asn Met Phe Ala
                1445                1450                1455

Thr Trp Ser Pro Ser Lys Ala Arg Leu His Leu Gln Gly Arg Ser Asn
            1460                1465                1470

Ala Trp Arg Pro Gln Val Asn Asn Pro Lys Glu Trp Leu Gln Val Asp
        1475                1480                1485

Phe Gln Lys Thr Met Lys Val Thr Gly Val Thr Thr Gln Gly Val Lys
    1490                1495                1500

Ser Leu Leu Thr Ser Met Tyr Val Lys Glu Phe Leu Ile Ser Ser Ser
1505                1510                1515                1520

Gln Asp Gly His Gln Trp Thr Leu Phe Phe Gln Asn Gly Lys Val Lys
                1525                1530                1535

Val Phe Gln Gly Asn Gln Asp Ser Phe Thr Pro Val Val Asn Ser Leu
            1540                1545                1550

Asp Pro Pro Leu Leu Thr Arg Tyr Leu Arg Ile His Pro Gln Ser Trp
        1555                1560                1565

Val His Gln Ile Ala Leu Arg Met Glu Val Leu Gly Cys Glu Ala Gln
    1570                1575                1580

Asp Leu Tyr
1585

<210>    7
<211>    1644
<212>    PRT
<213>    Artificial Sequence

<220>
<223>    G6_opt (scF7)

<400>    7
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Thr Leu Gln Ser Asp Gln Glu Glu Ile Asp Tyr
                965                 970                 975

Asp Asp Thr Ile Ser Val Glu Met Lys Lys Glu Asp Phe Asp Ile Tyr
            980                 985                 990

Asp Glu Asp Glu Asn Gln Ser Pro Arg Ser Phe Gln Lys Lys Thr Arg
        995                 1000                1005

His Tyr Phe Ile Ala Ala Val Glu Arg Leu Trp Asp Tyr Gly Met Ser
    1010                1015                1020

Ser Ser Pro His Val Leu Arg Asn Arg Ala Gln Ser Gly Ser Val Pro
1025                1030                1035                1040

Gln Phe Lys Lys Val Val Phe Gln Glu Phe Thr Asp Gly Ser Phe Thr
                1045                1050                1055

Gln Pro Leu Tyr Arg Gly Glu Leu Asn Glu His Leu Gly Leu Leu Gly
            1060                1065                1070

Pro Tyr Ile Arg Ala Glu Val Glu Asp Asn Ile Met Val Thr Phe Arg
        1075                1080                1085

Asn Gln Ala Ser Arg Pro Tyr Ser Phe Tyr Ser Ser Leu Ile Ser Tyr
    1090                1095                1100

Glu Glu Asp Gln Arg Gln Gly Ala Glu Pro Arg Lys Asn Phe Val Lys
1105                1110                1115                1120

Pro Asn Glu Thr Lys Thr Tyr Phe Trp Lys Val Gln His His Met Ala
                1125                1130                1135

Pro Thr Lys Asp Glu Phe Asp Cys Lys Ala Trp Ala Tyr Phe Ser Asp
            1140                1145                1150

Val Asp Leu Glu Lys Asp Val His Ser Gly Leu Ile Gly Pro Leu Leu
        1155                1160                1165

Val Cys His Thr Asn Thr Leu Asn Pro Ala His Gly Arg Gln Val Thr
    1170                1175                1180

Val Gln Glu Phe Ala Leu Phe Phe Thr Ile Phe Asp Glu Thr Lys Ser
1185                1190                1195                1200

Trp Tyr Phe Thr Glu Asn Met Glu Arg Asn Cys Arg Ala Pro Cys Asn
                1205                1210                1215

Ile Gln Met Glu Asp Pro Thr Phe Lys Glu Asn Tyr Arg Phe His Ala
            1220                1225                1230

Ile Asn Gly Tyr Ile Met Asp Thr Leu Pro Gly Leu Val Met Ala Gln
        1235                1240                1245

Asp Gln Arg Ile Arg Trp Tyr Leu Leu Ser Met Gly Ser Asn Glu Asn
    1250                1255                1260

Ile His Ser Ile His Phe Ser Gly His Val Phe Thr Val Arg Lys Lys
1265                1270                1275                1280

Glu Glu Tyr Lys Met Ala Leu Tyr Asn Leu Tyr Pro Gly Val Phe Glu
                1285                1290                1295

Thr Val Glu Met Leu Pro Ser Lys Ala Gly Ile Trp Arg Val Glu Cys
            1300                1305                1310

Leu Ile Gly Glu His Leu His Ala Gly Met Ser Thr Leu Phe Leu Val
        1315                1320                1325

Tyr Ser Asn Lys Cys Gln Thr Pro Leu Gly Met Ala Ser Gly His Ile
    1330                1335                1340

Arg Asp Phe Gln Ile Thr Ala Ser Gly Gln Tyr Gly Gln Trp Ala Pro
1345                1350                1355                1360

Lys Leu Ala Arg Leu His Tyr Ser Gly Ser Ile Asn Ala Trp Ser Thr
                1365                1370                1375

Lys Glu Pro Phe Ser Trp Ile Lys Val Asp Leu Leu Ala Pro Met Ile
            1380                1385                1390

Ile His Gly Ile Lys Thr Gln Gly Ala Arg Gln Lys Phe Ser Ser Leu
        1395                1400                1405

Tyr Ile Ser Gln Phe Ile Ile Met Tyr Ser Leu Asp Gly Lys Lys Trp
    1410                1415                1420

Gln Thr Tyr Arg Gly Asn Ser Thr Gly Thr Leu Met Val Phe Phe Gly
1425                1430                1435                1440

Asn Val Asp Ser Ser Gly Ile Lys His Asn Ile Phe Asn Pro Pro Ile
                1445                1450                1455

Ile Ala Arg Tyr Ile Arg Leu His Pro Thr His Tyr Ser Ile Arg Ser
            1460                1465                1470

Thr Leu Arg Met Glu Leu Met Gly Cys Asp Leu Asn Ser Cys Ser Met
        1475                1480                1485

Pro Leu Gly Met Glu Ser Lys Ala Ile Ser Asp Ala Gln Ile Thr Ala
    1490                1495                1500

Ser Ser Tyr Phe Thr Asn Met Phe Ala Thr Trp Ser Pro Ser Lys Ala
1505                1510                1515                1520

Arg Leu His Leu Gln Gly Arg Ser Asn Ala Trp Arg Pro Gln Val Asn
                1525                1530                1535

Asn Pro Lys Glu Trp Leu Gln Val Asp Phe Gln Lys Thr Met Lys Val
            1540                1545                1550

Thr Gly Val Thr Thr Gln Gly Val Lys Ser Leu Leu Thr Ser Met Tyr
        1555                1560                1565

Val Lys Glu Phe Leu Ile Ser Ser Ser Gln Asp Gly His Gln Trp Thr
    1570                1575                1580

Leu Phe Phe Gln Asn Gly Lys Val Lys Val Phe Gln Gly Asn Gln Asp
1585                1590                1595                1600

Ser Phe Thr Pro Val Val Asn Ser Leu Asp Pro Pro Leu Leu Thr Arg
                1605                1610                1615

Tyr Leu Arg Ile His Pro Gln Ser Trp Val His Gln Ile Ala Leu Arg
            1620                1625                1630

Met Glu Val Leu Gly Cys Glu Ala Gln Asp Leu Tyr
        1635                1640


<210>    8
<211>    1444
<212>    PRT
<213>    Artificial Sequence

<220>
<223>    G1 (scF1)


<400>    8
Ala Thr Arg Arg Tyr Tyr Leu Gly Ala Val Glu Leu Ser Trp Asp Tyr
  1               5                  10                  15

Met Gln Ser Asp Leu Gly Glu Leu Pro Val Asp Ala Arg Phe Pro Pro
             20                  25                  30

Arg Val Pro Lys Ser Phe Pro Phe Asn Thr Ser Val Val Tyr Lys Lys
         35                  40                  45

Thr Leu Phe Val Glu Phe Thr Asp His Leu Phe Asn Ile Ala Lys Pro
     50                  55                  60

Arg Pro Pro Trp Met Gly Leu Leu Gly Pro Thr Ile Gln Ala Glu Val
 65                  70                  75                  80

Tyr Asp Thr Val Val Ile Thr Leu Lys Asn Met Ala Ser His Pro Val
                 85                  90                  95

Ser Leu His Ala Val Gly Val Ser Tyr Trp Lys Ala Ser Glu Gly Ala

            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile

435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Thr Thr Leu Gln
        755                 760                 765

Ser Asp Gln Glu Glu Ile Asp Tyr Asp Asp Thr Ile Ser Val Glu Met

770                 775                 780

Lys Lys Glu Asp Phe Asp Ile Tyr Asp Glu Asp Glu Asn Gln Ser Pro
785                 790                 795                 800

Arg Ser Phe Gln Lys Lys Thr Arg His Tyr Phe Ile Ala Ala Val Glu
                805                 810                 815

Arg Leu Trp Asp Tyr Gly Met Ser Ser Ser Pro His Val Leu Arg Asn
            820                 825                 830

Arg Ala Gln Ser Gly Ser Val Pro Gln Phe Lys Lys Val Val Phe Gln
        835                 840                 845

Glu Phe Thr Asp Gly Ser Phe Thr Gln Pro Leu Tyr Arg Gly Glu Leu
    850                 855                 860

Asn Glu His Leu Gly Leu Leu Gly Pro Tyr Ile Arg Ala Glu Val Glu
865                 870                 875                 880

Asp Asn Ile Met Val Thr Phe Arg Asn Gln Ala Ser Arg Pro Tyr Ser
                885                 890                 895

Phe Tyr Ser Ser Leu Ile Ser Tyr Glu Glu Asp Gln Arg Gln Gly Ala
            900                 905                 910

Glu Pro Arg Lys Asn Phe Val Lys Pro Asn Glu Thr Lys Thr Tyr Phe
        915                 920                 925

Trp Lys Val Gln His His Met Ala Pro Thr Lys Asp Glu Phe Asp Cys
    930                 935                 940

Lys Ala Trp Ala Tyr Phe Ser Asp Val Asp Leu Glu Lys Asp Val His
945                 950                 955                 960

Ser Gly Leu Ile Gly Pro Leu Leu Val Cys His Thr Asn Thr Leu Asn
                965                 970                 975

Pro Ala His Gly Arg Gln Val Thr Val Gln Glu Phe Ala Leu Phe Phe
            980                 985                 990

Thr Ile Phe Asp Glu Thr Lys Ser Trp Tyr Phe Thr Glu Asn Met Glu
        995                 1000                1005

Arg Asn Cys Arg Ala Pro Cys Asn Ile Gln Met Glu Asp Pro Thr Phe
    1010                1015                1020

Lys Glu Asn Tyr Arg Phe His Ala Ile Asn Gly Tyr Ile Met Asp Thr
1025                1030                1035                1040

Leu Pro Gly Leu Val Met Ala Gln Asp Gln Arg Ile Arg Trp Tyr Leu
                1045                1050                1055

Leu Ser Met Gly Ser Asn Glu Asn Ile His Ser Ile His Phe Ser Gly
            1060                1065                1070

His Val Phe Thr Val Arg Lys Lys Glu Glu Tyr Lys Met Ala Leu Tyr
        1075                1080                1085

Asn Leu Tyr Pro Gly Val Phe Glu Thr Val Glu Met Leu Pro Ser Lys
    1090                1095                1100

Ala Gly Ile Trp Arg Val Glu Cys Leu Ile Gly Glu His Leu His Ala

1105                1110                1115                1120

Gly Met Ser Thr Leu Phe Leu Val Tyr Ser Asn Lys Cys Gln Thr Pro
                1125                1130                1135

Leu Gly Met Ala Ser Gly His Ile Arg Asp Phe Gln Ile Thr Ala Ser
            1140                1145                1150

Gly Gln Tyr Gly Gln Trp Ala Pro Lys Leu Ala Arg Leu His Tyr Ser
        1155                1160                1165

Gly Ser Ile Asn Ala Trp Ser Thr Lys Glu Pro Phe Ser Trp Ile Lys
    1170                1175                1180

Val Asp Leu Leu Ala Pro Met Ile Ile His Gly Ile Lys Thr Gln Gly
1185                1190                1195                1200

Ala Arg Gln Lys Phe Ser Ser Leu Tyr Ile Ser Gln Phe Ile Ile Met
                1205                1210                1215

Tyr Ser Leu Asp Gly Lys Lys Trp Gln Thr Tyr Arg Gly Asn Ser Thr
            1220                1225                1230

Gly Thr Leu Met Val Phe Phe Gly Asn Val Asp Ser Ser Gly Ile Lys
        1235                1240                1245

His Asn Ile Phe Asn Pro Pro Ile Ile Ala Arg Tyr Ile Arg Leu His
    1250                1255                1260

Pro Thr His Tyr Ser Ile Arg Ser Thr Leu Arg Met Glu Leu Met Gly
1265                1270                1275                1280

Cys Asp Leu Asn Ser Cys Ser Met Pro Leu Gly Met Glu Ser Lys Ala
                1285                1290                1295

Ile Ser Asp Ala Gln Ile Thr Ala Ser Ser Tyr Phe Thr Asn Met Phe
            1300                1305                1310

Ala Thr Trp Ser Pro Ser Lys Ala Arg Leu His Leu Gln Gly Arg Ser
        1315                1320                1325

Asn Ala Trp Arg Pro Gln Val Asn Asn Pro Lys Glu Trp Leu Gln Val
    1330                1335                1340

Asp Phe Gln Lys Thr Met Lys Val Thr Gly Val Thr Thr Gln Gly Val
1345                1350                1355                1360

Lys Ser Leu Leu Thr Ser Met Tyr Val Lys Glu Phe Leu Ile Ser Ser
                1365                1370                1375

Ser Gln Asp Gly His Gln Trp Thr Leu Phe Phe Gln Asn Gly Lys Val
            1380                1385                1390

Lys Val Phe Gln Gly Asn Gln Asp Ser Phe Thr Pro Val Val Asn Ser
        1395                1400                1405

Leu Asp Pro Pro Leu Leu Thr Arg Tyr Leu Arg Ile His Pro Gln Ser
    1410                1415                1420

Trp Val His Gln Ile Ala Leu Arg Met Glu Val Leu Gly Cys Glu Ala
1425                1430                1435                1440

Gln Asp Leu Tyr

<210> 9
<211> 6999
<212> DNA
<213> Homo sapiens

<400> 9
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagttcct taggaccccc aagtatgcca gttcattatg atagtcaatt agataccact 2760

ctatttggca aaaagtcatc tccccttact gagtctggtg gacctctgag cttgagtgaa 2820

gaaaataatg attcaaagtt gttagaatca ggtttaatga atagccaaga aagttcatgg 2880

ggaaaaaatg tatcgtcaac agagagtggt aggttattta aagggaaaag agctcatgga 2940

cctgctttgt tgactaaaga taatgcctta ttcaaagtta gcatctcttt gttaaagaca 3000

aacaaaactt ccaataattc agcaactaat agaaagactc acattgatgg cccatcatta 3060

ttaattgaga atagtccatc agtctggcaa aatatattag aaagtgacac tgagtttaaa 3120

aaagtgacac ctttgattca tgacagaatg cttatggaca aaaatgctac agctttgagg 3180

ctaaatcata tgtcaaataa aactacttca tcaaaaaaca tggaaatggt ccaacagaaa 3240

aaagagggcc ccattccacc agatgcacaa aatccagata tgtcgttctt taagatgcta 3300

ttcttgccag aatcagcaag gtggatacaa aggactcatg gaaagaactc tctgaactct 3360

gggcaaggcc ccagtccaaa gcaattagta tccttaggac cagaaaaatc tgtggaaggt 3420

cagaatttct tgtctgagaa aaacaaagtg gtagtaggaa agggtgaatt tacaaaggac 3480

gtaggactca aagagatggt ttttccaagc agcagaaacc tatttcttac taacttggat 3540

aatttacatg aaaataatac acacaatcaa gaaaaaaaaa ttcaggaaga aatagaaaag 3600

aaggaaacat taatccaaga gaatgtagtt ttgcctcaga tacatacagt gactggcact 3660

aagaatttca tgaagaacct tttcttactg agcactaggc aaaatgtaga aggttcatat 3720

gacggggcat atgctccagt acttcaagat tttaggtcat taaatgattc aacaaataga 3780

acaaagaaac acacagctca tttctcaaaa aaaggggagg aagaaaactt ggaaggcttg 3840

ggaaatcaaa ccaagcaaat tgtagagaaa tatgcatgca ccacaaggat atctcctaat 3900

acaagccagc agaattttgt cacgcaacgt agtaagagag ctttgaaaca attcagactc 3960

ccactagaag aaacagaact tgaaaaaagg ataattgtgg atgacacctc aacccagtgg 4020

tccaaaaaca tgaaacattt gaccccgagc accctcacac agatagacta caatgagaag 4080

gagaaagggg ccattactca gtctccctta tcagattgcc ttacgaggag tcatagcatc 4140

cctcaagcaa atagatctcc attacccatt gcaaaggtat catcatttcc atctattaga 4200

cctatatatc tgaccagggt cctattccaa gacaactctt ctcatcttcc agcagcatct 4260

tatagaaaga aagattctgg ggtccaagaa agcagtcatt tcttacaagg agccaaaaaa 4320

aataaccttt ctttagccat tctaaccttg gagatgactg gtgatcaaag agaggttggc 4380

tccctgggga caagtgccac aaattcagtc acatacaaga aagttgagaa cactgttctc 4440

ccgaaaccag acttgcccaa aacatctggc aaagttgaat tgcttccaaa agttcacatt 4500

tatcagaagg acctattccc tacggaaact agcaatgggt ctcctggcca tctggatctc 4560

gtggaaggga gccttcttca gggaacagag ggagcgatta agtggaatga agcaaacaga 4620

cctggaaaag ttccctttct gagagtagca acagaaagct ctgcaaagac tccctccaag 4680

ctattggatc ctcttgcttg ggataaccac tatggtactc agataccaaa agaagagtgg 4740

aaatcccaag agaagtcacc agaaaaaaca gcttttaaga aaaaggatac cattttgtcc 4800

ctgaacgctt gtgaaagcaa tcatgcaata gcagcaataa atgagggaca aaataagccc 4860

gaaatagaag tcacctgggc aaagcaaggt aggactgaaa ggctgtgctc tcaaaaccca 4920

ccagtcttga aacgccatca acgggaaata actcgtacta ctcttcagtc agatcaagag 4980

gaaattgact atgatgatac catatcagtt gaaatgaaga aggaagattt tgacatttat 5040

gatgaggatg aaaatcagag cccccgcagc tttcaaaaga aaacacgaca ctattttatt 5100

gctgcagtgg agaggctctg ggattatggg atgagtagct ccccacatgt tctaagaaac 5160

agggctcaga gtggcagtgt ccctcagttc aagaaagttg ttttccagga atttactgat 5220

ggctccttta ctcagccctt ataccgtgga gaactaaatg aacatttggg actcctgggg 5280

ccatatataa gagcagaagt tgaagataat atcatggtaa ctttcagaaa tcaggcctct 5340

cgtccctatt ccttctattc tagccttatt tcttatgagg aagatcagag gcaaggagca 5400

gaacctagaa aaaactttgt caagcctaat gaaaccaaaa cttacttttg gaaagtgcaa 5460

catcatatgg cacccactaa agatgagttt gactgcaaag cctgggctta tttctctgat 5520

gttgacctgg aaaaagatgt gcactcaggc ctgattggac cccttctggt ctgccacact 5580

aacacactga accctgctca tgggagacaa gtgacagtac aggaatttgc tctgtttttc 5640

accatctttg atgagaccaa aagctggtac ttcactgaaa atatggaaag aaactgcagg 5700

gctccctgca atatccagat ggaagatccc acttttaaag agaattatcg cttccatgca 5760

atcaatggct acataatgga tacactacct ggcttagtaa tggctcagga tcaaaggatt 5820

cgatggtatc tgctcagcat gggcagcaat gaaaacatcc attctattca tttcagtgga 5880

catgtgttca ctgtacgaaa aaaagaggag tataaaatgg cactgtacaa tctctatcca 5940

ggtgtttttg agacagtgga aatgttacca tccaaagctg gaatttggcg ggtggaatgc 6000

cttattggcg agcatctaca tgctgggatg agcacacttt ttctggtgta cagcaataag 6060

tgtcagactc ccctgggaat ggcttctgga cacattagag attttcagat tacagcttca 6120

ggacaatatg gacagtgggc cccaaagctg gccagacttc attattccgg atcaatcaat 6180

gcctggagca ccaaggagcc cttttcttgg atcaaggtgg atctgttggc accaatgatt 6240

attcacggca tcaagaccca gggtgcccgt cagaagttct ccagcctcta catctctcag 6300

tttatcatca tgtatagtct tgatgggaag aagtggcaga cttatcgagg aaattccact 6360

ggaaccttaa tggtcttctt tggcaatgtg gattcatctg ggataaaaca caatattttt 6420

aaccctccaa ttattgctcg atacatccgt ttgcacccaa ctcattatag cattcgcagc 6480

actcttcgca tggagttgat gggctgtgat ttaaatagtt gcagcatgcc attgggaatg 6540

gagagtaaag caatatcaga tgcacagatt actgcttcat cctactttac caatatgttt 6600

gccacctggt ctccttcaaa agctcgactt cacctccaag ggaggagtaa tgcctggaga 6660

cctcaggtga ataatccaaa agagtggctg caagtggact tccagaagac aatgaaagtc 6720

acaggagtaa ctactcaggg agtaaaatct ctgcttacca gcatgtatgt gaaggagttc 6780

ctcatctcca gcagtcaaga tggccatcag tggactctct tttttcagaa tggcaaagta 6840

aaggtttttc agggaaatca agactccttc acacctgtgg tgaactctct agacccaccg 6900

ttactgactc gctaccttcg aattcacccc cagagttggg tgcaccagat tgccctgagg 6960

atggaggttc tgggctgcga ggcacaggac ctctactga 6999

<210> 10
<211> 4404
<212> DNA
<213> Artificial Sequence

<220>
<223> G2

<400>    10
gccaccagaa gatactacct gggtgcagtg gaactgtcat gggactatat gcaaagtgat      60

ctcggtgagc tgcctgtgga cgcaagattt cctcctagag tgccaaaatc ttttccattc     120

aacacctcag tcgtgtacaa aaagactctg tttgtagaat tcacggatca ccttttcaac     180

atcgctaagc caaggccacc ctggatgggt ctgctaggtc ctaccatcca ggctgaggtt     240

tatgatacag tggtcattac acttaagaac atggcttccc atcctgtcag tcttcatgct     300

gttggtgtat cctactggaa agcttctgag ggagctgaat atgatgatca gaccagtcaa     360

agggagaaag aagatgataa agtcttccct ggtggaagcc atacatatgt ctggcaggtc     420

ctgaaagaga atggtccaat ggcctctgac ccactgtgcc ttacctactc atatctttct     480

catgtggacc tggtaaaaga cttgaattca ggcctcattg gagccctact agtatgtaga     540

gaagggagtc tggccaagga aaagacacag accttgcaca aatttatact actttttgct     600

gtatttgatg aagggaaaag ttggcactca gaaacaaaga actccttgat gcaggatagg     660

gatgctgcat ctgctcgggc ctggcctaaa atgcacacag tcaatggtta tgtaaacagg     720

tctctgccag gtctgattgg atgccacagg aaatcagtct attggcatgt gattggaatg     780

ggcaccactc ctgaagtgca ctcaatattc ctcgaaggtc acacatttct tgtgaggaac     840

catcgccagg cgtccttgga aatctcgcca ataactttcc ttactgctca aacactcttg     900

atggaccttg gacagtttct actgttttgt catatctctt cccaccaaca tgatggcatg     960

gaagcttatg tcaaagtaga cagctgtcca gaggaacccc aactacgaat gaaaaataat    1020

gaagaagcgg aagactatga tgatgatctt actgattctg aaatggatgt ggtcaggttt    1080

gatgatgaca actctccttc ctttatccaa attcgctcag ttgccaagaa gcatcctaaa    1140

acttgggtac attacattgc tgctgaagag gaggactggg actatgctcc cttagtcctc    1200

gcccccgatg acagaagtta taaaagtcaa tatttgaaca atggccctca gcggattggt    1260

aggaagtaca aaaaagtccg atttatggca tacacagatg aaacctttaa gactcgtgaa    1320

gctattcagc atgaatcagg aatcttggga cctttacttt atggggaagt tggagacaca    1380

ctgttgatta tatttaagaa tcaagcaagc agaccatata acatctaccc tcacggaatc    1440

actgatgtcc gtcctttgta ttcaaggaga ttaccaaaag gtgtaaaaca tttgaaggat    1500

tttccaattc tgccaggaga aatattcaaa tataaatgga cagtgactgt agaagatggg    1560

ccaactaaat cagatcctcg gtgcctgacc cgctattact ctagtttcgt taatatggag    1620

agagatctag cttcaggact cattggccct ctcctcatct gctacaaaga atctgtagat    1680

caaagaggaa accagataat gtcagacaag aggaatgtca tcctgttttc tgtatttgat    1740

gagaaccgaa gctggtacct cacagagaat atacaacgct ttctccccaa tccagctgga    1800

gtgcagcttg aggatccaga gttccaagcc tccaacatca tgcacagcat caatggctat    1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtactctt cagtcagatc aagaggaaat tgactatgat 2400

gataccatat cagttgaaat gaagaaggaa gattttgaca tttatgatga ggatgaaaat 2460

cagagccccc gcagctttca aaagaaaaca cgacactatt ttattgctgc agtggagagg 2520

ctctgggatt atgggatgag tagctcccca catgttctaa gaaacagggc tcagagtggc 2580

agtgtccctc agttcaagaa agttgttttc caggaattta ctgatggctc ctttactcag 2640

cccttatacc gtggagaact aaatgaacat ttgggactcc tggggccata tataagagca 2700

gaagttgaag ataatatcat ggtaactttc agaaatcagg cctctcgtcc ctattccttc 2760

tattctagcc ttatttctta tgaggaagat cagaggcaag gagcagaacc tagaaaaaac 2820

tttgtcaagc ctaatgaaac caaaacttac ttttggaaag tgcaacatca tatggcaccc 2880

actaaagatg agtttgactg caaagcctgg gcttatttct ctgatgttga cctggaaaaa 2940

gatgtgcact caggcctgat tggacccctt ctggtctgcc acactaacac actgaaccct 3000

gctcatggga gacaagtgac agtacaggaa tttgctctgt ttttcaccat ctttgatgag 3060

accaaaagct ggtacttcac tgaaaatatg gaaagaaact gcagggctcc ctgcaatatc 3120

cagatggaag atcccacttt taaagagaat tatcgcttcc atgcaatcaa tggctacata 3180

atggatacac tacctggctt agtaatggct caggatcaaa ggattcgatg gtatctgctc 3240

agcatgggca gcaatgaaaa catccattct attcatttca gtggacatgt gttcactgta 3300

cgaaaaaaag aggagtataa aatggcactg tacaatctct atccaggtgt ttttgagaca 3360

gtggaaatgt taccatccaa agctggaatt tggcgggtgg aatgccttat tggcgagcat 3420

ctacatgctg ggatgagcac actttttctg gtgtacagca ataagtgtca gactcccctg 3480

ggaatggctt ctggacacat tagagatttt cagattacag cttcaggaca atatggacag 3540

tgggccccaa agctggccag acttcattat tccggatcaa tcaatgcctg gagcaccaag 3600

gagccctttt cttggatcaa ggtggatctg ttggcaccaa tgattattca cggcatcaag 3660

acccagggtg cccgtcagaa gttctccagc ctctacatct ctcagtttat catcatgtat 3720

agtcttgatg ggaagaagtg gcagacttat cgaggaaatt ccactggaac cttaatggtc 3780

ttctttggca atgtggattc atctgggata aaacacaata tttttaaccc tccaattatt 3840

gctcgataca tccgtttgca cccaactcat tatagcattc gcagcactct tcgcatggag 3900

ttgatgggct gtgatttaaa tagttgcagc atgccattgg gaatggagag taaagcaata 3960

tcagatgcac agattactgc ttcatcctac tttaccaata tgtttgccac ctggtctcct 4020

tcaaaagctc gacttcacct ccaagggagg agtaatgcct ggagacctca ggtgaataat 4080

ccaaaagagt ggctgcaagt ggacttccag aagacaatga aagtcacagg agtaactact 4140

cagggagtaa aatctctgct taccagcatg tatgtgaagg agttcctcat ctccagcagt 4200

caagatggcc atcagtggac tctctttttt cagaatggca aagtaaaggt tttcagggga 4260

aatcaagact ccttcacacc tgtggtgaac tctctagacc caccgttact gactcgctac 4320

cttcgaattc acccccagag ttgggtgcac cagattgccc tgaggatgga ggttctgggc 4380

tgcgaggcac aggacctcta ctga 4404

<210> 11
<211> 4536
<212> DNA
<213> Artificial Sequence

<220>
<223> G3

<400> 11
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctactc ttcagtcaga tcaagaggaa 2520

attgactatg atgataccat atcagttgaa atgaagaagg aagattttga catttatgat 2580

gaggatgaaa atcagagccc ccgcagcttt caaaagaaaa cacgacacta ttttattgct 2640

gcagtggaga ggctctggga ttatgggatg agtagctccc cacatgttct aagaaacagg 2700

gctcagagtg gcagtgtccc tcagttcaag aaagttgttt tccaggaatt tactgatggc 2760

tcctttactc agcccttata ccgtggagaa ctaaatgaac atttgggact cctggggcca 2820

tatataagag cagaagttga agataatatc atggtaactt tcagaaatca ggcctctcgt 2880

ccctattcct tctattctag ccttatttct tatgaggaag atcagaggca aggagcagaa 2940

cctagaaaaa actttgtcaa gcctaatgaa accaaaactt acttttggaa agtgcaacat 3000

catatggcac ccactaaaga tgagtttgac tgcaaagcct gggcttattt ctctgatgtt 3060

gacctggaaa aagatgtgca ctcaggcctg attggacccc ttctggtctg ccacactaac 3120

acactgaacc ctgctcatgg gagacaagtg acagtacagg aatttgctct gtttttcacc 3180

atctttgatg agaccaaaag ctggtacttc actgaaaata tggaaagaaa ctgcagggct 3240

ccctgcaata tccagatgga agatcccact tttaaagaga attatcgctt ccatgcaatc 3300

aatggctaca taatggatac actacctggc ttagtaatgg ctcaggatca aaggattcga 3360

tggtatctgc tcagcatggg cagcaatgaa aacatccatt ctattcattt cagtggacat 3420

gtgttcactg tacgaaaaaa agaggagtat aaaatggcac tgtacaatct ctatccaggt 3480

gtttttgaga cagtggaaat gttaccatcc aaagctggaa tttggcgggt ggaatgcctt 3540

attggcgagc atctacatgc tgggatgagc acactttttc tggtgtacag caataagtgt 3600

cagactcccc tgggaatggc ttctggacac attagagatt ttcagattac agcttcagga 3660

caatatggac agtgggcccc aaagctggcc agacttcatt attccggatc aatcaatgcc 3720

tggagcacca aggagccctt ttcttggatc aaggtggatc tgttggcacc aatgattatt 3780

cacggcatca agacccaggg tgcccgtcag aagttctcca gcctctacat ctctcagttt 3840

atcatcatgt atagtcttga tgggaagaag tggcagactt atcgaggaaa ttccactgga 3900

accttaatgg tcttctttgg caatgtggat tcatctggga taaaacacaa tatttttaac 3960

cctccaatta ttgctcgata catccgtttg cacccaactc attatagcat tcgcagcact 4020

cttcgcatgg agttgatggg ctgtgattta aatagttgca gcatgccatt gggaatggag 4080

agtaaagcaa tatcagatgc acagattact gcttcatcct actttaccaa tatgtttgcc 4140

acctggtctc cttcaaaagc tcgacttcac ctccaaggga ggagtaatgc ctggagacct 4200

caggtgaata atccaaaaga gtggctgcaa gtggacttcc agaagacaat gaaagtcaca 4260

ggagtaacta ctcagggagt aaaatctctg cttaccagca tgtatgtgaa ggagttcctc 4320

atctccagca gtcaagatgg ccatcagtgg actctctttt ttcagaatgg caaagtaaag 4380

gtttttcagg gaaatcaaga ctccttcaca cctgtggtga actctctaga cccaccgtta 4440

ctgactcgct accttcgaat tcacccccag agttgggtgc accagattgc cctgaggatg 4500

gaggttctgg gctgcgaggc acaggacctc tactga 4536

<210> 12

<211>    4536
<212>    DNA
<213>    Artificial Sequence

<220>
<223>    G4


<400>    12
gccaccagaa gatactacct gggtgcagtg gaactgtcat gggactatat gcaaagtgat      60

ctcggtgagc tgcctgtgga cgcaagattt cctcctagag tgccaaaatc ttttccattc     120

aacacctcag tcgtgtacaa aaagactctg tttgtagaat tcacggatca ccttttcaac     180

atcgctaagc caaggccacc ctggatgggt ctgctaggtc ctaccatcca ggctgaggtt     240

tatgatacag tggtcattac acttaagaac atggcttccc atcctgtcag tcttcatgct     300

gttggtgtat cctactggaa agcttctgag ggagctgaat atgatgatca gaccagtcaa     360

agggagaaag aagatgataa agtcttccct ggtggaagcc atacatatgt ctggcaggtc     420

ctgaaagaga atggtccaat ggcctctgac ccactgtgcc ttacctactc atatctttct     480

catgtggacc tggtaaaaga cttgaattca ggcctcattg gagccctact agtatgtaga     540

gaagggagtc tggccaagga aaagacacag accttgcaca aatttatact actttttgct     600

gtatttgatg aagggaaaag ttggcactca gaaacaaaga actccttgat gcaggatagg     660

gatgctgcat ctgctcgggc ctggcctaaa atgcacacag tcaatggtta tgtaaacagg     720

tctctgccag gtctgattgg atgccacagg aaatcagtct attggcatgt gattggaatg     780

ggcaccactc ctgaagtgca ctcaatattc ctcgaaggtc acacatttct tgtgaggaac     840

catcgccagg cgtccttgga aatctcgcca ataactttcc ttactgctca aacactcttg     900

atggaccttg gacagtttct actgttttgt catatctctt cccaccaaca tgatggcatg     960

gaagcttatg tcaaagtaga cagctgtcca gaggaacccc aactacgaat gaaaaataat    1020

gaagaagcgg aagactatga tgatgatctt actgattctg aaatggatgt ggtcaggttt    1080

gatgatgaca actctccttc ctttatccaa attcgctcag ttgccaagaa gcatcctaaa    1140

acttgggtac attacattgc tgctgaagag gaggactggg actatgctcc cttagtcctc    1200

gcccccgatg acagaagtta taaaagtcaa tatttgaaca atggccctca gcggattggt    1260

aggaagtaca aaaaagtccg atttatggca tacacagatg aaacctttaa gactcgtgaa    1320

gctattcagc atgaatcagg aatcttggga cctttacttt atggggaagt tggagacaca    1380

ctgttgatta tatttaagaa tcaagcaagc agaccatata acatctaccc tcacggaatc    1440

actgatgtcc gtcctttgta ttcaaggaga ttaccaaaag gtgtaaaaca tttgaaggat    1500

tttccaattc tgccaggaga aatattcaaa tataaatgga cagtgactgt agaagatggg    1560

ccaactaaat cagatcctcg gtgcctgacc cgctattact ctagtttcgt taatatggag    1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctactc ttcagtcaga tcaagaggaa 2520

attgactatg atgataccat atcagttgaa atgaagaagg aagattttga catttatgat 2580

gaggatgaaa atcagagccc ccgcagcttt caaaagaaaa cacgacacta ttttattgct 2640

gcagtggaga ggctctggga ttatgggatg agtagctccc cacatgttct aagaaacagg 2700

gctcagagtg gcagtgtccc tcagttcaag aaagttgttt tccaggaatt tactgatggc 2760

tcctttactc agcccttata ccgtggagaa ctaaatgaac atttgggact cctggggcca 2820

tatataagag cagaagttga agataatatc atggtaactt tcagaaatca ggcctctcgt 2880

ccctattcct tctattctag ccttatttct tatgaggaag atcagaggca aggagcagaa 2940

cctagaaaaa actttgtcaa gcctaatgaa accaaaactt acttttggaa agtgcaacat 3000

catatggcac ccactaaaga tgagtttgac tgcaaagcct gggcttattt ctctgatgtt 3060

gacctggaaa aagatgtgca ctcaggcctg attggacccc ttctggtctg ccacactaac 3120

acactgaacc ctgctcatgg gagacaagtg acagtacagg aatttgctct gtttttcacc 3180

atctttgatg agaccaaaag ctggtacttc actgaaaata tggaaagaaa ctgcagggct 3240

ccctgcaata tccagatgga agatcccact tttaaagaga attatcgctt ccatgcaatc 3300

aatggctaca taatggatac actacctggc ttagtaatgg ctcaggatca aaggattcga 3360

tggtatctgc tcagcatggg cagcaatgaa aacatccatt ctattcattt cagtggacat 3420

gtgttcactg tacgaaaaaa agaggagtat aaaatggcac tgtacaatct ctatccaggt 3480

gtttttgaga cagtggaaat gttaccatcc aaagctggaa tttggcgggt ggaatgcctt 3540

attggcgagc atctacatgc tgggatgagc acactttttc tggtgtacag caataagtgt 3600

cagactcccc tgggaatggc ttctggacac attagagatt ttcagattac agcttcagga 3660

caatatggac agtgggcccc aaagctggcc agacttcatt attccggatc aatcaatgcc 3720

tggagcacca aggagccctt ttcttggatc aaggtggatc tgttggcacc aatgattatt 3780

cacggcatca agacccaggg tgcccgtcag aagttctcca gcctctacat ctctcagttt 3840

atcatcatgt atagtcttga tgggaagaag tggcagactt atcgaggaaa ttccactgga 3900

accttaatgg tcttctttgg caatgtggat tcatctggga taaaacacaa tatttttaac 3960

cctccaatta ttgctcgata catccgtttg cacccaactc attatagcat tcgcagcact 4020

cttcgcatgg agttgatggg ctgtgattta aatagttgca gcatgccatt gggaatggag 4080

agtaaagcaa tatcagatgc acagattact gcttcatcct actttaccaa tatgtttgcc 4140

acctggtctc cttcaaaagc tcgacttcac ctccaaggga ggagtaatgc ctggagacct 4200

caggtgaata atccaaaaga gtggctgcaa gtggacttcc agaagacaat gaaagtcaca 4260

ggagtaacta ctcagggagt aaaatctctg cttaccagca tgtatgtgaa ggagttcctc 4320

atctccagca gtcaagatgg ccatcagtgg actctctttt ttcagaatgg caaagtaaag 4380

gtttttcagg gaaatcaaga ctccttcaca cctgtggtga actctctaga cccaccgtta 4440

ctgactcgct accttcgaat tcacccccag agttgggtgc accagattgc cctgaggatg 4500

gaggttctgg gctgcgaggc acaggacctc tactga 4536

<210> 13
<211> 4935
<212> DNA
<213> Artificial Sequence

<220>
<223> G6

<400> 13
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagttcct taggaccccc aagtatgcca gttcattatg atagtcaatt agataccact 2760

ctatttggca aaaagtcatc tccccttact gagtctggtg gacctctgag cttgagtgaa 2820

gaaaataatg attcaaagtt gttagaatca ggtttaatga atagccaaga aagttcatgg 2880

ggaaaaaatg tatcgactct tcagtcagat caagaggaaa ttgactatga tgataccata 2940

tcagttgaaa tgaagaagga agattttgac atttatgatg aggatgaaaa tcagagcccc 3000

cgcagctttc aaaagaaaac acgacactat tttattgctg cagtggagag gctctgggat 3060

tatgggatga gtagctcccc acatgttcta agaaacaggg ctcagagtgg cagtgtccct 3120

cagttcaaga aagttgtttt ccaggaattt actgatggct cctttactca gcccttatac 3180

cgtggagaac taaatgaaca tttgggactc ctggggccat atataagagc agaagttgaa 3240

gataatatca tggtaacttt cagaaatcag gcctctcgtc cctattcctt ctattctagc 3300

cttatttctt atgaggaaga tcagaggcaa ggagcagaac ctagaaaaaa ctttgtcaag 3360

cctaatgaaa ccaaaactta cttttggaaa gtgcaacatc atatggcacc cactaaagat 3420

gagtttgact gcaaagcctg ggcttatttc tctgatgttg acctggaaaa agatgtgcac 3480

tcaggcctga ttggacccct tctggtctgc cacactaaca cactgaaccc tgctcatggg 3540

agacaagtga cagtacagga atttgctctg tttttcacca tctttgatga gaccaaaagc 3600

tggtacttca ctgaaaatat ggaaagaaac tgcagggctc cctgcaatat ccagatggaa 3660

gatcccactt ttaaagagaa ttatcgcttc catgcaatca atggctacat aatggataca 3720

ctacctggct tagtaatggc tcaggatcaa aggattcgat ggtatctgct cagcatgggc 3780

agcaatgaaa acatccattc tattcatttc agtggacatg tgttcactgt acgaaaaaaa 3840

gaggagtata aaatggcact gtacaatctc tatccaggtg tttttgagac agtggaaatg 3900

ttaccatcca aagctggaat ttggcgggtg gaatgcctta ttggcgagca tctacatgct 3960

gggatgagca cactttttct ggtgtacagc aataagtgtc agactcccct gggaatggct 4020

tctggacaca ttagagattt tcagattaca gcttcaggac aatatggaca gtgggcccca 4080

aagctggcca gacttcatta ttccggatca atcaatgcct ggagcaccaa ggagcccttt 4140

tcttggatca aggtggatct gttggcacca atgattattc acggcatcaa gacccagggt 4200

gcccgtcaga agttctccag cctctacatc tctcagttta tcatcatgta tagtcttgat 4260

gggaagaagt ggcagactta tcgaggaaat tccactggaa ccttaatggt cttctttggc 4320

aatgtggatt catctgggat aaaacacaat atttttaacc ctccaattat tgctcgatac 4380

atccgtttgc acccaactca ttatagcatt cgcagcactc ttcgcatgga gttgatgggc 4440

tgtgatttaa atagttgcag catgccattg ggaatggaga gtaaagcaat atcagatgca 4500

cagattactg cttcatccta ctttaccaat atgtttgcca cctggtctcc ttcaaaagct 4560

cgacttcacc tccaagggag gagtaatgcc tggagacctc aggtgaataa tccaaaagag 4620

tggctgcaag tggacttcca gaagacaatg aaagtcacag gagtaactac tcagggagta 4680

aaatctctgc ttaccagcat gtatgtgaag gagttcctca tctccagcag tcaagatggc 4740

catcagtgga ctctcttttt tcagaatggc aaagtaaagg tttttcaggg aaatcaagac 4800

tccttcacac ctgtggtgaa ctctctagac ccaccgttac tgactcgcta ccttcgaatt 4860

cacccccaga gttgggtgca ccagattgcc ctgaggatgg aggttctggg ctgcgaggca 4920

caggacctct actga 4935

<210> 14
<211> 4764
<212> DNA
<213> Artificial Sequence

<220>
<223> G4-flex

<400> 14
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagttctc aaaacccacc agtcactctt cagtcagatc aagaggaaat tgactatgat 2760

gataccatat cagttgaaat gaagaaggaa gattttgaca tttatgatga ggatgaaaat 2820

cagagccccc gcagctttca aaagaaaaca cgacactatt ttattgctgc agtggagagg 2880

ctctgggatt atgggatgag tagctcccca catgttctaa gaaacagggc tcagagtggc 2940

agtgtccctc agttcaagaa agttgttttc caggaattta ctgatggctc ctttactcag 3000

cccttatacc gtggagaact aaatgaacat ttgggactcc tggggccata tataagagca 3060

gaagttgaag ataatatcat ggtaactttc agaaatcagg cctctcgtcc ctattccttc 3120

tattctagcc ttatttctta tgaggaagat cagaggcaag gagcagaacc tagaaaaaac 3180

tttgtcaagc ctaatgaaac caaaacttac ttttggaaag tgcaacatca tatggcaccc 3240

actaaagatg agtttgactg caaagcctgg gcttatttct ctgatgttga cctggaaaaa 3300

gatgtgcact caggcctgat tggacccctt ctggtctgcc acactaacac actgaaccct 3360

gctcatggga gacaagtgac agtacaggaa tttgctctgt ttttcaccat ctttgatgag 3420

accaaaagct ggtacttcac tgaaaatatg gaaagaaact gcagggctcc ctgcaatatc 3480

cagatggaag atcccacttt taaagagaat tatcgcttcc atgcaatcaa tggctacata 3540

atggatacac tacctggctt agtaatggct caggatcaaa ggattcgatg gtatctgctc 3600

agcatgggca gcaatgaaaa catccattct attcatttca gtggacatgt gttcactgta 3660

cgaaaaaaag aggagtataa aatggcactg tacaatctct atccaggtgt ttttgagaca 3720

gtggaaatgt taccatccaa agctggaatt tggcgggtgg aatgccttat tggcgagcat 3780

ctacatgctg ggatgagcac actttttctg gtgtacagca ataagtgtca gactcccctg 3840

ggaatggctt ctggacacat tagagatttt cagattacag cttcaggaca atatggacag 3900

tgggccccaa agctggccag acttcattat tccggatcaa tcaatgcctg gagcaccaag 3960

gagccctttt cttggatcaa ggtggatctg ttggcaccaa tgattattca cggcatcaag 4020

acccagggtg cccgtcagaa gttctccagc ctctacatct ctcagtttat catcatgtat 4080

agtcttgatg ggaagaagtg gcagacttat cgaggaaatt ccactggaac cttaatggtc 4140

ttctttggca atgtggattc atctgggata aaacacaata tttttaaccc tccaattatt 4200

gctcgataca tccgtttgca cccaactcat tatagcattc gcagcactct tcgcatggag 4260

ttgatgggct gtgatttaaa tagttgcagc atgccattgg gaatggagag taaagcaata 4320

tcagatgcac agattactgc ttcatcctac tttaccaata tgtttgccac ctggtctcct 4380

tcaaaagctc gacttcacct ccaagggagg agtaatgcct ggagacctca ggtgaataat 4440

ccaaaagagt ggctgcaagt ggacttccag aagacaatga aagtcacagg agtaactact 4500

cagggagtaa aatctctgct taccagcatg tatgtgaagg agttcctcat ctccagcagt 4560

caagatggcc atcagtggac tctctttttt cagaatggca aagtaaaggt ttttcaggga 4620

aatcaagact ccttcacacc tgtggtgaac tctctagacc caccgttact gactcgctac 4680

cttcgaattc acccccagag ttgggtgcac cagattgccc tgaggatgga ggttctgggc 4740

tgcgaggcac aggacctcta ctga 4764

<210> 15
<211> 4945
<212> DNA
<213> Artificial Sequence

<220>
<223> G6_opt

<400> 15
ctgcttctcc gccacccggc ggtactacct gggcgccgtg gaactgtcct gggactacat 60

gcagtccgac ctgggcgagc tgcccgtgga cgccagattc cccccaagag tgcccaagtc 120

cttccccttc aacacctccg tggtgtacaa gaaaaccctg ttcgtggaat tcaccgacca 180

cctgttcaat atcgccaagc ctcggccccc ttggatgggc ctgctgggcc ctacaatcca 240

ggccgaggtg tacgacaccg tggtgatcac cctgaagaac atggcctccc accccgtgtc 300

cctgcacgcc gtgggcgtgt cctactggaa ggcctctgag ggcgccgagt acgacgacca 360

gacctcccag cgcgagaaag aggacgacaa ggtgttccct ggcggctccc acacctacgt 420

gtggcaggtg ctgaaagaaa acggccctat ggcctccgac cctctgtgcc tgacctactc 480

ctacctgtcc cacgtggacc tggtgaaaga cctgaactcc ggcctgatcg gcgccctgct 540

ggtgtgcaga gagggctccc tggccaaaga aaagacccag accctgcaca agttcatcct 600

gctgttcgcc gtgttcgacg agggcaagtc ctggcactcc gagacaaaga actccctgat 660

gcaggaccgg gacgccgcct ctgccagagc ctggcctaag atgcacaccg tgaacggcta 720

cgtgaaccgg tccctgcccg gactgatcgg ctgccaccgg aagtccgtgt actggcacgt 780

gatcggcatg ggcaccaccc ccgaggtgca ctccatcttt ctggaaggcc acacctttct 840

ggtgcgaaac caccggcagg cctccctgga aatctcccct atcaccttcc tgaccgctca 900

gaccctgctg atggacctgg gccagttcct gctgttttgc cacatctcca gccaccagca 960

cgacggcatg gaagcctacg tgaaggtgga ctcctgcccc gaggaacccc agctgcggat 1020

gaagaacaac gaggaagccg aggactacga cgacgacctg accgactccg agatggacgt 1080

ggtgcgattc gacgacgaca actcccccag cttcatccag atcagatccg tggccaagaa 1140

gcaccccaag acctgggtgc actatatcgc cgccgaggaa gaggactggg actacgcccc 1200

tctggtgctg gcccctgacg accggtccta caagtcccag tacctgaaca acggccccca 1260

gcggatcggc cggaagtaca agaaagtgcg gttcatggcc tacaccgacg agacattcaa 1320

gaccagagag gccatccagc acgagtccgg catcctgggc cccctgctgt acggcgaagt 1380

gggcgacaca ctgctgatca tcttcaagaa ccaggcctcc cggccctaca acatctaccc 1440

ccacggcatc accgacgtgc ggcccctgta ctctcggcgg ctgcctaagg gcgtgaagca 1500

cctgaaggac ttccccatcc tgcccggcga gatcttcaag tacaagtgga ccgtgaccgt 1560

ggaagatggc cccaccaagt ccgaccccag atgcctgacc cggtactact cctccttcgt 1620

gaacatggaa cgggacctgg ccagcggcct gattggacct ctgctgatct gctacaaaga 1680

atccgtggac cagcggggca accagatcat gtccgacaag cggaacgtga tcctgttcag 1740

cgtgttcgat gagaaccggt cctggtatct gaccgagaac atccagcggt ttctgcccaa 1800

ccctgccggc gtgcagctgg aagatcccga gttccaggcc tccaacatca tgcacagcat 1860

caatggctac gtgttcgact ccctgcagct gagcgtgtgc ctgcacgagg tggcctactg 1920

gtacatcctg tccatcggcg cccagaccga cttcctgtcc gtgttcttct ccggctacac 1980

cttcaagcac aagatggtgt acgaggatac cctgaccctg ttccccttct ccggcgaaac 2040

cgtgttcatg tccatggaaa accccggcct gtggatcctg ggctgccaca actccgactt 2100

ccggaaccgg ggcatgaccg ccctgctgaa ggtgtcctcc tgcgacaaga acaccggcga 2160

ctactacgag gactcttacg aggacatctc cgcctacctg ctgtccaaga acaacgccat 2220

cgagccccgg tccttcagcc agaactcccg gcacccttcc acccggcaga agcagttcaa 2280

cgccaccacc atccccgaga acgacatcga aaagaccgac ccttggtttg cccaccggac 2340

ccccatgccc aagatccaga acgtgtcctc ctctgacctg ctgatgctgc tgcggcagtc 2400

ccccacccct cacggcctgt ctctgagcga cctgcaggaa gctaagtacg agactttctc 2460

cgacgacccc tcccctggcg ccatcgactc caacaactcc ctgtccgaga tgacccactt 2520

ccggccccag ctgcaccact ccggcgacat ggtgttcacc cccgagtctg gcctgcagct 2580

gcggctgaac gagaagctgg gcaccacagc cgccaccgag ctgaagaaac tggacttcaa 2640

ggtgtccagc acctccaaca acctgatctc caccatccct tccgacaacc tggccgctgg 2700

caccgacaac acctcttccc tgggccctcc ctccatgccc gtgcactacg actcccagct 2760

ggacaccacc ctgttcggca agaagtccag ccccctgacc gagtccggcg gacccctgtc 2820

cctgtctgag gaaaacaacg actccaagct gctggaatct ggcctgatga actcccagga 2880

atcctcctgg ggcaagaatg tgtccaccct gcagagcgac caggaagaga tcgattacga 2940

cgacaccatc tccgtggaaa tgaagaaaga ggatttcgac atctacgacg aggacgagaa 3000

ccagtcccct cggtccttcc agaagaaaac ccggcactac tttatcgccg ctgtggaacg 3060

gctgtgggac tacggcatgt cctcctcccc ccacgtgctg cggaacagag cccagtccgg 3120

ctccgtgccc cagttcaaga aagtggtgtt ccaggaattc acagacggca gcttcaccca 3180

gcccctgtac cggggcgagc tgaatgagca cctgggactg ctgggacctt acatcagagc 3240

tgaggtggaa gataacatca tggtgacatt ccggaaccag gccagcagac cctactcctt 3300

ctactccagc ctgatctcct acgaagagga ccagcggcag ggcgctgagc cccggaagaa 3360

cttcgtgaag cccaacgaga ctaagaccta cttctggaag gtgcagcacc acatggctcc 3420

taccaaggac gagttcgact gcaaggcctg ggcctacttc tccgatgtgg acctggaaaa 3480

ggacgtgcac agcgggctga ttggcccact gctggtgtgc cacaccaaca ccctgaaccc 3540

cgcccacggc agacaggtga cagtgcagga attcgccctg ttcttcacca tcttcgacga 3600

gacaaagtcc tggtacttca ccgagaatat ggaacggaac tgcagagccc cctgcaacat 3660

ccagatggaa gatcctacct tcaaagagaa ctaccggttc cacgccatca acggctacat 3720

catggacacc ctgcccggcc tggtgatggc ccaggaccag agaatccggt ggtatctgct 3780

gtccatgggc tccaacgaga atatccactc catccacttc tccggccacg tgttcaccgt 3840

gcggaagaaa gaagagtaca agatggccct gtacaacctg taccccggcg tgttcgagac 3900

agtggaaatg ctgccctcca aggccggcat ctggcgggtg gaatgtctga tcggcgagca 3960

tctgcacgcc ggaatgtcta ccctgttcct ggtgtactcc aacaagtgcc agacccccct 4020

gggcatggcc tccggccaca tccgggattt ccagatcacc gcctctggcc agtacggcca 4080

gtgggcccct aagctggccc ggctgcacta cagcggctcc atcaacgcct ggtctaccaa 4140

agagcccttc agctggatca aggtggacct gctggccccc atgatcatcc atggcatcaa 4200

gacccagggc gccagacaga agttctcctc cctgtacatc tcccagttca tcatcatgta 4260

ctccctggat ggcaagaagt ggcagaccta ccggggcaac tccaccggca ccctgatggt 4320

gttcttcggc aacgtggact cctccggcat caagcacaac atcttcaacc cccccatcat 4380

tgcccggtac atccggctgc accccaccca ctactccatc cggtctaccc tgcggatgga 4440

actgatgggc tgcgacctga acagctgctc catgcctctg ggcatggaat ccaaggccat 4500

ctccgacgcc cagatcacag ccagctccta cttcaccaac atgttcgcca cctggtcccc 4560

cagcaaggcc agactgcatc tgcagggccg gtccaacgct tggcggcctc aggtgaacaa 4620

ccccaaagaa tggctgcagg tggacttcca gaaaaccatg aaggtgacag gcgtgaccac 4680

ccagggcgtg aaatccctgc tgacctctat gtacgtgaaa gagttcctga tcagctcctc 4740

ccaggacggc caccagtgga ccctgttctt tcagaacggc aaagtgaaag tgttccaggg 4800

caaccaggac tccttcaccc ctgtggtgaa ctccctggac cccccactgc tgaccagata 4860

cctgcggatc cacccccagt cttgggtgca ccagatcgcc ctgagaatgg aagtgctggg 4920

atgcgaggcc caggatctgt actga 4945

<210> 16
<211> 4335
<212> DNA
<213> Artificial Sequence

<220>

<223>    G1

<400>    16
gccaccagaa gatactacct gggtgcagtg gaactgtcat gggactatat gcaaagtgat      60
ctcggtgagc tgcctgtgga cgcaagattt cctcctagag tgccaaaatc ttttccattc     120
aacacctcag tcgtgtacaa aaagactctg tttgtagaat tcacggatca ccttttcaac    180
atcgctaagc caaggccacc ctggatgggt ctgctaggtc ctaccatcca ggctgaggtt     240
tatgatacag tggtcattac acttaagaac atggcttccc atcctgtcag tcttcatgct     300
gttggtgtat cctactggaa agcttctgag ggagctgaat atgatgatca gaccagtcaa     360
agggagaaag aagatgataa agtcttccct ggtggaagcc atacatatgt ctggcaggtc     420
ctgaaagaga atggtccaat ggcctctgac ccactgtgcc ttacctactc atatctttct     480
catgtggacc tggtaaaaga cttgaattca ggcctcattg gagccctact agtatgtaga     540
gaagggagtc tggccaagga aaagacacag accttgcaca aatttatact actttttgct     600
gtatttgatg aagggaaaag ttggcactca gaaacaaaga actccttgat gcaggatagg     660
gatgctgcat ctgctcgggc ctggcctaaa atgcacacag tcaatggtta tgtaaacagg     720
tctctgccag gtctgattgg atgccacagg aaatcagtct attggcatgt gattggaatg     780
ggcaccactc ctgaagtgca ctcaatattc ctcgaaggtc acacatttct tgtgaggaac     840
catcgccagg cgtccttgga aatctcgcca ataactttcc ttactgctca aacactcttg     900
atggaccttg gacagtttct actgttttgt catatctctt cccaccaaca tgatggcatg     960
gaagcttatg tcaaagtaga cagctgtcca gaggaacccc aactacgaat gaaaaataat    1020
gaagaagcgg aagactatga tgatgatctt actgattctg aaatggatgt ggtcaggttt    1080
gatgatgaca actctccttc ctttatccaa attcgctcag ttgccaagaa gcatcctaaa    1140
acttgggtac attacattgc tgctgaagag gaggactggg actatgctcc cttagtcctc    1200
gcccccgatg acagaagtta taaaagtcaa tatttgaaca atggccctca gcggattggt    1260
aggaagtaca aaaaagtccg atttatggca tacacagatg aaacctttaa gactcgtgaa    1320
gctattcagc atgaatcagg aatcttggga cctttacttt atggggaagt tggagacaca    1380
ctgttgatta tatttaagaa tcaagcaagc agaccatata acatctaccc tcacggaatc    1440
actgatgtcc gtcctttgta ttcaaggaga ttaccaaaag gtgtaaaaca tttgaaggat    1500
tttccaattc tgccaggaga aatattcaaa tataaatgga cagtgactgt agaagatggg    1560
ccaactaaat cagatcctcg gtgcctgacc cgctattact ctagtttcgt taatatggag    1620
agagatctag cttcaggact cattggccct ctcctcatct gctacaaaga atctgtagat    1680
caaagaggaa accagataat gtcagacaag aggaatgtca tcctgttttc tgtatttgat     1740
gagaaccgaa gctggtacct cacagagaat atacaacgct ttctccccaa tccagctgga    1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atactactct tcagtcagat caagaggaaa ttgactatga tgataccata 2340

tcagttgaaa tgaagaagga agattttgac atttatgatg aggatgaaaa tcagagcccc 2400

cgcagctttc aaaagaaaac acgacactat tttattgctg cagtggagag gctctgggat 2460

tatgggatga gtagctcccc acatgttcta agaaacaggg ctcagagtgg cagtgtccct 2520

cagttcaaga aagttgtttt ccaggaattt actgatggct cctttactca gcccttatac 2580

cgtggagaac taaatgaaca tttgggactc ctggggccat atataagagc agaagttgaa 2640

gataatatca tggtaacttt cagaaatcag gcctctcgtc cctattcctt ctattctagc 2700

cttatttctt atgaggaaga tcagaggcaa ggagcagaac ctagaaaaaa ctttgtcaag 2760

cctaatgaaa ccaaaactta cttttggaaa gtgcaacatc atatggcacc cactaaagat 2820

gagtttgact gcaaagcctg ggcttatttc tctgatgttg acctggaaaa agatgtgcac 2880

tcaggcctga ttggacccct tctggtctgc cacactaaca cactgaaccc tgctcatggg 2940

agacaagtga cagtacagga atttgctctg tttttcacca tctttgatga gaccaaaagc 3000

tggtacttca ctgaaaatat ggaaagaaac tgcagggctc cctgcaatat ccagatggaa 3060

gatcccactt ttaaagagaa ttatcgcttc catgcaatca atggctacat aatggataca 3120

ctacctggct tagtaatggc tcaggatcaa aggattcgat ggtatctgct cagcatgggc 3180

agcaatgaaa acatccattc tattcatttc agtggacatg tgttcactgt acgaaaaaaa 3240

gaggagtata aaatggcact gtacaatctc tatccaggtg tttttgagac agtggaaatg 3300

ttaccatcca aagctggaat ttggcgggtg gaatgcctta ttggcgagca tctacatgct 3360

gggatgagca cactttttct ggtgtacagc aataagtgtc agactcccct gggaatggct 3420

tctggacaca ttagagattt tcagattaca gcttcaggac aatatggaca gtggccccca 3480

aagctggcca gacttcatta ttccggatca atcaatgcct ggagcaccaa ggagcccttt 3540

tcttggatca aggtggatct gttggcacca atgattattc acggcatcaa gacccagggt 3600

gcccgtcaga agttctccag cctctacatc tctcagttta tcatcatgta tagtcttgat 3660

gggaagaagt ggcagactta tcgaggaaat tccactggaa ccttaatggt cttctttggc 3720

aatgtggatt catctgggat aaaacacaat atttttaacc ctccaattat tgctcgatac 3780

atccgtttgc acccaactca ttatagcatt cgcagcactc ttcgcatgga gttgatgggc 3840

tgtgatttaa atagttgcag catgccattg ggaatggaga gtaaagcaat atcagatgca 3900

cagattactg cttcatccta ctttaccaat atgtttgcca cctggtctcc ttcaaaagct 3960

cgacttcacc tccaagggag gagtaatgcc tggagacctc aggtgaataa tccaaaagag 4020

tggctgcaag tggacttcca gaagacaatg aaagtcacag gagtaactac tcagggagta 4080

aaatctctgc ttaccagcat gtatgtgaag gagttcctca tctccagcag tcaagatggc 4140

catcagtgga ctctcttttt tcagaatggc aaagtaaagg tttttcaggg aaatcaagac 4200

tccttcacac ctgtggtgaa ctctctagac ccaccgttac tgactcgcta ccttcgaatt 4260

cacccccaga gttgggtgca ccagattgcc ctgaggatgg aggttctggg ctgcgaggca 4320

caggacctct actga 4335

<210> 17
<211> 4746
<212> DNA
<213> Artificial Sequence

<220>
<223> G4_A2_1

<400> 17
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga tgtccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 18
<211> 4746
<212> DNA
<213> Artificial Sequence

<220>
<223> G4_A2_2

<400> 18
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gttgtaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 19
<211> 4803
<212> DNA
<213> Artificial Sequence

<220>
<223> G4_A2_3

<400> 19
agaggaatgt catcctgttt tctgtatttg atgagaaccg aagctggtac ctcacagaga 60

atatacaacg ctttctcccc aatccagctg gagtgcagct tgaggatcca gagttccaag 120

cctccaacat catgcacagc atcaatggct atgtttttga tagtttgcag ttgtcagttt 180

gtttgcatga ggtggcatac tggtacattc taagcattgg agcacagact gacttccttt 240

ctgtcttctt ctctggatat accttcaaac acaaaatggt ctatgaagac acactcaccc 300

tattcccatt ctcaggagaa actgtcttca tgtcgatgga aaacccaggt ctatggattc 360

tggggtgcca caactcagac tttcggaaca gaggcatgac cgccttactg aaggtttcta 420

gttgtgacaa gaacactggt gattattacg aggacagtta tgaagatatt tcagcatact 480

tgctgagtaa aaacaatgcc attgaaccaa gaagcttctc ccagaattca agacacccta 540

gcactaggca aaagcaattt aatgccacca caattccaga aaatgacata gagaagactg 600

acccttggtt tgcacacaga acacctatgc ctaaaataca aaatgtctcc tctagtgatt 660

tgttgatgct cttgcgacag agtcctactc cacatgggct atccttatct gatctccaag 720

aagccaaata tgagactttt tctgatgatc catcacctgg agcaatagac agtaataaca 780

gcctgtctga aatgacacac ttcaggccac agctccatca cagtggggac atggtattta 840

cccctgagtc aggcctccaa ttaagattaa atgagaaact ggggacaact gcagcaacag 900

agttgaagaa acttgatttc aaagtttcta gtacatcaaa taatctgatt tcaacaattc 960

catcagacaa tttggcagca ggtactgata atacaagtac tcttcagtca gatcaagagg 1020

aaattgacta tgatgatacc atatcagttg aaatgaagaa ggaagatttt gacatttatg 1080

atgaggatga aaatcagagc ccccgcagct ttcaaaagaa aacacgacac tattttattg 1140

ctgcagtgga gaggctctgg gattatggga tgagtagctc cccacatgtt ctaagaaaca 1200

gggctcagag tggcagtgtc cctcagttca agaaagttgt tttccaggaa tttactgatg 1260

gctcctttac tcagccctta taccgtggag aactaaatga acatttggga ctcctggggc 1320

catatataag agcagaagtt gaagataata tcatggtaac tttcagaaat caggcctctc 1380

gtccctattc cttctattct agccttattt cttatgagga agatcagagg caaggagcag 1440

aacctagaaa aaactttgtc aagcctaatg aaaccaaaac ttacttttgg aaagtgcaac 1500

atcatatggc acccactaaa gatgagtttg actgcaaagc ctgggcttat ttctctgatg 1560

ttgacctgga aaaagatgtg cactcaggcc tgattggacc ccttctggtc tgccacacta 1620

acacactgaa ccctgctcat gggagacaag tgacagtaca ggaatttgct ctgtttttca 1680

ccatctttga tgagaccaaa agctggtact tcactgaaaa tatggaaaga aactgcaggg 1740

ctccctgcaa tatccagatg gaagatccca cttttaaaga gaattatcgc ttccatgcaa 1800

tcaatggcta cataatggat acactacctg gcttagtaat ggctcaggat caaaggattc 1860

gatggtatct gctcagcatg ggcagcaatg aaaacatcca ttctattcat ttcagtggac 1920

atgtgttcac tgtacgaaaa aaagaggagt ataaaatggc actgtacaat ctctatccag 1980

gtgtttttga gacagtggaa atgttaccat ccaaagctgg aatttggcgg gtggaatgcc 2040

ttattggcga gcatctacat gctgggatga gcacactttt tctggtgtac agcaataagt 2100

gtcagactcc cctgggaatg gcttctggac acattagaga ttttcagatt acagcttcag 2160

gacaatatgg acagtgggcc ccaaagctgg ccagacttca ttattccgga tcaatcaatg 2220

cctggagcac caaggagccc ttttcttgga tcaaggtgga tctgttggca ccaatgatta 2280

ttcacggcat caagacccag ggtgcccgtc agaagttctc cagcctctac atctctcagt 2340

ttatcatcat gtatagtctt gatgggaaga agtggcagac ttatcgagga aattccactg 2400

gaaccttaat ggtcttcttt ggcaatgtgg attcatctgg gataaaacac aatattttta 2460

accctccaat tattgctcga tacatccgtt tgcacccaac tcattatagc attcgcagca 2520

ctcttcgcat ggagttgatg ggctgtgatt taaatagttg cagcatgcca ttgggaatgg 2580

agagtaaagc aatatcagat gcacagatta ctgcttcatc ctactttacc aatatgtttg 2640

ccacctggtc tccttcaaaa gctcgacttc acctccaagg gaggagtaat gcctggagac 2700

ctcaggtgaa taatccaaaa gagtggctgc aagtggactt ccagaagaca atgaaagtca 2760

caggagtaac tactcaggga gtaaaatctc tgcttaccag catgtatgtg aaggagttcc 2820

tcatctccag cagtcaagat ggccatcagt ggactctctt ttttcagaat ggcaaagtaa 2880

aggtttttca gggaaatcaa gactccttca cacctgtggt gaactctcta gacccaccgt 2940

tactgactcg ctaccttcga attcaccccc agagttgggt gcaccagatt gccctgagga 3000

tggaggttct gggctgcgag gcacaggacc tctactgaat gcaaatagag ctctccacct 3060

gcttctttct gtgccttttg cgattctgct ttagtgccac cagaagatac tacctgggtg 3120

cagtggaact gtcatgggac tatatgcaaa gtgatctcgg tgagctgcct gtggacgcaa 3180

gatttcctcc tagagtgcca aaatcttttc cattcaacac ctcagtcgtg tacaaaaaga 3240

ctctgtttgt agaattcacg gatcaccttt tcaacatcgc taagccaagg ccaccctgga 3300

tgggtctgct aggtcctacc atccaggctg aggtttatga tacagtggtc attacactta 3360

agaacatggc ttcccatcct gtcagtcttc atgctgttgg tgtatcctac tggaaagctt 3420

ctgagggagc tgaatatgat gatcagacca gtcaaaggga gaaagaagat gataaagtct 3480

tccctggtgg aagccataca tatgtctggc aggtcctgaa agagaatggt ccaatggcct 3540

ctgacccact gtgccttacc tactcatatc tttctcatgt ggacctggta aaagacttga 3600

attcaggcct cattggagcc ctactagtat gtagagaagg gagtctggcc aaggaaaaga 3660

cacagacctt gcacaaattt atactacttt ttgctgtatt tgatgaaggg aaaagttggc 3720

actcagaaac aaagaactcc ttgatgcagg atagggatgc tgcatctgct cgggcctggc 3780

ctaaaatgca cacagtcaat ggttatgtaa acaggtctct gccaggtctg attggatgcc 3840

acaggaaatc agtctattgg catgtgattg gaatgggcac cactcctgaa gtgcactcaa 3900

tattcctcga aggtcacaca tttcttgtga ggaaccatcg ccaggcgtcc ttggaaatct 3960

cgccaataac tttccttact gctcaaacac tcttgatgga ccttggacag tttctactgt 4020

tttgtcatat ctcttcccac caacatgatg gcatggaagc ttatgtcaaa gtagacagct 4080

gtccagagga accccaacta cgaatgaaaa ataatgaaga agcggaagac tatgatgatg 4140

atcttactga ttctgaaatg gatgtggtca ggtttgatga tgacaactct ccttccttta 4200

tccaaattcg ctcagttgcc aagaagcatc ctaaaacttg ggtacattac attgctgctg 4260

aagaggagga ctgggactat gctcccttag tcctcgcccc cgatgacaga agttataaaa 4320

gtcaatattt gaacaatggc cctcagcgga ttggtaggaa gtacaaaaaa gtccgattta 4380

tggcatacac agatgaaacc tttaagactc gtgaagctat tcagcatgaa tcaggaatct 4440

tgggaccttt actttatggg gaagttggag acacactgtt gattatattt aagaatcaag 4500

caagcagacc atataacatc taccctcacg gaatcactga tgtccgtcct ttgtattcaa 4560

ggagattacc aaaaggtgta aaacattgta aggattttcc aattctgcca ggagaaatat 4620

tcaaatataa atggacagtg actgtagaag atgggccaac taaatcagat cctcggtgcc 4680

tgacccgcta ttactctagt ttcgttaata tggagagaga tctagcttca ggactcattg 4740

gccctctcct catctgctac aaagaatctg tagatcaaag aggaaaccag ataatgtcag 4800

aca 4803

<210> 20
<211> 4746
<212> DNA
<213> Artificial Sequence

<220>
<223> G4_A3_1

<400> 20
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actgtgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 21
<211> 4746
<212> DNA
<213> Artificial Sequence

<220>
<223> G4_B1

<400> 21
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140
acttgggtac attacattgc tgctgaagag gaggactggg actatgctcc cttagtcctc 1200
gcccccgatg acagaagtta taaaagtcaa tatttgaaca atggccctca gcggattggt 1260
aggaagtaca aaaaagtccg atttatggca tacacagatg aaacctttaa gactcgtgaa 1320
gctattcagc atgaatcagg aatcttggga cctttacttt atggggaagt tggagacaca 1380
ctgttgatta tatttaagaa tcaagcaagc agaccatata acatctaccc tcacggaatc 1440
actgatgtcc gtcctttgta ttcaaggaga ttaccaaaag gtgtaaaaca tttgaaggat 1500
tttccaattc tgccaggaga aatattcaaa tataaatgga cagtgactgt agaagatggg 1560
ccaactaaat cagatcctcg gtgcctgacc cgctattact ctagtttcgt taatatggag 1620
agagatctag cttcaggact cattggccct ctcctcatct gctacaaaga atctgtagat 1680
caaagaggaa accagataat gtcagacaag aggaatgtca tcctgttttc tgtatttgat 1740
gagaaccgaa gctggtacct cacagagaat atacaacgct ttctccccaa tccagctgga 1800
gtgcagcttg aggatccaga gttccaagcc tccaacatca tgcacagcat caatggctat 1860
gtttttgata gtttgcagtt gtcagtttgt ttgcatgagg tggcatactg gtacattcta 1920
agcattggag cacagactga cttcctttct gtcttcttct ctggatatac cttcaaacac 1980
aaaatggtct atgaagacac actcacccta ttcccattct caggagaaac tgtcttcatg 2040
tcgatggaaa acccaggtct atggattctg gggtgccaca actcagactt tcggaacaga 2100
ggcatgaccg ccttactgaa ggtttctagt tgtgacaaga acactggtga ttattacgag 2160
gacagttatg aagatatttc agcatacttg ctgagtaaaa acaatgccat tgaaccaaga 2220
agcttctccc agaattcaag acaccctagc actaggcaat gccaatttaa tgccaccaca 2280
attccagaaa atgacataga gaagactgac ccttggtttg cacacagaac acctatgcct 2340
aaaatacaaa atgtctcctc tagtgatttg ttgatgctct tgcgacagag tcctactcca 2400
catgggctat ccttatctga tctccaagaa gccaaatatg agactttttc tgatgatcca 2460
tcacctggag caatagacag taataacagc ctgtctgaaa tgacacactt caggccacag 2520
ctccatcaca gtggggacat ggtatttacc cctgagtcag gcctccaatt aagattaaat 2580
gagaaactgg ggacaactgc agcaacagag ttgaagaaac ttgatttcaa agtttctagt 2640
acatcaaata atctgatttc aacaattcca tcagacaatt tggcagcagg tactgataat 2700
acaagtactc ttcagtcaga tcaagaggaa attgactatg atgataccat atcagttgaa 2760
atgaagaagg aagattttga catttatgat gaggatgaaa atcagagccc ccgcagcttt 2820
caaaagaaaa cacgacacta ttttattgct gcagtggaga ggctctggga ttatgggatg 2880
agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210>    22
<211>    4746
<212>    DNA
<213>    Artificial Sequence

<220>
<223>    G4_1D


<400>    22
gccaccagaa gatactacct gggtgcagtg gaactgtcat gggactatat gcaaagtgat      60

ctcggtgagc tgcctgtgga cgcaagattt cctcctagag tgccaaaatc ttttccattc     120

aacacctcag tcgtgtacaa aaagactctg tttgtagaat tcacggatca ccttttcaac     180

atcgctaagc caaggccacc ctggatgggt ctgctaggtc ctaccatcca ggctgaggtt     240

tatgatacag tggtcattac acttaagaac atggcttccc atcctgtcag tcttcatgct     300

gttggtgtat cctactggaa agcttctgag ggagctgaat atgatgatca gaccagtcaa     360

agggagaaag aagatgataa agtcttccct ggtggaagcc atacatatgt ctggcaggtc     420

ctgaaagaga atggtccaat ggcctctgac ccactgtgcc ttacctactc atatctttct     480

catgtggacc tggtaaaaga cttgaattca ggcctcattg gagccctact agtatgtaga     540

gaagggagtc tggccaagga aaagacacag accttgcaca aatttatact actttttgct     600

gtatttgatg aagggaaaag ttggcactca gaaacaaaga actccttgat gcaggatagg     660

gatgctgcat ctgctcgggc ctggcctaaa atgcacacag tcaatggtta tgtaaacagg     720

tctctgccag gtctgattgg atgccacagg aaatcagtct attggcatgt gattggaatg     780

ggcaccactc ctgaagtgca ctcaatattc ctcgaaggtc acacatttct tgtgaggaac     840

catcgccagg cgtccttgga aatctcgcca ataactttcc ttactgctca aacactcttg     900

atggaccttg gacagtttct actgttttgt catatctctt cccaccaaca tgatggcatg     960

gaagcttatg tcaaagtaga cagctgtcca gaggaacccc aactacgaat gaaaaataat    1020

gaagaagcgg aagactatga tgatgatctt actgattctg aaatggatgt ggtcaggttt    1080

gatgatgaca actctccttc ctttatccaa attcgctcag ttgccaagaa gcatcctaaa    1140

acttgggtac attacattgc tgctgaagag gaggactggg actatgctcc cttagtcctc    1200

gcccccgatg acagaagtta taaaagtcaa tatttgaaca atggccctca gcggattggt    1260

aggaagtaca aaaaagtccg atttatggca tacacagatg aaacctttaa gactcgtgaa    1320

gctattcagc atgaatcagg aatcttggga cctttacttt atggggaagt tggagacaca    1380

ctgttgatta tatttaagaa tcaagcaagc agaccatata acatctaccc tcacggaatc    1440

actgatgtcc gtcctttgta ttcaaggaga ttaccaaaag gtgtaaaaca tttgaaggat    1500

tttccaattc tgccaggaga aatattcaaa tataaatgga cagtgactgt agaagatggg    1560

ccaactaaat cagatcctcg gtgcctgacc cgctattact ctagtttcgt taatatggag    1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa attgtataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtggccccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 23
<211> 4746
<212> DNA
<213> Artificial Sequence

<220>
<223> G4_2D

<400> 23
   gccaccagaa gatactacct gggtgcagtg gaactgtcat gggactatat gcaaagtgat 60
   ctcggtgagc tgcctgtgga cgcaagattt cctcctagag tgccaaaatc ttttccattc 120
   aacacctcag tcgtgtacaa aaagactctg tttgtagaat tcacggatca ccttttcaac 180
   atcgctaagc caaggccacc ctggatgggt ctgctaggtc ctaccatcca ggctgaggtt 240
   tatgatacag tggtcattac acttaagaac atggcttccc atcctgtcag tcttcatgct 300
   gttggtgtat cctactggaa agcttctgag ggagctgaat atgatgatca gaccagtcaa 360
   agggagaaag aagatgataa agtcttccct ggtggaagcc atacatatgt ctggcaggtc 420
   ctgaaagaga atggtccaat ggcctctgac ccactgtgcc ttacctactc atatctttct 480
   catgtggacc tggtaaaaga cttgaattca ggcctcattg gagccctact agtatgtaga 540
   gaagggagtc tggccaagga aaagacacag accttgcaca aatttatact actttttgct 600
   gtatttgatg aagggaaaag ttggcactca gaaacaaaga actccttgat gcaggatagg 660
   gatgctgcat ctgctcgggc ctggcctaaa atgcacacag tcaatggtta tgtaaacagg 720
   tctctgccag gtctgattgg atgccacagg aaatcagtct attggcatgt gattggaatg 780
   ggcaccactc ctgaagtgca ctcaatattc ctcgaaggtc acacatttct tgtgaggaac 840
   catcgccagg cgtccttgga aatctcgcca ataactttcc ttactgctca aacactcttg 900
   atggaccttg gacagtttct actgttttgt catatctctt cccaccaaca tgatggcatg 960
   gaagcttatg tcaaagtaga cagctgtcca gaggaacccc aactacgaat gaaaaataat 1020
   gaagaagcgg aagactatga tgatgatctt actgattctg aaatggatgt ggtcaggttt 1080
   gatgatgaca actctccttc ctttatccaa attcgctcag ttgccaagaa gcatcctaaa 1140
   acttgggtac attacattgc tgctgaagag gaggactggg actatgctcc cttagtcctc 1200
   gcccccgatg acagaagtta taaaagtcaa tatttgaaca atggccctca gcggattggt 1260
   aggaagtaca aaaaagtccg atttatggca tacacagatg aaacctttaa gactcgtgaa 1320
   gctattcagc atgaatcagg aatcttggga cctttacttt atggggaagt tggagacaca 1380
   ctgttgatta tatttaagaa tcaagcaagc agaccatata acatctaccc tcacggaatc 1440
   actgatgtcc gtcctttgta ttcaaggaga ttaccaaaag gtgtaaaaca tttgaaggat 1500
   tttccaattc tgccaggaga aatattcaaa tataaatgga cagtgactgt agaagatggg 1560
   ccaactaaat cagatcctcg gtgcctgacc cgctattact ctagtttcgt taatatggag 1620
   agagatctag cttcaggact cattggccct ctcctcatct gctacaaaga atctgtagat 1680
   caaagaggaa accagataat gtcagacaag aggaatgtca tcctgttttc tgtatttgat 1740
   gagaaccgaa gctggtacct cacagagaat atacaacgct ttctccccaa tccagctgga 1800
   gtgcagcttg aggatccaga gttccaagcc tccaacatca tgcacagcat caatggctat 1860
   gtttttgata gtttgcagtt gtcagtttgt ttgcatgagg tggcatactg gtacattcta 1920
   agcattggag cacagactga cttcctttct gtcttcttct ctggatatac cttcaaacac 1980
   aaaatggtct atgaagacac actcacccta ttcccattct caggagaaac tgtcttcatg 2040
   tcgatggaaa acccaggtct atggattctg gggtgccaca actcagactt tcggaacaga 2100
   ggcatgaccg ccttactgaa ggtttctagt tgtgacaaga acactggtga ttattacgag 2160
   gacagttatg aagatatttc agcatacttg ctgagtaaaa acaatgccat tgaaccaaga 2220
   agcttctccc agaattcaag acaccctagc actaggcaaa agcaatttaa tgccaccaca 2280
   attccagaaa atgacataga gaagacttgt ccttggtttg cacacagaac acctatgcct 2340
   aaaatacaaa atgtctcctc tagtgatttg ttgatgctct tgcgacagag tcctactcca 2400
   catgggctat ccttatctga tctccaagaa gccaaatatg agactttttc tgatgatcca 2460
   tcacctggag caatagacag taataacagc ctgtctgaaa tgacacactt caggccacag 2520
   ctccatcaca gtggggacat ggtatttacc cctgagtcag gcctccaatt aagattaaat 2580
   gagaaactgg ggacaactgc agcaacagag ttgaagaaac ttgatttcaa agtttctagt 2640
   acatcaaata atctgatttc aacaattcca tcagacaatt tggcagcagg tactgataat 2700
   acaagtactc ttcagtcaga tcaagaggaa attgactatg atgataccat atcagttgaa 2760
   atgaagaagg aagattttga catttatgat gaggatgaaa atcagagccc ccgcagcttt 2820
   caaaagaaaa cacgacacta ttttattgct gcagtggaga ggctctggga ttatgggatg 2880
   agtagctccc cacatgttct aagaaacagg gctcagagtg gcagtgtccc tcagttcaag 2940
   aaagttgttt tccaggaatt tactgatggc tcctttactc agcccttata ccgtggagaa 3000
   ctaaatgaac atttgggact cctggggcca tatataagag cagaagttga agataatatc 3060
   atggtaactt tcagaaatca ggcctctcgt ccctattcct tctattctag ccttatttct 3120
   tatgaggaag atcagaggca aggagcagaa cctagaaaaa actttgtcaa gcctaatgaa 3180
   accaaaactt acttttggaa agtgcaacat catatggcac ccactaaaga tgagtttgac 3240
   tgcaaagcct gggcttattt ctctgatgtt gacctggaaa aagatgtgca ctcaggcctg 3300
   attggacccc ttctggtctg ccacactaac acactgaacc ctgctcatgg gagacaagtg 3360
   acagtacagg aatttgctct gtttttcacc atctttgatg agaccaaaag ctggtacttc 3420
   actgaaaata tggaaagaaa ctgcagggct ccctgcaata tccagatgga agatcccact 3480
   tttaaagaga attatcgctt ccatgcaatc aatggctaca taatggatac actacctggc 3540
   ttagtaatgg ctcaggatca aaggattcga tggtatctgc tcagcatggg cagcaatgaa 3600
   aacatccatt ctattcattt cagtggacat gtgttcactg tacgaaaaaa agaggagtat 3660
   aaaatggcac tgtacaatct ctatccaggt gtttttgaga cagtggaaat gttaccatcc 3720
   aaagctggaa tttggcgggt ggaatgcctt attggcgagc atctacatgc tgggatgagc 3780
   acactttttc tggtgtacag caataagtgt cagactcccc tgggaatggc ttctggacac 3840
   attagagatt ttcagattac agcttcagga caatatggac agtggccccc aaagctggcc 3900
   agacttcatt attccggatc aatcaatgcc tggagcacca aggagccctt ttcttggatc 3960
   aaggtggatc tgttggcacc aatgattatt cacggcatca agacccaggg tgcccgtcag 4020
   aagttctcca gcctctacat ctctcagttt atcatcatgt atagtcttga tgggaagaag 4080
   tggcagactt atcgaggaaa ttccactgga accttaatgg tcttctttgg caatgtggat 4140
   tcatctggga taaaacacaa tatttttaac cctccaatta ttgctcgata catccgtttg 4200
   cacccaactc attatagcat tcgcagcact cttcgcatgg agttgatggg ctgtgattta 4260
   aatagttgca gcatgccatt gggaatggag agtaaagcaa tatcagatgc acagattact 4320
   gcttcatcct actttaccaa tatgtttgcc acctggtctc cttcaaaagc tcgacttcac 4380
   ctccaaggga ggagtaatgc ctggagacct caggtgaata atccaaaaga gtggctgcaa 4440
   gtggacttcc agaagacaat gaaagtcaca ggagtaacta ctcagggagt aaaatctctg 4500
   cttaccagca tgtatgtgaa ggagttcctc atctccagca gtcaagatgg ccatcagtgg 4560
   actctctttt ttcagaatgg caaagtaaag gtttttcagg gaaatcaaga ctccttcaca 4620
   cctgtggtga actctctaga cccaccgtta ctgactcgct accttcgaat tcacccccag 4680
   agttgggtgc accagattgc cctgaggatg gaggttctgg gctgcgaggc acaggacctc 4740
   tactga 4746

<210> 24
<211> 4746
<212> DNA
<213> Artificial Sequence

<220>
<223> G4_3R

<400> 24
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacactgtac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 25
<211> 4746
<212> DNA
<213> Artificial Sequence

<220>
<223> G4_B2

<400> 25
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

tgtatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 26
<211> 4746
<212> DNA
<213> Artificial Sequence

<220>
<223> G4_B3

<400>    26
gccaccagaa gatactacct gggtgcagtg gaactgtcat gggactatat gcaaagtgat      60

ctcggtgagc tgcctgtgga cgcaagattt cctcctagag tgccaaaatc ttttccattc     120

aacacctcag tcgtgtacaa aaagactctg tttgtagaat tcacggatca ccttttcaac     180

atcgctaagc caaggccacc ctggatgggt ctgctaggtc ctaccatcca ggctgaggtt     240

tatgatacag tggtcattac acttaagaac atggcttccc atcctgtcag tcttcatgct     300

gttggtgtat cctactggaa agcttctgag ggagctgaat atgatgatca gaccagtcaa     360

agggagaaag aagatgataa agtcttccct ggtggaagcc atacatatgt ctggcaggtc     420

ctgaaagaga atggtccaat ggcctctgac ccactgtgcc ttacctactc atatctttct     480

catgtggacc tggtaaaaga cttgaattca ggcctcattg gagccctact agtatgtaga     540

gaagggagtc tggccaagga aaagacacag accttgcaca aatttatact actttttgct     600

gtatttgatg aagggaaaag ttggcactca gaaacaaaga actccttgat gcaggatagg     660

gatgctgcat ctgctcgggc ctggcctaaa atgcacacag tcaatggtta tgtaaacagg     720

tctctgccag gtctgattgg atgccacagg aaatcagtct attggcatgt gattggaatg     780

ggcaccactc ctgaagtgca ctcaatattc ctcgaaggtc acacatttct tgtgaggaac     840

catcgccagg cgtccttgga aatctcgcca ataactttcc ttactgctca aacactcttg     900

atggaccttg gacagtttct actgttttgt catatctctt cccaccaaca tgatggcatg     960

gaagcttatg tcaaagtaga cagctgtcca gaggaacccc aactacgaat gaaaaataat    1020

gaagaagcgg aagactatga tgatgatctt actgattctg aaatggatgt ggtcaggttt    1080

gatgatgaca actctccttc ctttatccaa attcgctcag ttgccaagaa gcatcctaaa    1140

acttgggtac attacattgc tgctgaagag gaggactggg actatgctcc cttagtcctc    1200

gcccccgatg acagaagtta taaaagtcaa tatttgaaca atggccctca gcggattggt    1260

aggaagtaca aaaaagtccg atttatggca tacacagatg aaacctttaa gactcgtgaa    1320

gctattcagc atgaatcagg aatcttggga cctttacttt atggggaagt tggagacaca    1380

ctgttgatta tatttaagaa tcaagcaagc agaccatata acatctaccc tcacggaatc    1440

actgatgtcc gtcctttgta ttcaaggaga ttaccaaaag gtgtaaaaca tttgaaggat    1500

tttccaattc tgccaggaga aatattcaaa tataaatgga cagtgactgt agaagatggg    1560

ccaactaaat cagatcctcg gtgcctgacc cgctattact ctagtttcgt taatatggag    1620

agagatctag cttcaggact cattggccct ctcctcatct gctacaaaga atctgtagat    1680

caaagaggaa accagataat gtcagacaag aggaatgtca tcctgttttc tgtatttgat    1740

gagaaccgaa gctggtacct cacagagaat atacaacgct ttctccccaa tccagctgga    1800

gtgcagcttg aggatccaga gttccaagcc tccaacatca tgcacagcat caatggctat    1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaatgtcaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 27
<211> 4746
<212> DNA
<213> Artificial Sequence

<220>
<223> G4_B4

<400> 27
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc ttgtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 28
<211> 4746
<212> DNA
<213> Artificial Sequence

<220>
<223> G4_B5

<400> 28
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag catgtgacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210>	29
<211>	4746
<212>	DNA
<213>	Artificial Sequence

<220>
<223>	G4_4L

<400>	29
gccaccagaa gatactacct gggtgcagtg gaactgtcat gggactatat gcaaagtgat	60

ctcggtgagc tgcctgtgga cgcaagattt cctcctagag tgccaaaatc ttttccattc	120

aacacctcag tcgtgtacaa aaagactctg tttgtagaat tcacggatca ccttttcaac	180

atcgctaagc caaggccacc ctggatgggt ctgctaggtc ctaccatcca ggctgaggtt	240

tatgatacag tggtcattac acttaagaac atggcttccc atcctgtcag tcttcatgct	300

gttggtgtat cctactggaa agcttctgag ggagctgaat atgatgatca gaccagtcaa	360

agggagaaag aagatgataa agtcttccct ggtggaagcc atacatatgt ctggcaggtc	420

ctgaaagaga atggtccaat ggcctctgac ccactgtgcc ttacctactc atatctttct	480

catgtggacc tggtaaaaga cttgaattca ggcctcattg gagccctact agtatgtaga	540

gaagggagtc tggccaagga aaagacacag accttgcaca aatttatact actttttgct	600

gtatttgatg aagggaaaag ttggcactca gaaacaaaga actccttgat gcaggatagg	660

gatgctgcat ctgctcgggc ctggcctaaa atgcacacag tcaatggtta tgtaaacagg	720

tctctgccag gtctgattgg atgccacagg aaatcagtct attggcatgt gattggaatg	780

ggcaccactc ctgaagtgca ctcaatattc ctcgaaggtc acacatttct tgtgaggaac	840

catcgccagg cgtccttgga aatctcgcca ataactttcc ttactgctca aacactcttg	900

atggaccttg gacagtttct actgttttgt catatctctt cccaccaaca tgatggcatg	960

gaagcttatg tcaaagtaga cagctgtcca gaggaacccc aactacgaat gaaaaataat	1020

gaagaagcgg aagactatga tgatgatctt actgattctg aaatggatgt ggtcaggttt	1080

gatgatgaca actctccttc ctttatccaa attcgctcag ttgccaagaa gcatcctaaa	1140

acttgggtac attacattgc tgctgaagag gaggactggg actatgctcc cttagtcctc	1200

gcccccgatg acagaagtta taaaagtcaa tatttgaaca atggccctca gcggattggt	1260

aggaagtaca aaaaagtccg atttatggca tacacagatg aaacctttaa gactcgtgaa	1320

gctattcagc atgaatcagg aatcttggga cctttacttt atggggaagt tggagacaca	1380

ctgttgatta tatttaagaa tcaagcaagc agaccatata acatctaccc tcacggaatc	1440

actgatgtcc gtcctttgta ttcaaggaga ttaccaaaag gtgtaaaaca tttgaaggat	1500

tttccaattc tgccaggaga aatattcaaa tataaatgga cagtgactgt agaagatggg	1560

ccaactaaat cagatcctcg gtgcctgacc cgctattact ctagtttcgt taatatggag	1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgattgt ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtggccccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 30
<211> 4746
<212> DNA
<213> Artificial Sequence

<220>
<223> G4_B6

<400> 30
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag catgtgacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 31
<211> 4746
<212> DNA
<213> Artificial Sequence

<220>
<223> G4_B7

<400> 31
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcatg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 32
<211> 4746
<212> DNA
<213> Artificial Sequence

<220>
<223> G4_1I1D

<400> 32
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa attgtataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgatacctg ttcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 33
<211> 1581
<212> PRT
<213> Artificial Sequence

<220>
<223> G4_A2_1

<400>    33
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Cys Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    34
<211>    1581
<212>    PRT
<213>    Artificial Sequence

<220>
<223>    G4_A2_2

<400>    34
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Cys Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    35
<211>    1581
<212>    PRT
<213>    Artificial Sequence

<220>
<223>    G4_A2_3

<400>    35
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Cys Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
        740                 745                 750

Gln Lys Gln Phe Asn Ala Thr Thr Ile Pro Glu Asn Asp Ile Glu Lys
    755                 760                 765

Thr Asp Pro Trp Phe Ala His Arg Thr Pro Met Pro Lys Ile Gln Asn
770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    36
<211>    1581
<212>    PRT
<213>    Artificial Sequence

<220>
<223>    G4_A3_1

<400>    36
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Cys Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    37
<211>    1581
<212>    PRT
<213>    Artificial Sequence

<220>
<223>    G4_B1

<400>    37
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Cys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                    805                 810                 815

Ser Asp Asp Pro Ser Pro Gly Ala Ile Asp Ser Asn Asn Ser Leu Ser
                820                 825                 830

Glu Met Thr His Phe Arg Pro Gln Leu His His Ser Gly Asp Met Val
            835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    38
<211>    1581
<212>    PRT
<213>    Artificial Sequence

<220>
<223>    G4_1D

<400>    38
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Cys Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    39
<211>    1581
<212>    PRT
<213>    Artificial Sequence

<220>
<223>    G4_2D

<400>    39
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Cys Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    40
<211>    1581
<212>    PRT
<213>    Artificial Sequence

<220>
<223>    G4_3R

<400>    40
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Cys Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
1570                1575                1580

<210>   41
<211>   1581
<212>   PRT
<213>   Artificial Sequence

<220>
<223>   G4_B2

<400>   41
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Cys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    42
<211>    1581
<212>    PRT
<213>    Artificial Sequence

<220>
<223>    G4_B3


<400>    42
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Cys Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    43
<211>    1581
<212>    PRT
<213>    Artificial Sequence

<220>

<223>   G4_B4

<400>   43
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Cys Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>   44
<211>   1581
<212>   PRT
<213>   Artificial Sequence

<220>
<223>   G4_B5

<400>   44
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Cys Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580



<210>   45
<211>   1581
<212>   PRT
<213>   Artificial Sequence

<220>
<223>   G4_4L


<400>   45
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
        50                  55                  60

Arg Pro Pro Trp Met Gly Leu Leu Gly Pro Thr Ile Gln Ala Glu Val
 65                  70                  75                  80

Tyr Asp Thr Val Val Ile Thr Leu Lys Asn Met Ala Ser His Pro Val
                 85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Cys Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210> 46
<211> 1581
<212> PRT
<213> Artificial Sequence

<220>
<223> G4_B6

<400> 46
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Cys Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    47
<211>    1581
<212>    PRT
<213>    Artificial Sequence

<220>
<223>    G4_B7

<400>    47
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
        450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Cys Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

<210>    48
<211>    1581
<212>    PRT
<213>    Artificial Sequence

<220>
<223>    G4_1I1D

<400>    48
Ala Thr Arg Arg Tyr Tyr Leu Gly Ala Val Glu Leu Ser Trp Asp Tyr
 1               5                  10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Cys Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Cys Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val
1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys
                1445                1450                1455

Ala Arg Leu His Leu Gln Gly Arg Ser Asn Ala Trp Arg Pro Gln Val
            1460                1465                1470

Asn Asn Pro Lys Glu Trp Leu Gln Val Asp Phe Gln Lys Thr Met Lys
        1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
            1540                1545                1550

Arg Tyr Leu Arg Ile His Pro Gln Ser Trp Val His Gln Ile Ala Leu
        1555                1560                1565

Arg Met Glu Val Leu Gly Cys Glu Ala Gln Asp Leu Tyr
    1570                1575                1580

## Description

### Technical Field

The present invention relates to a preparation technique of recombinant single-chain FVIII protein used for the treatment of hemophilia and its chemical conjugates.

### Background Art

Hemophilia is a disease in which bleeding occurs continuously due to lack of coagulation factors. The coagulation factors in the blood are composed of I to XIII. Among them, the factors related to hemophilia are VIII, IX and XI. Hemophilia is caused by the genetic defect of the above factors. Depending on the type of a deficient coagulation factor, hemophilia can be classified into Hemophilia A where Factor VIII is deficient, Hemophilia B where Factor IX is deficient, and Hemophilia C where Factor XI is deficient. Hemophilia A accounts for about 80 to 85% of the hemophilia.

The goal of hemophilia therapy is hemostasis. The treatment is conducted in two aspects, i.e., prevention and on demand treatment, using the enzyme replacement therapy (ERT). The current trend of treatment is to shift toward prevention.

As for the protein therapeutic agent used for the ERT, FVIII (Hemophilia A) and FIX (Hemophilia B), which were extracted from whole blood and plasma and concentrated, were used in the past. However, there were problems such as side effects and complications arising from using the human-derived raw material. Therefore, recently, various factors produced by recombinant DNA technology have been developed and used. FVIII is also produced by recombinant DNA technology, which reduces the risk of infectious diseases and complications. However, it has a short half-life like blood-derived coagulation FVIII, which requires frequent administrations, and is inconvenient for patients. Thus, continuous efforts have been made to improve the life quality of patients by developing various FVIII having long *in vivo* half-life.

One of them was pegylated FVIII. It was developed to lower the binding of LRP1 (low density lipoprotein receptor related protein), which is known to be responsible for the *in vivo* clearance of FVIII, with pegylated FVIII. The pegylated FVIII improved the *in vivo* half-life by preventing the clearance of FVIII. However, the extent of the improvement was below expectations. Since FVIII strongly interacts with vWF, most of FVIII exist *in vivo* in association with vWF. And thus, the half-life of FVIII is determined by the *in vivo* half-life of vWF bound to FVIII. This situation also applies to pegylated FVIII. Since the half-life of pegylated FVIII was also controlled by the half-life of vWF after pegylation, there was a limitation that the improvement of the half-life is below the half-life of vWF.

In addition, FVIII-Fc fusion protein (FVIII-Fc) in which the Fc domain of the antibody is fused to the C-terminal of FVIII have been developed. FVIII-Fc has a longer *in vivo* half-life than FVIII due to *in vivo* recycling by FcRn. However, by the binding with vWF, the *in vivo* retention time of FVIII-Fc was also controlled by the half-life of vWF.

Moreover, for the treatment of Hemophilia A, there are ongoing efforts to replace the function of FVIII as a blood coagulation factor through other pathways other than the present FVIII, and to promote an *in vivo* sustained effect and patient convenience.

As an example, FVIII-mimetic bi-specific antibody, which is a bi-specific antibody in the form of an intact IgG form containing two types of Fabs specific for FIX (or activated FIX) and FX, has been designed to replace the function of FVIII *in vivo* (Kitazawa et al, Nature medicine 2012 18 (10): 1570-4).

Another example is the use of anti-TFPI (Tissue factor pathway inhibitor). This is a substance that inhibits the function of the TFPI and is being developed in the form of an aptamer in addition to antibodies and peptides (Hilden et al., BLOOD 2012, 119 (24): 5871-8). TFPI prevents the activation process of FX by TF (tissue factor)-activated FVII complex to inhibit the production of FXa, thereby inhibiting the production of thrombin by FXa to stop the blood coagulation progress.

Anti-thrombin inhibitor has also been proposed. It inhibits the function of thrombin inhibitor in blood to improve the activity of thrombin, thereby facilitating blood coagulation in hemophilia animals or hemophilia patients.

However, these approaches do not directly replace or supplement the activity of FVIII and FIX proteins deficient in Hemophilia A or Hemophilia B, but indirectly mimic or bypass the activity of FVIII. Since the modes of action of these approaches are complex, their safety and effectiveness in clinical trials should be sufficiently demonstrated.

Korean Patent Laid-Open Publication No. 2014-0114266 discloses an anti-hemophilia Factor VIII having an increased specific activity.

The therapeutic agents developed so far have the problems of short intervals of administrations (intravenous injections) in terms of functions, and of frequent bleedings arising from the loss of efficacy due to short half-lives. Also, in terms of productivity, the problems of low productivity and high cost of treatment should also be solved. Thus, it is necessary to develop a FVIII having an improved half-life and high productivity.

### Disclosure of Invention

### Technical Problem

An object of the present invention is to provide a single-chain FVIII which shows improved *in vivo* retention time and can be easily produced on mass scale.

### Solution to Problem

According to one embodiment of the present invention, there is provided a single-chain blood coagulation factor VIII (Factor VIII) comprising a heavy chain, a light chain and a partially-deleted B domain fragment of human Factor VIII, wherein the partially-deleted B domain fragment lacks at least 5 amino acids from residue 1648 of SEQ ID NO:1 to the N-terminal direction and at least 5 amino acids from residue 1649 of SEQ ID NO:1 to the C-terminal direction, so as not to comprise a cleavage site by furin protease, and contains 4 to 6 glycosylation sites.

According to one embodiment of the present invention, the partially-deleted B domain fragment included in the single-chain blood coagulation factor VIII of the present invention comprises about 15% to 40% of a wild type B domain sequence, but is not limited thereto so long as the effect according to the present invention is achieved. Those skilled in the art can select an appropriate single-chain blood coagulation factor VIII in consideration of the condition of the B domain fragment included in the aforementioned single-chain blood coagulation factor VIII. Herein, the B domain is interpreted to include the a3 region as described later.

According to one embodiment of the present invention, a portion deleted in the partially-deleted B domain fragment satisfying the above-mentioned condition may be continuous or non-continuous, and the partially-deleted B domain fragment may be represented by the sequence of (i) amino acid residues 741 to 902 and 1654 to 1689; (ii) amino acid residues 741 to 965 and 1654 to 1689; or (iii) amino acid residues 741 to 902, 1637 to 1642, and 1654 to 1689, based on the sequence of SEQ ID NO: 1.

According to one embodiment of the present invention, the single-chain FVIII in the present invention includes the sequences having the amino acid sequences represented by SEQ ID NOS: 2 to 7 or the sequence having about 90% or more homology thereto, which may be easily determined with reference to the homology determination method described later and the disclosure relating to the factor according to the present invention and biological functional equivalence.

According to one embodiment of the present invention, the nucleic acid molecules encoding the single-chain FVIII of the present invention having the amino acid sequences represented by SEQ ID NOS: 2 to 7 include the sequences represented by SEQ ID NOS: 10 to 15, codon-optimized sequences therefrom for the expression in a cell, or altered nucleic acid sequences due to a degenerate codon.

The single-chain FVIII according to the present invention has a specific activity of 90% or higher than that of a two-chain blood coagulation factor VIII when measured by a CS or APTT method.

According to one embodiment of the present invention, the single-chain FVIII is conjugated to a hydrophilic polymer, which includes polyethylene glycol (PEG), polyethylene oxide, dextran, or polisialic, and when PEG is used, it may be conjugated at the conjugation position through acryloyl, sulfone or a maleimide.

According to one embodiment of the present invention, the conjugated blood coagulation factor VIII is conjugated to a hydrophilic polymer at some amino acid residues of the A domain and/or B domain fragment; the conjugation position of the B domain fragment is at least one selected from the group consisting of amino acid residues 754, 781,782, 788, 789, 825 and 897 based on the sequence of SEQ ID NO: 1 and the conjugation position of the A domain is at least one selected from the group consisting of amino acid residues 491, 495, 498 and 1806 based on the sequence of SEQ ID NO: 1; and the residue of the conjugation position may be substituted with cysteine for the conjugation with the hydrophilic polymer.

According to another embodiment of the present invention, the single-chain FVIII is conjugated to PEG, particularly having an average molecular weight of 20 kDa or more.

According to another embodiment of the present invention, there is provided a single-chain FVIII, specifically a single-chain FVIII represented by SEQ ID NOS: 2 to 7, or a single-chain FVIII where amino acid residue of the conjugation positions are substituted with cysteine for conjugation to a hydrophilic polymer, wherein the conjugation positions in the B domain are at least one selected from the group consisting of amino acid residues 754, 781,782, 788, 789, 825 and 897 based on the sequence of SEQ ID NO: 1; and the conjugation positions in the A domain are at least one selected from the group consisting of amino acid residues 491, 495, 498 and 1806 based on the sequence of SEQ ID NO: 1; or a nucleic acid molecule encoding the single-chain FVIII comprising a substituted cysteine group

According to another embodiment of the present invention, the residues into which cysteine was introduced are as shown in Table 3, and the nucleic acid molecule in which specific residues are substituted with cysteine for conjugation to a hydrophilic polymer is represented by SEQ ID NOS: 17 to 32, and the amino acid sequences corresponding thereto are represented by SEQ ID NOS: 33 to 48. Further, a codon-optimized sequence for expression in a cell or altered nucleic acid sequence by a degenerate codon is also comprised in the scope of the present invention.

According to another embodiment of the present invention, there is also provided a vector comprising the nucleic acid molecule according to the present invention, and a cell comprising the vector.

According to another embodiment of the present invention, there is provided a method for producing a single-chain FVIII comprising: transfecting the vector of the present invention into a eukaryotic cell; or alternatively providing the cell transfected with the vector of the present invention; expressing a single-chain FVIII where the free thiol group of the cysteine introduced thereinto is masked with a disulfide bond by cysteine or glutathione through culturing the cells in a culture solution; collecting the single-chain FVIII expressed from the culture solution and treating the single-chain FVIII with a reducing agent to release the masked cysteine or glutathione; and treating the treated culture solution with a pegylation buffer.

In another embodiment of the present invention, there is also provided a blood coagulation kit comprising at least one of the single-chain FVIII disclosed in the present invention to be used for a hemophilia patient or a patient requiring blood coagulation, a use of the single-chain FVIII for the treatment of hemophilia patient or a composition, or a blood coagulation method comprising administering the single-chain FVIII to a hemophilia patient or a patient requiring blood coagulation in a therapeutically effective amount.

### Advantageous Effects of Invention

A single-chain FVIII according to the present invention comprises a heavy chain and a light chain which are connected by a B domain where a part of the sequence is deleted. Specifically, the single-chain FVIII comprises the partially-deleted B domain which does not comprise a cleavage site cleaved by furin protease, which is generated during the normal FVIII expression process, and contains at least 4 glycosylation sites. The single-chain Factor VIII according to the present invention or the single-chain FVIII in which some residues of the A or B domain are pegylated not only retains activities but also can be easily produced on mass scale because of its single-chain form. The said single-chain Factor VIII has an increased *in vivo* half-life through pegylation to enhance convenience for patients as a therapeutic agent for Hemophilia A and to lead a reduction of medical expenses through reduction in production cost.

### Brief Description of Drawings

Fig. 1 schematically shows a pegylated single-chain FVIII structure according to one embodiment of the present invention, wherein 1648 residue represents the furin cleavage site.
Fig. 2 schematically depicts the preparation of a single-chain FVIII according to one embodiment of the present invention from a full length FVIII. The furin cleavage site (1648^{th} amino acid residue based on SEQ ID NO: 1) is shown, and the numbers in the Figure indicate the respective amino acid residues corresponding to the designated sites.
Fig. 3 demonstrates the result of Western blot analysis of the single-chain FVIII expression according to one embodiment of the present invention.
Fig. 4 indicates the pegylation positions tested to determine the optimal pegylation position of the single-chain FVIII according to one embodiment of the present invention.
Fig. 5 illustrates the analysis results of FVIII activity after the expression of the mutant single-chain FVIII into which free cysteine was introduced according to one embodiment of the present invention.
Fig. 6 shows the results of SDS-PAGE analysis after pegylation of the mutant single-chain FVIII into which free cysteine was introduced according to one embodiment of the present invention, and the lines marked with an asterisk indicate the introduced cysteine that pegylation efficiency is relatively high.
Fig. 7 describes the test results of the PK (pharmacokinetics) of scFVIII according to one embodiment of the present invention compared to the commercialized FVIII substance.
Figs. 8a and 8b are brief illustration and experimental results, respectively, of an experimental method for comparing tail bleeding hemostatic efficacy test of the pegylated scFVIII according to one embodiment of the present invention with the efficacy of commercialized FVIII substance. Fig. 8b shows PEG40kDa-scFVIII-B3.

### Best Mode for Carrying out the present invention

The present invention is based on the development of coagulation factor VIII mutants that can be expressed in the form of single-chain with increased productivity and *in vivo* stability, and the development of blood coagulation factors with improved compliance in terms of patient's administration intervals through the pegylation of the mutant of Factor VIII.

Blood coagulation factor VIII is composed of A1-A2-B-A3-C1-C2 domains, which is synthesized as a single-chain protein in hepatocytes. After synthesis, it is matured through processing to form a 280 kDa heterodimer composed of a heavy chain and a light chain. Among them, the light chain has molecular weight of 80 kDa and is composed of A3-C1-C2 domains. And the heavy chain is composed of A1-A2-B domains and has a molecular weight of 90-200 kDa, and its molecular weight varies significantly depending on the differences in the length and glycosylation degree of the B domain. The a1 domain between the A1 and the A2 domains, the a2 domain between the A2 and B domains, and the a3 domain are included in the heavy chain. The heterodimer is present in an inactivated state in the blood by binding to vWF, and when exposed to stimuli such as vascular injury, it is cut by thrombin after arginine residue, that is, after the residues 372, 740 and 1689. As a result, it is separated from vWF and activated to form a trimer of A1, A2 and A3-C1-C2. Then the trimer catalyzes the activation of FX by FIXa, and is rapidly deactivated.

It has been reported that single-chain FVIII is structurally stable and shows an increase in the expression level (*see* WO2004/067566). The FVIII in the form of a two-chain has been reported to show differences in the expression levels depending on the length of the B domain (Miao et al., Blood 2004 May 1; 103 (9): 3412-9).

In the present invention, it was found that the specific activity and the APTT/CS ratio were changed in addition to the differences in the expression level depending on the length and sequence of the B domain including a3 in the preparation of the single-chain FVIII. A recombinant single-chain FVIII with a specific activity and APTT/CS ratio similar to those of the natural-type of a two-chain FVIII was prepared by optimizing the length and sequence of the B domain. The single-chain FVIII according to the present invention was found to be stable and excellent in the expression level while maintaining the inherent property of wild type FVIII.

Thus, according to one embodiment, the present invention provides a single-chain Factor VIII in which a heavy chain region and a light chain region of human Factor VIII are linked by a partially-deleted B domain fragment of human Factor VIII. Specifically, the partially-deleted B domain fragment lacks at least 5 amino acids from residue 1648 of SEQ ID NO:1 to the N-terminal direction and at least 5 amino acids from residue 1649 of SEQ ID NO: 1 to the C-terminal direction, so as not to comprise a cleavage site by furin protease, and contains 4 to 6 glycosylation sites.

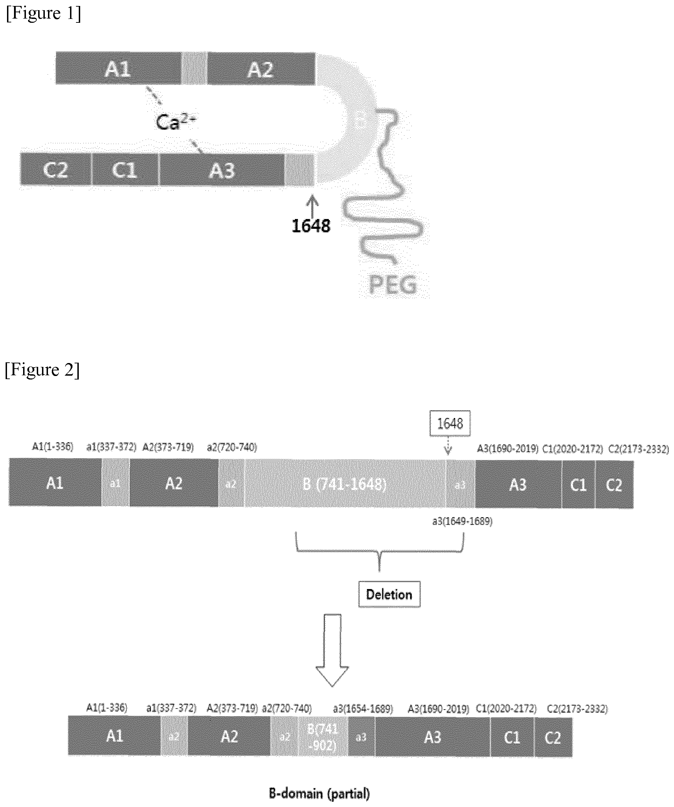

The single-chain FVIII according to the present invention comprises the A1-A2-partial B-A3-C1-C2 domains. The residue positions and sequences of each domain comprised in the FVIII in the present invention are known in the art, and the human amino acid sequence such as human FVIII can be represented by the sequence of SEQ ID NO: 1. Based on the sequence, the heavy chain is composed of 1-740 residues including the A1 and A2 domains; the B domain is composed of 741-1689 residues; and the light chain is composed of 1690-2332 residues including the A3, C1 and C2 domains (Fig. 2; and Orlova et al., Acta Naturae. 2013 Apr-Jun; 5(2): 19-39). The B domain in the present invention is intended to include the a3 domain (1649 to 1689) described in Fig. 2.

In this respect, the single-chain FVIII according to the present invention can be represented by the formula A1-A2-B'-A3-C1-C2, wherein A1 is the A1 domain, A2 is the A2 domain, B' is a part of the B domain, A3 is the A3 domain, C1 is the C1 domain, and C2 is the C2 domain. Reference can be made to the above-mentioned descriptions for each domain.

The partially-deleted B domain (B') in the single-chain FVIII according to the present invention lacks a cleavage site of the proteolytic enzyme furin. According to one embodiment, the B' domain lacks at least 5 amino acids from residue 1648 of SEQ ID NO:1 to the N-terminal direction and at least 5 amino acids from residue 1649 of SEQ ID NO:1 to the C-terminal direction. Since the B domain has several glycosylation sites and the glycosylation of a protein affects the activity and stability of the protein during and after the expression process, the number of glycosylation may be taken into consideration when determining the deletion sites of the B domain.

According to one embodiment of the present invention, the deletion site of the B domain is determined so as to include 4 to 6 glycosylation sites.

When determining the deletion site of the B domain, the B domain having deletion of the furin cleavage site included in the single-chain FVIII according to the present invention can be determined within a range including about 15 to 40% of the entire wild type B domain.

The deletion sites of the partially-deleted B domain comprised in the single-chain FVIII according to the present invention may be continuous or non-continuous.

In an embodiment, in case where the single-chain FVIII according to the present invention comprises a B domain having continuous deletions including the furin cleavage site, the B domain may have a sequence represented by (i) amino acid residues 741 to 902 and 1654 to 1689 (i.e., 903 to 1653 residues of the B domain are deleted continuously); (ii) amino acid residues 741 to 965 and 1654 to 1689 (i.e., residues 966 to 1653 of the B domain are deleted); or in case where the single-chain FVIII according to the present invention comprises a B domain having discontinuous deletions, the B domain may be represented by the sequence of (iii) amino acid residues 741 to 902, 1637 to 1642, and 1654 to 1689, based on the sequence of SEQ ID NO: 1.

According to another embodiment, the FVIII amino acid sequence according to the present invention, including such deleted B domains, may be represented by SEQ ID NOS: 2 to 7.

The recombinant FVIII disclosed herein is not limited to the above sequences, but includes biological equivalents thereof. Biological equivalents mean that they have substantially the same activity as the polypeptides according to the present invention although additional modifications have been made to the amino acid sequences disclosed in the present invention, and such modifications include, for example, deletion, insertion and/or substitution of amino acid sequence residues.

According to one embodiment, a recombinant FVIII polypeptide prepared by conducting a conservative amino acid substitution to the FVIII according to the present invention is included in the present invention.

Conservative amino acid substitution refers to a substitution that does not substantially affect or diminish the activity of a particular polypeptide, and includes, for example, 1 to 15 conservative substitutions, or 1 to 12, for example, 1, 2, 5, 7, 9, 12, or 15 conservative amino acid substitutions.

Conservative amino acid substitutions are known in the art, and reference can be made to, for example, the descriptions in Creighton (1984) Proteins based on Blosum (BLOcks SUBstitution Matrix). W. H. Freeman and Company (Eds); and Henikoff, S.; Henikoff, J.G. (1992). "Amino Acid Substitution Matrices from Protein Blocks". PNAS 89 (22): 10915-10919. doi: 10.1073/pnas.89.22.10915; WO2009012175 A1, and the like.

Such amino acid modifications, especially substitutions, are made based on the relative similarity of the amino acid side chain substituents, such as hydrophobicity, hydrophilicity, charge, size, and the like. Based on the analysis of the size, shape and type of amino acid side chain substituents, arginine, lysine and histidine are all positively charged residues; alanine, glycine and serine have similar sizes; phenylalanine, tryptophan and tyrosine have similar shapes. Thus, considering the above, arginine, lysine and histidine; alanine, glycine and serine; and phenylalanine, tryptophan and tyrosine are biologically functional equivalents.

Also, when introducing amino acid substitutions, the hydropathic index of amino acids can be taken into consideration. Each amino acid is given a hydropathic index depending on its hydrophobicity and charge: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). Such hydropathic index is particularly important in order to impart an interactive biological function to the protein. It is known in the art that it is necessary to substitute with an amino acid having a similar hydropathic index to retain a similar biological activity. When a mutation is introduced with reference to the hydropathic index, it is advantageous to conduct substitution between amino acids which shows the hydropathic index difference, preferably within±2, more preferably within±1, even more preferably within±0.5.

It is also known in the art that the substitution between amino acids with similar hydrophilicity values results in a protein with an equivalent biological activity. For example, as disclosed in U.S. Patent No. 4,554,101, the following hydrophilicity values are given to each amino acid residue: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4).

Amino acid substitutions that do not entirely alter the activity of a recombinant protein according to the present invention are known in the art (H. Neurath, R. L. Hill, The Proteins, Academic Press, New York, 1979). For example, the most commonly performed substitutions are those between the amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu and Asp/Gly.

Thus, variants having biological and functional equivalence as described above have a substantial identity with an amino acid sequence disclosed in the present specification or a nucleic acid molecule encoding the same, and are included within the scope of the present invention
Such substantial identity can be determined using sequence comparison methods known in the art. The sequences showing preferably at least 80% homology, specifically 85% or more, more specifically 90% or more homology, even more specifically 95% or more homology means substantial identity when the sequence disclosed herein and any other sequence are aligned for maximum correspondence between them and the aligned sequences are analyzed using algorithms commonly used in the art. Alignment methods for sequence comparison are known in the art. For example, they are disclosed in: Smith and Waterman, Adv. Appl. Math. (1981) 2:482; Needleman and Wunsch, J. Mol. Bio. (1970) 48:443; Pearson and Lipman, Methods in Mol. Biol. (1988) 24: 307-31; Higgins and Sharp, Gene (1988) 73:237-44; Higgins and Sharp, CABIOS (1989) 5:151-3; Corpet et al., Nuc. Acids Res. (1988) 16:10881-90; Huang et al., Comp. Appl. BioSci. (1992) 8:155-65 and Pearson et al., Meth. Mol. Biol. (1994) 24:307-31. The NCBI Basic Local Alignment Search Tool (BLAST) (Altschul et al., J. MoI. Biol. 215: 403-10(1990)) is available from the National Center for Biological Information (NBCI, Bethesda, Md.), etc., for use in connection with the sequence analysis programs such as blast, blastp, blasm, blastx, tblastn and tblastx. BLAST can be accessed at www.ncbi.nlm.nih.gov/BLAST/ and a sequence homology comparison method using this program is available at www.ncbi.nlm.nih.gov/BLAST/blast_help.html.

The recombinant single-chain FVIII according to the present invention includes those having a molar activity of 90% or more, 95% or more, 97% or more, 98% or more, 99% or more, or 100% as compared to the wild type two-chain FVIII. The methods for measuring the wild type two-chain FVIII and the activity thereof are known in the art, and can be conducted, for example, by determining the specific activity values measured by CS and clotting assay (APTT: activated partial thromboplastin time) and (APTT)/CS ratios (Clotting assays Circulation 2005; 112: e53-e60). Those skilled in the art will be able to measure the activities by selecting a suitable method with reference to the techniques in the art and descriptions of reference documents, and determine the recombinant single-chain FVIII included in the scope of the present invention based on the measurement. The two-chain FVIII is processed by furin as described above and is in the form of a dimer composed of a light chain and a heavy chain. In the present invention, comparisons were made with the recombinant protein of wild type FVIII (Advate®), which is expressed in a recombinant form in CHO cells and most of which is in the form of a two-chain containing a B domain of various lengths. The recombinant protein of wild type FVIII has compositions similar to the plasma-derived wild type FVIII.

The two-chain FVIII to be compared with the single-chain FVIII according to the present invention comprises or does not comprise the B domain. The two-chain FVIII which does not comprise the B domain can be mainly produced by intentionally removing the B domain through a recombinant method using a protein cleavage process by FXa (an active form of FX) and thrombin during the procedure of wild type FVIII expression. The two-chain FVIII which comprises a portion of or the entire B domain can be produced by a recombinant method, or by a protein cleavage process by FXa (an active form of FX) and thrombin during the procedure of wild type FVIII expression. However, the two-chain FVIII compared with the single-chain FVIII of the present invention was recombinantly expressed from the wild type FVIII comprising the entire B domain. The two-chain FVIIIs are a mixture of the two-chain FVIII comprising the entire B domain produced by the furin processing when secreted from the inside of the cell to the outside and the FVIII which does not comprise the B domain at all or comprises a portion of the B domain produced by removing a portion of or entire B domain by thrombin or FIXa or FXa thereafter, most of which are recombinant FVIIIs similar to the wild type FVIII.

According to another embodiment, the present invention provides a nucleic acid molecule or polynucleotide encoding the single-chain FVIII according to the present invention, or a vector comprising the polynucleotide, or a cell (line) transfected with the vector.

The nucleic acid molecule encoding the recombinant single-chain FVIII according to the present invention includes codon-optimized one for the type of cells in which the recombinant single-chain FVIII according to the present invention is expressed. According to one embodiment of the present invention, the codon-optimized nucleic acid sequence may be optimized for the CHO cell codon, which is an optimized nucleic acid sequence encoding the protein of SEQ ID NO: 5, and can be represented by SEQ ID NO: 15.

A nucleic acid molecule encoding a recombinant single-chain FVIII according to the present invention also includes one encoding FVIII which is substantially identical and biological equivalent, as described above.

Also, the nucleic acid molecules encoding the recombinant single-chain FVIII according to the present invention may also be used for cloning various expression vectors for various purposes. The specific constitution of the expression vector may vary depending on the host cell in which the recombinant single-chain FVIII according to the present invention is to be expressed. However, it comprises the sequence which regulates the expression of the nucleic acid molecule of the present invention to mRNA or the expression of the mRNA to a protein, such as, for example, a promoter and/or an enhancer, and the like. Various vectors and regulatory sequences which may be used for the above or various other purposes are known in the art and may be selected appropriately by a person skilled in the art in light of the specific objects and effects of the present invention. Such vectors and sequences may include, for example, those disclosed in the Examples and Figures of the present invention, but are not limited thereto.

A vector comprising a nucleic acid molecule encoding a recombinant single-chain FVIII according to the present invention may be prepared by methods known in the art, for example, by operatively linking a nucleic acid molecule encoding a recombinant single-chain FVIII according to the present invention to a promoter and/or an enhancer. According to one embodiment, it is inserted into a recombinant expression vector which is capable of expressing a foreign gene in a cell, and examples of such vectors include, but are not limited to, general protein expression vectors such as pMSGneo and pcDNA3.1(+). The pMSGneo vector is an expression vector containing a MAR (Matrix attachment region) element that binds to a nuclear matrix, and the MAR element plays a role in enhancing gene expression by inducing position-independent expression when used in an expression vector. Thus, when the pMSGneo vector is used, a stable and high expression level can be achieved. In addition, the pcDNA3.1 (+) vector contains a strong CMV promoter and thus it is widely used for protein expression.

In addition, the recombinant expression vector comprising a nucleic acid molecule encoding the recombinant single-chain FVIII according to the present invention may be transfected into an appropriate host cell as described below for various purposes. In order to express the nucleic acid molecule contained in the vector as a protein, the coding sequence of the nucleic acid molecule encoding the protein may be suitably optimized for the desired cell.

In this respect, the present invention provides a recombinant cell transfected or transformed with a vector according to the present invention. Such cells include both prokaryotic and eukaryotic cells that can be used for the preparation of the desired protein by amplification of the vector according to the present invention and/or expression of the nucleic acid molecule contained in the vector. Various cells which can be used for the above purpose are known in the art, and a person skilled in the art will be able to select appropriate cells in consideration of the specific purposes and effects of the present invention, including those described in the Examples and Figures of the present invention, but is not limited thereto. For example, *E. coli*, mammalian cells, yeasts, plant cells, and insect cells can be included. According to one embodiment of the present invention, eukaryotic cells, in particular, mammalian cell lines, are used for the expression of recombinant FVIII. A person skilled in the art will be able to select appropriate cell lines capable of preparing proteins having such property in consideration of the property of the recombinant FVIII according to the present invention. For example, CHO, BHK, COS7, HEK cell lines, etc., which are known in the art, may be used as such mammalian cell lines. Preferably, CHO cell line, specifically CHO-S cell line, CHO-DG44 or CHO-K1 cell line, or HEK cell line, specifically HEK293 cell line, may be used.

The vector according to the present invention is transferred to said host cells for expression. Methods for transferring vectors to such host cells are known in the art, which may be implemented by known methods in the art, such as calcium phosphate precipitation, a shotgun method, a method using liposome, a nanoneedle method or electroporation, but are not limited thereto.

The single-chain FVIII according to the present invention may be modified at a specific residue by a hydrophilic polymer for the purpose of increasing the half-life.

The protein structure of the B domain has not yet been revealed. The B domain is known as a protein having a flexible structure, high degree of glycosylation and abundant negative charges, but its structure is difficult to predict. In other words, it is theoretically possible to predict the structure of the B domain using the molecular modeling technique, but it is difficult to predict the B domain structure when considering the heterogeneity due to the post-translational modification and glycosylation of the B domain. In order to select the modification position in the B domain, the structure information on the surface of the B domain protein structure is required. However, since there is no information on the structure, it is very difficult to select the pegylation position.

However, in the present invention, the residue of the A domain and/or the B domain which does not particularly affect the activity of the single-chain FVIII was selected through an unexpected effort and modified with a hydrophilic polymer. As a result, a single-chain FVIII having an improved half-life while retaining its inherent property was obtained.

In particular, in the present invention, the positions between -6 and +6 residues based on the residue (0) near the glycosylation of the B domain were selected as pegylation positions.

In this regard, the present invention is related to a single-chain FVIII wherein the single-chain FVIII is conjugated to a hydrophilic polymer at some amino acid residues of the A domain and/or B domain fragment; the conjugation position of the B domain fragment is at least one selected from the group consisting of amino acid residues 754, 781,782, 788, 789, 825 and 897 based on the sequence of SEQ ID NO: 1 and the conjugation position of the A domain is at least one selected from the group consisting of amino acid residues 491, 495, 498 and 1806 based on the sequence of SEQ ID NO: 1; and the residue of the conjugation position is substituted with cysteine for the conjugation with the hydrophilic polymer.

Various polymers known in the art can be used as the hydrophilic polymer for use in the modification of the single-chain FVIII according to the present invention, which include, for example, polyethylene glycol, polyethylene oxide, dextran, polisialic, etc., but is not limited thereto. These polymers have a biocompatible (non-toxic or low toxicity) hydrophilic flexible structure, and when chemically conjugated to a protein, the polymers play a role in inhibiting the loss of activities of the conjugated protein due to adhesion to the surface of other molecule or material by a non-specific interaction, and inhibiting inactivation by protease in the blood.

According to one embodiment of the present invention, PEG is used, and specifically, PEG of 20 kDa or more is used. PEG is conjugated to a protein and provides an effect of wrapping around the protein, which leads to the defense against the loss after binding to a scavenger receptor or degradation by the inactivating protease. PEG is a polymer in a chain form that is linear or branched, and PEG having small molecular weight cannot fully wrap the protein and thus cannot fully protect the protein. Thus, the molecular weight should be the same or above the critical level to sufficiently wrap the protein to be protected. Pegylation of the single-chain FVIII of the present invention can be carried out by substituting the amino acid at the pegylation position with cysteine and then conjugating to the PEG containing an acryloyl, sulfone or maleimide group specific to cysteine at one end.

That is, in the single-chain FVIII according to the present invention, specific residue may be substituted for modification to a hydrophilic polymer, and specifically, the residue to be substituted varies depending on the position where the hydrophilic polymer is conjugated in the single-chain FVIII, and a person skilled in the art may be able to modify with a suitable residue for the conjugation of a hydrophilic polymer.

According to one embodiment of the present invention, pegylation is used for the modification. In such case, the residue to be modified is substituted by cysteine.

According to one embodiment of the present invention, modification is conducted at 495 valine residue of the A2 domain and/or 782 isoleucine residue of the B domain, and specifically the residues are substituted with cysteines for pegylation, and the residues are pegylated.

Expression of a protein substituted with cysteine for modification can be conducted using a method known in the art. For example, it is necessary to increase the pegylation efficacy by stabilizing the introduced free cysteine through masking the introduced free cysteine until the free cysteine introduced in a reduction condition is restored just before pegylation. Usually, the introduced free cysteine of a protein to be pegylated is stabilized by a disulfide bond with the low molecular weight substance containing free thiol, cysteine or glutathione, during the expression inside a cell or after the secretion outside the cell.

Pegylation can be conducted using a method known in the art. For example, cysteine or glutathione used for masking free cysteine should be removed prior to the pegylation reaction so that the free thiol of the introduced cysteine can be restored, and said process can be conducted including a step of treating with reduction agents such as TCEP, DTT, beta-mercaptoethanol, cysteine, and glutathione (reduced type), and an oxidation step for restoring the FVIII's original disulfide bonds cleaved by reduction.

Masking of the introduced cysteine of the expressed recombinant protein can be carried out using known methods. For example, the cysteine or glutathione used for masking free cysteine may be further added to the cell culture medium components, or the cysteine or glutathione components normally produced, stored, and secreted by cells during cell growth and maintenance can be used without further addition to the cell culture medium component.

In another aspect, the present invention also provides a method for producing a single-chain FVIII comprising transfecting a vector according to the present invention described above to a eukaryotic cell; culturing the cell in a culture solution; collecting the culture solution to purify a cysteine-introduced FVIII; and treating the purified cysteine-introduced FVIII with a pegylation buffer solution; restoring the free thiol group of the introduced cysteine through a reduction process; oxidizing the resultant to recover a separated disulfide bond in the single-chain FVIII during the reduction process; pegylating the cysteine of the single-chain FVIII restored with a reduced thiol group, and isolating the pegylated single-chain FVIII.

Reference can be made to those described in the Examples herein regarding specific vectors, cells and processing steps used in the method according to the present invention.

The recombinant single-chain FVIII according to the present invention can be useful for blood coagulation of a patient with a disease requiring blood coagulation, i.e., hemophilia, specifically, Hemophilia A, or for the treatment of Hemophilia A.

Accordingly, the present invention also provides a blood coagulation kit for use by a hemophilia patient or a patient requiring blood coagulation, comprising any one of the single-chain FVIIIs described in the present invention including the claims, a use of said single-chain FVIII in treating hemophilia or a composition comprising the same, a composition comprising a nucleotide sequence encoding said single-chain FVIII, a use of the composition in genetic treatment of Hemophilia A, a method for hemophilia treatment or blood coagulation comprising administering the composition comprising a nucleotide sequence encoding said single-chain FVIII or administering said single-chain FVIII in a therapeutically effective amount to a hemophilia patient or a patient requiring blood coagulation.

The recombinant single-chain FVIII according to the present invention can be provided in the form of a pharmaceutical composition for treating hemophilia which also contains a pharmaceutical acceptable carrier.

In addition, the pharmaceutical composition of the present invention can be used alone or in combination with other drug therapies and methods using a biological response modifier.

In addition to the above-mentioned active ingredients, the composition of the present invention may be prepared by further comprising at least one pharmaceutically or physiologically acceptable carrier.

The term "carrier" as used herein is intended to mean a pharmaceutically acceptable carrier, excipient, or stabilizer that is non-toxic to a cell or mammal exposed to the same at a dose and concentration employed. Examples of such carriers include saline, Ringer's solution, buffered saline, buffers such as phosphate, citrates and other organic acids, antioxidant such as ascorbic acids, polypeptides with low molecular weight (about less than 10 amino acids), proteins such as serum albumin, gelatin or immunoglobulin; hydrophilic polymer such as polyvinylpyrrolidone, amino acids such as glycine, glutamine, asparagine, arginine or lysine, carbohydrates such as monosaccharides, disaccharides, and glucose, mannose or dextrin, chelating agents such as EDTA, sugar alcohols such as mannitol or sorbitol, counter ions for salt formation such as sodium, and/or non-ionic surfactant such as tween®, polyethylene glycol (PEG) and PLURONICS®.

If desired, the composition may further comprise other additives known in the art such as antioxidants, buffers, antibacterial agents. The composition may be formulated into injectable formulations such as solution, suspension, emulsion, etc., by adding diluents, dispersing agents, surfactants, binders and lubricants.

Further, the composition can be preferably formulated depending on the disease or ingredients, using appropriate methods in the art or as disclosed in Remington's Pharmaceutical Science (recent edition; Mack Publishing Company, Easton PA).

The composition of the present invention is preferably administered parenterally (for example, intravenous, subcutaneous, intraperitoneal) according to a desired method. The dosage varies depending on the disease condition and weight of a patient, degree of the disease, the type of a drug, the administration route and time, but can be appropriately selected by a person skilled in the art.

The composition according to the present invention is administered in a therapeutically effective amount. In the present invention, "a therapeutically effective amount" refers to an amount sufficient to treat a disease with a reasonable benefit/risk ratio which is applicable to a medicinal treatment. The level of effective dose may be determined depending on factors including types of diseases of patients, severity, activity of a drug, sensitivity to a drug, administration time, administration routes, excretion rates, period of treatment, a simultaneously used drug, and other factors known in the medicinal field. The composition of the present invention may be administered as a single therapeutic agent or administered in combination with other therapeutic agents. Also, the composition of the present invention may be administered with a conventional therapeutic agent sequentially or simultaneously, and the composition may be used for single or multiple administrations. It is important to administer the composition at a minimal dose which can lead to a maximum effect without side effects in consideration of all of the factors described above, and the doses may be easily determined by a person skilled in the art.

The recombinant single-chain FVIII according to the present invention may be administered as it is or in the form of a composition as described above in a therapeutically effective amount to a subject with hemophilia or in need of blood coagulation and may be provided in the form of a blood coagulation method or a method for treating hemophilia, and reference can be made to the foregoing description.

As used herein, the term "treatment" refers to any action that improves or alters the symptoms of a disease in a beneficial way by administration of a composition of the present invention. The subjects for whom the method according to the present invention is used may be primates, including humans, but are not limited thereto.

Hereinafter, examples to aid in the understanding of the present invention will be presented. However, the following examples are provided only for the purpose of easier understanding of the present invention, and the present invention is not limited to the following examples.

### Example

### Example 1. Construction of single-chain FVIII

In order to prepare a human FVIII in a single-chain form (single-chain FVIII, scFVIII) which has a high expression rate while retaining the inherent property of human FVIII, recombinant scFVIIIs containing various length of the B domain were constructed and expressed by modifying the length of the B domain including a3 of FVIII, and the expression levels and activities (APTT, CS) thereof were analyzed. Detailed methods and results are as follows.

### Example 1-1. Preparation of scFVIII

For the construction of FVIII expressed in a single-chain form, FVIII was constructed in which a portion of the B domain including the furin cleavage site, i.e., the residue 1648 (based on the amino acid sequence of SEQ ID NO: 1 which represents the full length FVIII amino acid sequence), was deleted. Since the length of the B domain connecting the heavy chain and light chain may affect the final activity and property of FVIII, a total of 7 types of FVIII were constructed as shown in Table 1 for the determination of the length of the B domain comprised in the scFVIII. Each scFVIII was constructed such that it comprises the B domain of a specific length counting from the N-terminal (the 741^{st} amino acid residue of SEQ ID NO: 1) of the B domain, and the B domain contains the originally present sugar chain as it is.

In addition, in order to impart a property similar to the wild type FVIII activity by minimizing the structural interference caused by the B domain connecting the light chain and the heavy chain region, a structure in which a portion of the a3 domain adjacent to the light chain domain is contained more than other scFVIIIs was also prepared (SEQ ID NO: 6). Further, in order to improve the expression in CHO cells, scFVIII optimized with the CHO codon was prepared (SEQ ID NO: 15).

Each scFVIII comprising the partially-deleted B domain was synthesized based on the human FVIII nucleic acid sequence (NM000132). In order to prepare the scFVIII gene expression vector, G1 protein sequence (leader-heavy chain-B domain (741-764, 1653-1689)-light chain) was encoded and the entire gene was synthesized by GeneArt to include the *Not*I*-Asis*I enzyme cleavage site at the 5' end and *Xho*I*- Pac*I enzyme cleavage site at the 3' end based on the human FVIII nucleic acid sequence (NM000132). The pcDSW-G1 expression vector was constructed by cloning the synthesized gene into the *Not*I*lXho*I site of the pcDSW vector. The accuracy of the expression vector construction was confirmed by size comparison of the bands which were cleaved using the *Asis*I*lPac*I enzyme. Then, each gene corresponding to each scFVIII in which the B domain was deleted (G2, G3, G4, G4-flex, G6) was synthesized by GeneArt to include a sequence from the *BamH*I site (GGATCC) present in the A2 domain of the FVIII heavy chain to the *Pac*I enzyme cleavage site at the end of the light chain. Then, the pre-existing FVIII region was removed from the previously prepared pcDSW-G1 expression vector using the enzyme *BamH*I/*Pac*I*,* and the synthesized gene was cleaved with *BamH*I/*Pac*I enzyme and cloned into the *BamH*I/*Pac*I site of the pcDSW-G1 expression vector to construct the pcDSW-scFVIII expression vector. The accuracy of the expression vector construction was confirmed by size comparison after cleavage with *BamH*I/*Pac*I enzyme. In order to improve expression, regarding scFVIII (G6_opt), codon optimization and gene synthesis were conducted by GneArt to include *Not*I*-Asis*I cleavage site at 5' and *Xho*I*-Pac*I cleavage site at 3', and to be suitable for animal cell expression. The synthesized genes were cloned into the *Not*I/*Xho*I site of the pcDSW vector to construct an expression vector. The accuracy of the expression vector construction was confirmed by size comparison after cleavage with *BamH*I enzyme.

**Table 1. scFVIIIs comprising B domains of various lengths constructed in the present invention**

| **scFVIII** | | **Heavy chain sequence*** | **Portion of B domain sequence* (Including a3 domain)** | **Light chain sequence*** | **Number of Glycosylation in B domain** | **Protein SEQ ID NO** | **Nucleic acid SEQ ID NO** |
|---|---|---|---|---|---|---|---|
| *G1* | *scf1* | *1-740* | *(741-764)-*-- *(1653-1 689)* | *1690-2332* | *2(757, 764)* | **SEQ ID NO: 8** | **SEQ ID NO: 16** |
| *G2* | *scF2* | *1-740* | *(741-788)----(1654-1 689)* | *1690*-*2332* | *2(757, 784)* | **SEQ ID NO: 2** | **SEQ ID NO**: **10** |
| *G3* | *scF3* | *1-740* | *(741-832)----(1654-1 689)* | *1690-2332* | *3(757, 784, 82 8)* | **SEQ ID NO**: **3** | **SEQ ID NO: 11** |
| *G4* | *scF4* | *1-740* | *(741-902)---(1654-16 89)* | *1690*-*2332* | *4(757, 784, 82 8, 900)* | **SEQ ID NO: 4** | **SEQ ID NO: 12** |
| *G6* | *scF5* | *1-740* | *(741-965) --- (1654-1689)* | *1690-2332* | *6(757, 784, 82 8, 900, 943, 96* 3) | **SEQ ID NO**: **5** | **SEQ ID NO: 13** |
| *G4_flex* | *scF6* | *1-740* | *(741-902) ---(1637-1 642)---(16 64-1689)* | *1690-2332* | *4(757, 784, 82 8,900)* | **SEQ ID NO: 6** | **SEQ ID NO: 14** |
| *G6_opt* | *scF7*** | *1-740* | *(741-965) --- (1654-1689)* | *1690-2332* | *6(757, 784, 828, 900, 943, 963)* | **SEQ ID NO: 7** | **SEQ ID NO: 15** |

| | | | | | | | |
|---|---|---|---|---|---|---|---|
| * Described based on the sequence of SEQ ID NO: 1 ** Codon-Optimization for CHO cells | | | | | | | |

### Example 1-2. scFVIII expression and activity measurement

### scFVIII expression

The scFVIII constructed in Example 1-1 was expressed in cells, and the cells were cultured in a cell culture medium. First, for the expression, the expression vector constructed in Example 1-1 was transfected into Expi293F™ cells using the Expi293F™ Expression System Kit (Thermofisher Co., Catalog Number A14635), a transient expression system. The Expi293F™ cells were subcultured at a concentration of 2.0 x 10⁶ cells/mL using the Expi 293 culture medium according to the expected required amount 24 hours prior to transfection, and on the day of transfection, the cell number and cell viability were measured, and if the cell viability was 95% or higher, transformation was carried out. 7.5 x 10⁷ cells were added to a 125 mL flask with the Expi293 culture medium to a final volume of 25.5 mL (based on 30 mL). 30 µg of the expression vector constructed in Example 1-1 was mixed with Opti-MEM to obtain the total volume of 1500 µ1. 80 µl of a transfection reagent was mixed with Opti-MEM to a total volume of 1500 µl and incubated at room temperature for 5 minutes. Five minutes later, the Opti-MEM containing the transfection reagent was added to the Opti-MEM containing DNA and the resultant was gently mixed. The reaction was carried out at room temperature for 20 to 30 minutes. 3 mL of DNA: transfection reagent complex was added dropwise to 125 mL flask of Expi293F™ cells (total volume: 28.5 mL) prepared beforehand and the cells were incubated at 37°C in a 5% CO₂ shaking incubator at 125 rpm. After 16 ∼ 20 hours, 150 µl and 1.5 ml of Enhancer 1 and Enhancer 2 were added thereto, respectively, and the cells were cultured in a shaking incubator of 34°C and 5% CO₂ at 125 rpm. On the third day of culture, the medium was harvested and the activity of scFVIII was measured as follows.

### Measurement of expressed scFVIII activity

Currently, two types of recombinant FVIII, a full length FVIII and FVIII having a deleted B domain, are used clinically. However, the issue of whether these two recombinant FVIIIs are clinically equivalent in activity and efficacy has been raised (GRUPPO, R. A. et al., 2003. Comparative effectiveness of full-length and B domain deleted Factor VIII for prophylaxis-a metaanalysis. Haemophilia, 9, 251-60; LOLLAR, P. 2003. The Factor VIII assay problem: neither rhyme nor reason. J Thromb Haemost, 1, 2275-9; MIKAELSSON, M. et al., 2001. Measurement of Factor VIII activity of B domain deleted recombinant Factor VIII. Semin Hematol, 38, 13-23).

The activity of BDD-FVIII measured by clotting assay (APTT) was found to be lower than that measured by chromogenic assay (CS) by up to 50%, while the activities of the FVIII of a full length form was similar in both measurements (BARROWCLIFFE et al., SEMIN THROMB HEMOST, vol. 28 (3), 2002: 47-56). The low activity of BDD in the clotting assay has been suggested as a reason that BDD-FVIII is less effective in preventing hemorrhage in patients with Hemophilia A as compared to full length FVIII in actaul long-term prophylaxis (GRUPPO, R. A., et al., 2003. Comparative effectiveness of full-length and B domain deleted Factor VIII for prophylaxis--a metaanalysis. Haemophilia, 9, 251-60; GRUPPO, R. A. et al., 2004. Meta-analytic evidence of increased breakthrough bleeding during prophylaxis with B domain deleted Factor VIII. Haemophilia,10, 747-50; GRUPPO, R. A. et al., 2004. Increased breakthrough bleeding during prophylaxis with B domain deleted Factor VIII-a robust metaanalytic finding. Haemophilia, 10, 449-51).

Thus, in the present invention, in order to select scFVIII having specific activities (CS, APTT) and an specific activity ratio (APTT/CS) similar to those of the full length recombinant FVIII, the specific activities and specific activity ratio (APTT/CS) measured by CS and clotting assay (APTT) were analyzed regarding each of the scFVIII constructed in Example 1-1 and the result was used as criteria for scFVIII selection. As a comparative group, recombinant FVIII (ADVATE®, Baxalta) products in a full length, and two-chain forms, similar to the actual FVIII *in vivo*, were used as a control for comparison.

Specifically, the FVIII activity measurement test methods used here were the one-stage method and chromogenic method. The one-stage method is a method of measuring the FVIII activity of a sample by mixing the FVIII deficient plasma, activator, phospholipids and FVIII sample, and then measuring the blood coagulation time. The chromogenic method is a method in which FIXa, FX, thrombin, calcium, phospholipids and FVIII samples are mixed, and then a chromogenic substrate which forms color when cleaved by FXa is added to measure the amount of FXa activated by the FVIII sample, and the activity of FVIII is measured according to the degree of color formation of the chromogenic substrate. The difference between these two methods is that the one-stage method uses FVIII deficient plasma and measures the clotting time while the chromogenic method does not use the FVIII deficient plasma but measure the degree of color formation of a substrate after adding the factors necessary for activation of FVIII and the substrate for measuring the activated FVIII only.

Activity measurements by the one-stage method were conducted as follows using the automated assay method prepared before in the ACL TOP CTS 500 instrument. Samples were diluted to 1 IU/mL using FVIII deficient plasma, and the standard sample was put into the NIBSC FVIII standard vial and dissolved by adding 1 mL of distilled water thereto, and then diluted to 1 IU/mL using FVIII deficient plasma. Consumption reagents and measurement samples were prepared according to the rack type of the experimental equipment. The calcium chloride vials were serially put into the R rack of the ACL TOP CTS 500 equipment such that the barcodes of the vials could be recognized, and a magnetic stirring bar was put into the APTT-SP vial, which was then put into the R rack such that the barcodes of the vials could be recognized. The FVIII deficient plasma was prepared according to the number of samples x 400 µL (per each sample), and tape was placed around their barcodes so that the barcodes cannot be recognized, and then put into the R rack. Into the DA rack, a vial containing the G.O. buffer (Imidazole 3.4 g, NaCl 5.8 g, 1 L, pH 7.4) added with 2.63 mL of albumin was placed. After putting 400 µL or more of the samples and the standard sample prepared before into the sample cup, which was then placed in in the sample rack. In the ACL TOP program, information of unrecognized reagents and measurement samples were inputted, and the automated assay method prepared before was carried out. All reactions in the automated assay method were proceeded as follows at 37°C. First, the measurement samples were pre-diluted by 10-fold with the G.O. buffer, and then the samples were further diluted by 1, 3, and 10-fold to prepare 100%, 33,33%, and 10% samples, respectively. 50 µL each of the diluted samples was allowed to stand for 30 to 45 seconds, mixed with 50 µL of FVIII deficient plasma, and then allowed to stand for 60 to 70 seconds. After addition of 50 µL of the intermediate reagent APTT-SP, the resultant was allowed to stand for 300-340 seconds, and 50 µL of the start reagent (APTT-SP CₐCl₂) was added thereto finally, and then the clotting time was measured.

As for the activity measurement by the chromogenic method, the chromogenic assay kit sold by CHROMOGENIX Co., was used as the endpoint method, and the test was conducted by modifying the test method provided by the manufacturer to be suitable for the present test as follows. Specifically, samples are diluted to be in the standard range (0.25-1 IU/mL) using 1 x dilution buffer. Calibration plasmas were prepared using 1 x dilution buffer at the concentrations of 0, 0.25, 0.5, and 1 IU/mL. The prepared samples and 10 µL of the calibration plasma were diluted by 790 µL of 1 x dilution buffer, and 50 µL of the diluted samples were dispensed into a 96-well plate and allowed to stand at 37°C for 5 minutes. 50 µL of a factor reagent was dispensed into each well using an automatic dispenser pipette, which was then reacted for 2 minutes at 37°C. 50 µL of a substrate was dispensed into each well, which was then allowed to stand for 2 minutes at 37°C for color formation. The reaction was stopped by adding 50 mL of 2% citric acid to the samples showing color formation. The absorbance was measured at a wavelength of 405 nm to draw a linear calibration curve, and the absorbance of the sample was substituted into the calibration curve to measure the activity of the sample.

The measured results are shown in Table 2.

**Table 2. Results of measurement of the activities of the expressed recombinant scFVIIIs**

| **Single chain FVIII** | | **CS activity (IU/mL)** | | | **APTT/CS ratio** |
|---|---|---|---|---|---|
| | | **Expression level (ng/mL)** | **Activity (IU/mL)** | **Specific activity (IU/mg)** | |
| *G1* | *scF1* | 268 | 0.56 | 2090 | 0.88 |
| *G2* | *scF2* | 103 | 0.46 | 4437 | NA |
| *G3* | *scF3* | 143 | 1.01 | 7082 | NA |
| *G4* | *scF4* | 677 | 5.22 | 7714 | 1.05 |
| *G6* | *scF5* | 980 | 6.57 | 6704 | NA |
| *G4_flex* | *scF6*** | 801 | 5.64 | 7041 | 1.12 |
| *G6_opt* | *scF7** | 619 | 4.83 | 7803 | NA |
| - | *Advate* | | | 6000-7000 | 1.13 |

As a result of expression, it was found that the scFVIII expression level in the culture fluid increased as the length of the B domain increased, and the expression levels of scF4, scF5, scF6, and scF7 having the B domain of 198 amino acids or more were significantly increased. In addition, scF3, scF4, scF5, scF6, and scF7, which had the B domain of 92 amino acids or more in length, showed the specific activities similar to that of the full length FVIII in a recombinant form (Advate®, Baxalta product) used as a control for comparison, and also showed APTT/CS ratios similar to the ADVATE® used as a control. On the other hand, scF1, scF2 and scF3 of the above Example, which had the B domain shorter than the 128 amino acid protein sequence, showed significantly lower expression levels and scF1 and scF2 of the above Example, which had B domain shorter than the 84 amino acid protein sequence showed significantly lower specific activities than the full length FVIII, Advate®, used as a control for comparison.

The single-chain FVIII is activated by thrombin cleavage at the site containing the B domain connecting the heavy and light chains in the activation step. When the length of the B domain, which is the linkage site between the heavy chain and the light chain, is short, it is considered that single-chain FVIII is not normally converted to an active form FVIII due to the structural restriction from the short linkage, which can affect its activity. However, in the case of two-chain FVIII, it is considered that there is no such structural restriction, and thus thrombin-induced activation is smooth regardless of the length of the B domain, which is evident from the finding that the activity of the two-chain FVIII in which pre-existing B domain had been completely removed shows an activity similar to the full length FVIII. In the present invention, through unexpected efforts, the length and sequence of the B domain of the single-chain FVIII were selected so as not to affect thrombin activation, and the thrombin activation site between the heavy and light chains (1689-1690 and 740-741; based on the sequence of SEQ ID NO: 1) was activated in a way similar to the natural-type (i.e., wild type) FVIII, to obtain the single-chain FVIII showing the specific activity similar to the full length FVIII used for comparison.

### Example 1-3. Analysis of expression pattern of constructed scFVIII

The stability of the single-chain FVIII constructed herein in the culture was confirmed by Western blot analysis.

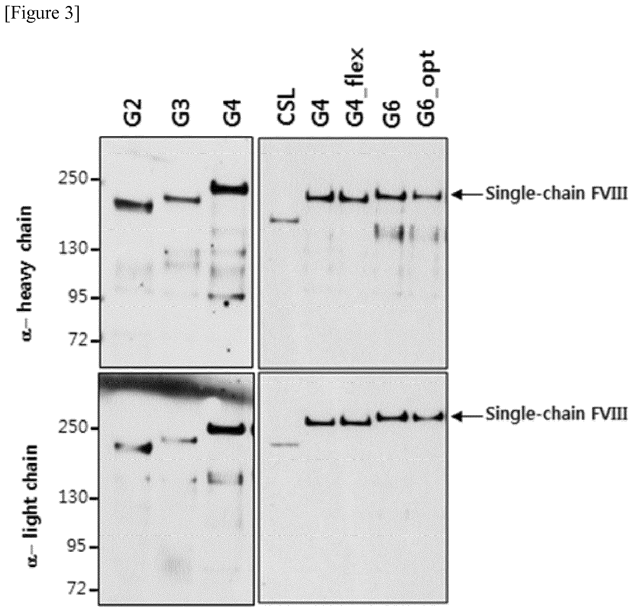

For this, the culture solution and the LDS sample buffer were mixed at a ratio of 3:1, which was mixed with 4-12% bis-tris (BT) gel to prepare a sample, and 30 µL of the sample was loaded and run at 150 volts for about 1 hour. After the transferring the gel which completed the running to the Nitrocellulose (NC) membrane, the gel was left in a blocking buffer (Thermo Scientific) for 1 hour. The primary antibodies recognizing the heavy chain (GMA-012, Green Mountain Antibodies) and the light chain (ab41188, Abcam) of FVIII were prepared by dilution in the TBST buffer and then added to the NC membrane which completed blocking, and reacted for 1 hour. After washing the membrane three times with the TBST buffer at an interval of 5 minutes, secondary antibodies (Goat anti-mouse IgG-HRP conjugate) diluted in the TBST buffer were reacted with the NC membrane for 10 minutes. And then the membrane was washed 5 times at an interval of 5 minutes using the TBST buffer. After scattering the ECL Prime Western Blotting Detection Reagent to the NC membrane for the membrane to form colors, the membrane was developed in Hyperfilm ECL (GE healthcare) in a dark room. As a result, as shown in Fig. 3, it was found that all of the FVIIIs produced in a single-chain form were expressed in a single-chain form. In addition, it was found that the single-chain FVIII secreted to the culture medium which had shorter length of the B domain showed less decomposition in the culture medium and more stability. The scF1, scF2, and scF3, in which the B domains are relatively short, showed superior stability to scF4, scF5, scF6, and scF7, but showed a relatively low expression level or low specific activity.

In the present invention, the single-chain FVIIIs having the B domains of various length derived from natural-type two-chain FVIII was constructed and their properties were analyzed. The effects of the length of B domain on the expression level, specific activity and stability were evaluated, thereby preparing the recombinant single-chain FVIII having the activity characteristics similar to the natural-type FVIII and showing high expression level.

### Example 2. Pegylation of scFVIII

### Selection of pegylization position

As the sequence and length of the B domain in the single-chain FVIII including a portion of the B domain affects the activity and characteristics of the recombinant blood coagulation FVIII by affecting the activity characteristics of FVIII, in this Example, the pegylation positions were selected based on the scFVIII prepared in Example 1 comprising the B domain with a length and sequence capable of retaining the activity characteristics of a natural-type two-chain FVIII in order to provide *in vivo* persistence.

In order to optimize the pegylation reaction efficiency in the B domain, pegylation was conducted at various positions of the B domain. As the pegylation targets the surface residues of the protein, a three-dimensional structure of the B domain is required to select the pegylation position, but the structure of the B domain is not known, making it difficult to select the pegylation position. The pegylation position of the B domain selected in the present invention was selectively pegylated around the sugar chain of the B domain. That is, the sugar chain is located outside the protein structure, and the residues around the sugar chain in the B domain are likely to be always directing outward, which is considered to be advantageous for pegylation. However, there was a problem that the closer to the sugar chain, the more difficult pegylation can be owing to the steric hindrance by the sugar chain's interfere with the access of PEG. In the present invention, the residues near the sugar chain positioned at an appropriate distance from the sugar chain, which are directing outward in the protein structure of the B domain and free from steric hindrance by the sugar chain thereby ensuring the high pegylation efficiency were selected through unexpected efforts.

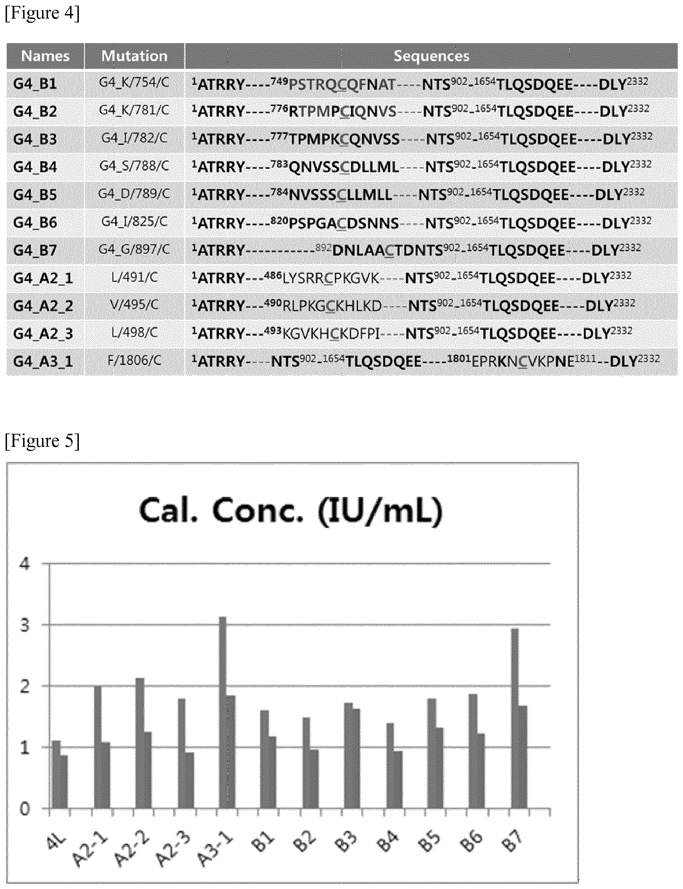

In this Example, as shown in Table 3 and Fig. 4, recombinant scFVIIIs to which cysteine was introduced in various positions near the sugar chain of the B domain based on the G4 constructed in Example 1, that is, between the +6 and -6 residues based on the N-sugar chain, were produced, and pegylation was selectively conducted to the introduced cysteine. Such pegylation has been described with regard to the G4 and single-chain FVIII, but can also be applied to pegylation of other scFVIII including a sugar chain constructed herein with the same logic.

### Example 2-1. Construction of scFVIII expression vector in which specific site is substituted with cysteine

The gene was synthesized by GeneArt to include a sequence from the *BamHI* site (GGATCC) present in the A2 domain of the FVIII heavy chain including the cysteine substitution site to the light chain and the *Pac*I enzyme cleavage site. Then, the pre-existing corresponding FVIII region was removed from the pcDSW-scFVIII G4 expression vector produced in Example 1-1 using the enzyme *BamH*I*]Pac*I*,* and the synthesized gene was cloned into the *BamH*I/*Pac*I site to construct the pcDSW-scFVIII G4 (B1, B2, B3, B4, B5, B6, B7, A3-1 or 4L) expression vector substituted with cysteine. The accuracy of the expression vector construction was confirmed by sequence analysis. In addition, regarding the scFVIII G4 (A2-1, A2-2, or A2-3), the gene was synthesized by GeneArt to include a sequence from the *Asis*I enzyme cleavage site present in the A1 domain of the FVIII heavy chain including the cysteine substitution site to the *Kpn*I site (GGTACC) present in the A2 domain of the FVIII heavy chain. Then, the pre-existing corresponding FVIII region was removed from the expression vector pcDSW-scFVIII G4 produced in the Example 1-1 using the enzyme *Asis*I/*Kpn*I*,* and the synthesized gene was cloned into the *Asis*I/*Kpn*I site to construct pcDSW-scFVIII G4 (A2-1, A2-2, or A2-3) expression vectors substituted with cysteine. The accuracy of the expression vector construction was confirmed by sequence analysis.

Transient expression was conducted by the same method as in Example 1-2 using the constructed mscFVIII expression vector into which cysteine was introduced. Each of the expression levels of the cysteine-introduced FVIIIs is shown in Table 3 below. As shown in Table 3, the scFVIII mutant into which cysteine is introduced around the glycan chain of the B domain was found to be expressed normally in the culture solution. Its expression level was somewhat lower than scFVIII prior to the introduction of cysteine, suggesting that the cysteine introduced into the scFVIII affects the expression process and the stability of scFVIII in the culture solution.

### Example 2-2. Pegylation

For the screening of peglyation positions, pegylation of cysteine-introduced scFVIII mutants as in Example 2-1 was conducted as follows. 50 ml of the cell culture solution was concentrated by 20-fold with 5 kDa MWCO (molecular weight cutoff), and 5 M NaCl conc solution was added thereto to the final concentration of 1 M NaCl. 0.1 mL volume of V8 select resin (GE) equilibrated with the equilibrium buffer (20 mM Histidine, 500 mM NaCl, 0.1% (w/v) tween® 80, pH 7.0) and a concentrated sample were thoroughly stirred together, washed with the equilibration buffer, and eluted with the elution buffer (20 mM Histidine, 500 mM NaCl, 0.1% (w/v) tween® 80, pH 7.0, 50% (v/v) propylene glycol). The eluted samples were exchanged with the pegylation buffer (20 mM Histidine, 500 mM NaCl, 0.1% (w/v) tween® 80, pH 7.0, 5% (w/v) sucrose) using a 5 kDa MWCO membrane, and then the TCEP was treated at 0.2 mM in final and reacted at 4°C for 1 hour. Then, in order to remove TCEP of the reactant, the sample was desalted with the pegylation buffer (20 mM Histidine, 500 mM NaCl, 0.1% (w/v) tween® 80, pH 7.0, 5% (w/v) sucrose), and then the maleimide PEG 40 kDa (NOF) was added at a molar ratio of 1:20 (scFVIII: maleimide PEG 40 kDa) and the resultant was reacted at 4°C for 1 hour.

In the scFVIII mutants into which cysteine was introduced, cysteine is masked by cysteine or glutathione with a disulfide bond when expressed through cell culture. Thus, the masking cysteine and glutathione should be removed for pegylation. In the present invention, TCEP (tris (2-carboxyethyl) phosphine) was added to remove cysteine or glutathione which is masking the free thiol group of cysteine, and the TCEP was removed through a desalting column (PD10). And then, the resultant was treated with maleimide-PEG (40 kDa) and pegylated.

SDS PAGE was conducted for the pegylated reactants to analyze the degrees of pegylation.

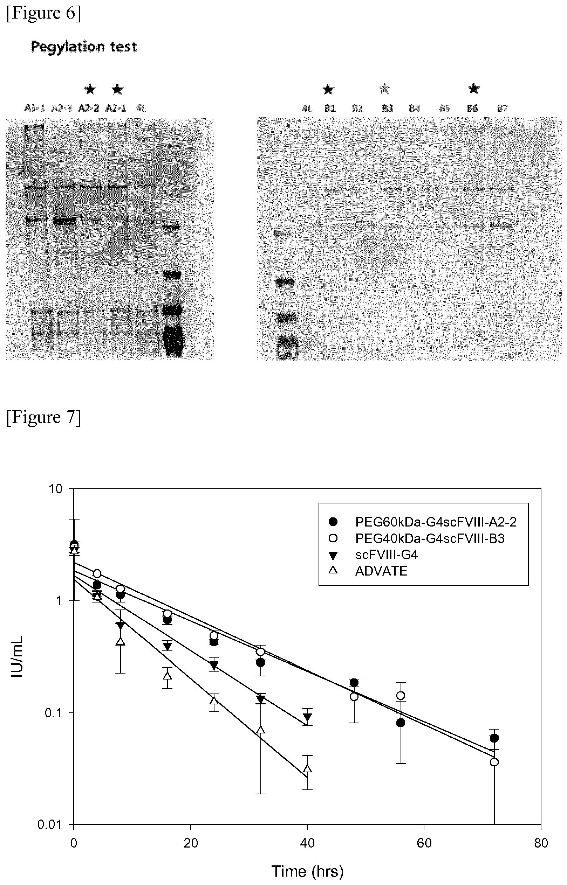

The results are shown in Fig. 6. It was found that the G4scFVIII mutant into which cysteine is introduced was expressed in an active form in the culture solution and the pegylation was proceeded easily. There was a difference in the expression level and pegylation rate depending on the introduction site of cysteine, which is considered to be due to the difference in the stability of FVIII and pegylation reaction depending on the site of introduced cysteine.

**[Table 3] Expression levels and pegylation rates of the mutants in which the scFVIII conjugation site is substituted with cys.**

| **ScFVIII mutant** | **Cys introduction position*** | | **Expression level (IU/mL)** | **Protein stability (western)** | **Pegylation efficiency** | **Nucleic acid sequence** | **Amino acid sequence** |
|---|---|---|---|---|---|---|---|
| | **Position** | **Domain** | | | | | |
| **G4_A2_1** | L/491/C | A2 | 1.53 | + + + | + + | **SEQ ID NO**: **17** | **33** |
| **G4_A2_2** | V/495/C | A2 | 1,68 | + + + | + + | **SEQ ID NO**: **18** | **34** |
| **G4_A2_3** | L/498/C | A2 | 1.35 | + + + | + | **SEQ ID NO**: 1 **9** | **35** |
| **G4_A3_1** | F/1806/C | A3 | 2.47 | + + + | + | **SEQ ID NO**: **20** | **36** |
| **G4_B1** | K/754/C | B | 2.94 | + + + | + + | **SEQ ID NO: 21** | **37** |
| **G4_1D** | D/765/C | B | 0.97 | + | - | **SEQ ID NO**: **22** | **38** |
| **G4_2D** | D/770/C | B | 0.51 | + | - | **SEQ ID NO**: **23** | **39** |
| **G4_3R** | R/776/C | B | 0.41 | + | - | **SEQ ID NO**: **24** | **40** |
| **G4_B2** | K/781/C | B | 6.33 | + + + | + + | **SEQ ID NO**: **25** | **41** |
| **G4**_**B3** | I/782/C | B | 9.07 | + + + | + + | **SEQ ID NO**: **26** | **42** |
| **G4_B4** | S/788/C | B | 4,74 | + + + | + + | **SEQ ID NO**: **27** | **43** |
| **G4_B5** | D/789/C | B | 5.07 | + + + | + + | **SEQ ID NO**: **28** | **44** |
| **G4_4L** | L/790/C | B | 4.13 | + + + | + + | **SEQ ID NO**: **29** | **45** |
| **G4_B6** | I/825/C | B | 6.64 | + + + | + + | **SEQ ID NO**: **30** | **46** |
| **G4_B7** | G/897/C | B | 10.9 | + + + | + | **SEQ ID NO**: **31** | **47** |
| **G4_1I1D** | D/765/C,I/1 668/C | Band A3 | 0.13 | - | - | **SEQ ID NO: 32** | **48** |

| | | | | | | | |
|---|---|---|---|---|---|---|---|
| *: The residues in Table 3 above are based on the sequence of SEQ ID NO: 1 | | | | | | | |

### Example 3. Production of pegylated scFVIII

In this Example, the scFVIIIs pegylated at the sites selected in Example 2 were produced as follows. In addition, the pegylated scFVIIIs were produced and their activities as long-acting type blood coagulation FVIII *in vivo* and the effect of pegylation on the activity of blood coagulation FVIII were evaluated. Site-specific conjugates of G4 and B3, which is scFVIII in which the isoleucine at 782 in the B domain of the scFVIII was substituted with cysteine, were produced and their activities were measured by the CS and OS (APTT) methods, and compared with ADVATE®, arecombinant blood coagulation FVIII of a two-chain form.

### Example 3-1. Cell culture

The scFVIII G4 (B3) and scFVIII G4 (A2_1) to which cysteine was introduced and constructed in Example 2-1 were expressed and cultured in each cell. Specifically, for expression, the expression vector constructed in Example 2-1 was transfected into Expi293F™ cells using the Expi293F™ Expression System Kit (Thermofisher, Catalog Number A14635) according to the manufacturer's method. In summary, cell numbers and viability were measured at the time of transformation and transformation was carried out when the cell viability was 95% or higher. The Expi293 culture medium was added to a 125 mL flask to obtain the concentration of 7.5 X 10⁷ cells, which was adjusted to 25.5 mL. 30 ug of the expression vector constructed in Example 2-2 was mixed with Opti-MEM to a total volume of 1500 ul. 80 ul of the transfection reagent was mixed with Opti-MEM to a total volume of 1500 ul and incubated at room temperature for 5 minutes. Five minutes later, the Opti-MEM containing the transfection reagent was added to the Opti-MEM containing DNA and the resultant was gently mixed. The reaction was carried out at room temperature for 20 to 30 minutes. 3 mL of DNA: transfection reagent complex was added dropwise to 125 mL flask of Expi293F™ cells (Total volume: 28.5 mL) prepared beforehand and the cells were incubated at 37°C in a 5% CO₂ shaking incubator at 125 rpm. After 16 ∼ 20 hours, 150 µl and 1.5 ml of Enhancer 1 and Enhancer 2 were added thereto respectively, and the cells were cultured in a shaking incubator of 34°C and 5% CO₂ at 125 rpm. On the second day of culture, all cells were centrifuged and the pre-existing culture medium was completely removed. Cells were completely resuspended in 30 mL of new culture medium and cultured at 34°C in a 5% CO₂ shaking incubator at 125 rpm. On the third day from the transfection, scFVIII-expressing cells were all centrifuged and the culture solution was recovered. The cells remaining after the recovery of the medium were resuspended in a new culture medium of the same volume and cultured at 34°C for 1 day. This process was repeated until the 5th day of transfection, and the culture solutions on the 3rd, 4th, and 5th days from the transfection were recovered to conduct the activity analysis and purification of cysteine-introduced scFVIII.

### Example 3-2. Pegylation and purification

The pegylation and purification process is composed of five steps. The culture solutions on the 3rd, 4th, and 5th days of culture medium in Example 3-1 were pooled to carry out purification.

In the first step, the process of separating and purifying FVIII from the culture medium was conducted using the VIIISelect resin developed by GE Inc. for purification. The VIIISelect resin was packed into a column and the column was washed with 2% citric acid. It was equilibrated by flowing an equilibrium buffer (20 mM Histidine, 5 mM CaCl₂, 1 M NaCl, 0.02% tween® 80, pH 7.0). 5 M NaCl buffer was added to the culture solution to a final concentration of 1 M NaCl, which was then titrated to pH 7.0, and loaded onto the column. After loading the culture solution, it was equilibrated by flowing the equilibrium buffer until the UV fell to the baseline. ScFVIII was rapidly eluted by flowing an elution buffer (20 mM Histidine, 5 mM CaCl₂, 0.9 M Arginine, 45% propylene glycol, 0.02% tween® 80, pH 6.5).

And, in the second step, a process of rapidly removing the propylene glycol mixed in the eluent in the first step was conducted using SP fast flow resin from GE Inc. After the SP fast flow resin was packed into a column, the column was washed by flowing a washing buffer (0.5 M NaOH, 1 M NaCl). It was equilibrated by flowing an equilibrium buffer (20 mM Histidine, 5 mM CaCl₂, 0.02% tween® 80, pH 7.0). The eluent in the VIIISelect process was diluted 10-fold by an equilibration buffer, titrated to pH 7.0, and loaded onto the column. After the loading, it was equilibrated by flowing an equilibrium buffer until the UV fell to the baseline. ScFVIII was rapidly eluted by flowing an elution buffer (20 mM Histidine, 5 mM CaCl₂, 400 mM NaCl, 0.02% tween® 80, pH 6.5).

In the third step, a process of conjugating PEG to the purified scFVIII was conducted. TCEP was added to the purified scFVIII solution to a final concentration of 0.1 mM, and allowed to stand at 4°C for 1 hour to reduce the inserted cysteine. Residual TCEP was removed using a PD-10 column (GE healthcare) equilibrated with pegylated buffer (20 mM Histidine, 5 mM CaCl₂, 200 mM NaCl, 0.02% tween® 80, pH 7.0). It was allowed to stand at 4°C for 2 hours to oxidize the disulfide bond of the reduced FVIII itself. For the PEG conjugation to the scFVIII G4 (B3), a 40-kDa branched-type methoxy maleimide PEG solution (50 mg/mL) dissolved in DMSO at the PEG to protein ratio of 1: 20 was added, and then the resultant was allowed to stand at 4°C for 12-16 hours. The scFVIII G4 (A2_1) PEG conjunction was carried out using 60 kDa of methoxy maleimide PEG in a branched form.

And, in the fourth step, a process of removing scFVIII without PEG and scFVIII with two or more PEGs, which are impurities formed during the pegylation process, was conducted using SP fast flow resin of GE Inc. The SP fast flow resin was packed into the column and washed with the washing buffer (0.5 M NaOH, 1 M NaCl) to wash the column. The equilibration buffer (20 mM Histidine, 5 mM CaCl₂, 0.02% tween®80, pH 7.0) was added to reach an equilibrium. The pegylated FVIII was diluted by 10-fold with an equilibration buffer, titrated to pH 7.0, and loaded onto the column. After loading, the equilibrium buffer was flowed until UV fell to the baseline, to reach an equilibrium. The PEG-scFVIII was selectively eluted while the NaCl concentration of the elution buffer (20 mM Histidine, 5 mM CaCl₂, 50-400 mM NaCl, 0.02% tween® 80, pH 6.5) was increased stepwise.

Finally, in the fifth step, the PEG-scFVIII eluted in the fourth step was concentrated. To this end, the PEG-scFVIII was concentrated to 25 IU/mL using amicon 30 kDa of Millipore Co.

### Example 3-3. Activity measurement

The activities of the purified pegylated scFVIIIs (PEG-scFVIII-B3, and PEG-scFVIII-A2-2) used in Example 3-2 were measured by the CS and APTT methods. The CS test was carried out by the same method as described in Example 1-2, and the APTT test was carried out by modifying the method described in Example 1-2. It is known that, when measuring the activity of the pegylated FVIII by APTT method, activity values lower than original values are obtained upon analysis if APTT-SP of colloidal silica series is used as an activator, whereas normal activity is measured if synthAFax (IL) of the ellagic acid series is used (Gu. J. M. et al., 2014. Evaluation of the activated partial thromboplastin time assay for clinical monitoring of PEGylated recombinant Factor VIII (BAY 94-9027) for haemophilia A. Haemophilia, 20, 593-600). SynthAFax was used as an activator in the APTT test in consideration of the property of the pegylated scFVIII. The same method as in Example 1-2 was used for the other test methods. The results are shown in Table 4.

**[Table 4]**

| | **Activity IU/mL** | | **APTT/CS ratio** |
|---|---|---|---|
| | **APTT (clotting assay), synthAFax** | **CS (chromogenic assay)** | |
| PEG-sc FVIII -B3 (40K PEG) | 19.34 | 20.21 | 0.96 |
| PEG-scFVIII-A2-2 (60K PEG) | 18.62 | 18.04 | 1.03 |
| Advate | 25.87 | 23.77 | 1.09 |
| Xyntha | 25.32 | 33.88 | 0.75 |

As a result of the experiment, it was found that the APTT/CS ratios (APTT/CS) of PEG-scFVIII-B3, PEG-scFVIII-A2-2 were similar to that of ADVATE®, which is a two-chain FVIII containing the B domain of full length used as a comparative substance, but were different from that of Xyntha, which is a two-chain FVIII from which the B domain was removed. Considering that the activities of the natural-type FVIII are almost the same in CS and APTT, the result indicated that the scFVIII protein produced by the present invention did not show changes in the activity characteristics of blood coagulation FVIII due to single-chain formation and pegylation. Xyntha is a recombinant FVIII from which the B domain is completely removed, and in this Example, its blood coagulation activity (APTT) was decreased as compared with that in CS, which is consistent with the previous report that the coagulation activity is decreased when the B domain is completely removed. Considering that PEG-scFVIII-B3 and PEG-scFVIII-A2-2 maintain blood coagulation activity even after single-chain formation and pegylation, these two substances according to the present invention are considered to have advantages as hemophilia therapeutic agents.

### Example 4. Animal PK Test

The half-life of the pegylated scFVIII was compared with ADVATE®, a comparison substance, in the Hemophilia A mouse model (B6; 129S4-F8tm1Kaz/J (FVIII-knock-out)) of 11-12 weeks of age, which was distributed from Pennsylvania University, bred, and confirmed by genotyping. The test substances were G4, which is scFVIII, PEG40kDa-G4scFVIII-B3, which was position-specifically pegylated with 40 kDa branched PEG maleimide after the 782 isoleucine of the B domain of G4 was substituted with cysteine (see Example 3), PEG60 kda-G4scFVIII-A2-2, which was position-specific pegylated with a 60 kDa branched PEG maleimide after the 495 valine in the A2 domain of the G4 were substituted with cysteine, and Advate®, which is a two-chain recombinant FVIII.

Test animals used in drug pharmacokinetics were hemophilia mice (females), and each sample was administered to 3 animals into the tails veins at a level of 125 IU/kg. Blood samples were collected from the orbit at each time point after the administration (5 min, 4 hours, 8 hours, 16 hours, 24 hours, 32 hours, 40 hours, 48 hours, 56 hours, and 72 hours), which were treated with an anticoagulant (3.2% sodium citrate) by 10% and centrifuged to remove plasma. The activity of FVIII present in the plasma was measured using the CS assay reagent as described in Example 1-2. The profile of FVIII activity average in plasma collected from the mice in each sample group at each time is shown in Fig. 7.

As for the PK parameter analysis, the mean and standard deviation of FVIII CS activity measurement values in plasma were calculated by the NCA (Non Compartmental Analysis) method using the WinNonlin software, and the result is shown in Table 5. The half-lives of the drugs were found to be in the following order: B3 ≒ A2-2 > G4 > Advate® (Table 5). When evaluated based on the mean data, the half-lives of the drugs B3, A2-2, and G4 were respectively increased by about 1.9-fold, about 1.8-fold, and about 1.2-fold, based on Advate® (Table 5).

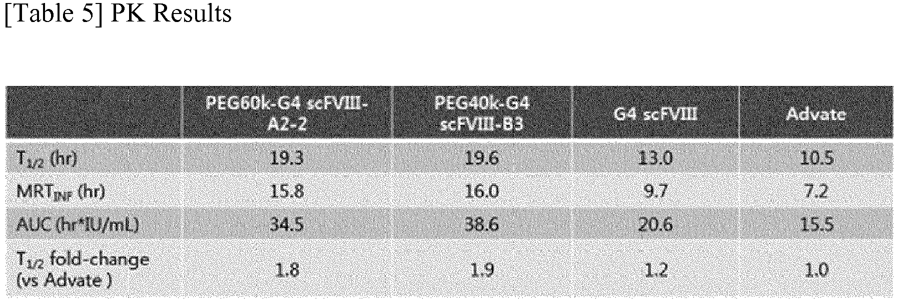

### Example 5. Animal PD test (Long-term)

The coagulation effect regarding tail bleeding in mice was measured as follows.

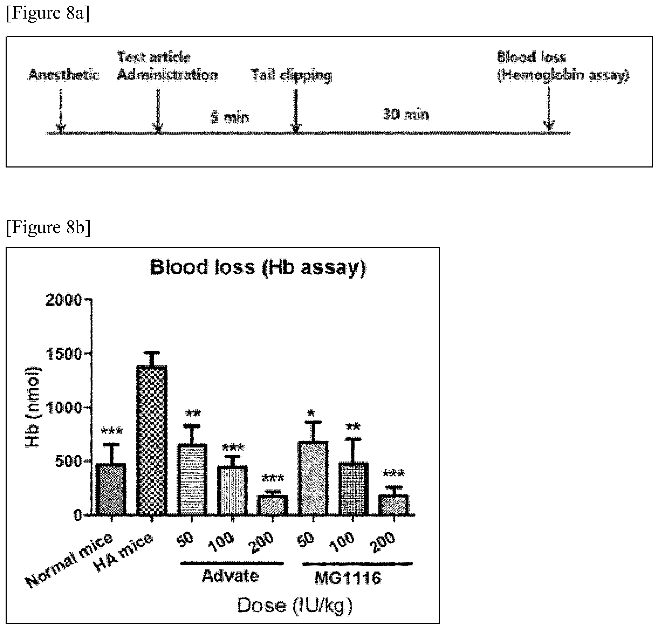

The experiment outline is shown in Fig. 8A. Specifically, male C57BL/6 mice (provided by Orient Biotech) at 11-12 weeks of age and Hemophilia A mice (B6; 129S4-F8^{tm1Kaz}/J (FVIII-knock-out), FVIII KO mice) which was distributed from Pennsylvania University, bred, and confirmed by genotyping were used for the test. C57BL/6 mice were subjected to the acclimation process for 7 days. On the day of the test, the saline was filled in a 15 mL conical tube up to 14 mL (x1/rat), and the tube was soaked in a heated water bath to adjust the temperature of the saline to 37°C. The weights of the mice were measured and recorded before administration of the test substance. Entobar (pentobarbital sodium) was administered intraperitoneally at a concentration of 60 mg/kg to anesthetize the mice. After the doses of the test substance based on the weights of the mice, which were 19-25 g, were calculated, the test substance was administered into the jugular vein. 5 minutes after the administration, the tails of the mice were cut at the point which was 4 mm from the end. About 2 cm of mouse tail cut was immediately soaked in conical tubes containing saline and the blood was collected for 30 minutes.

The test substance was PEG-scFVIII-B3 prepared in Example 3, which was compared with Advate®. The doses (doses and dose volumes) and the distribution of mice included in each test substance group are shown in Table 6 below.

**[Table 6]**

| **Test group** | **Animal species** | **Test substance** | **Administration concentration (IU/kg)** | **Administration dose (mL/kg)** | **Number of animals** |
|---|---|---|---|---|---|
| G1 | C57BL/6 | PBS | 0 | 5 | 13 |
| G2 | FVIII KO mice | PBS | 0 | 5 | 12 |
| G3 | | Advate® | 50 | 5 | 10 |
| G4 | | | 100 | 5 | 10 |
| G5 | | | 200 | 5 | 10 |
| G6 | | 40kDaPEG-G4scFVIII-B3 | 50 | 5 | 5 |
| G7 | | | 100 | 5 | 6 |
| G8 | | | 200 | 5 | 4 |

The blood collected from each mouse for 30 minutes was centrifuged at 1500 x g for 5 minutes and the supernatant was removed, then, tertiary distilled water was added to a conical tube up to 10 mL using a 10mL pipet, and then, the blood was subjected to complete hemolysis using a vortex. The blood loss measurement was conducted according to the manual provided by Hemoglobin assay kit (Sigma, MAK115-1KT). In summary, 0.9 ml of tertiary distilled water and 0.1 ml of a hemolysis sample were put into a 1.5 mL eppendorf tube and mixed well for 10-fold dilution. 50 µl of tertiary distilled water and 50 µl of a calibrator were put into a 96 well plate (duplication), and 200 µl of tertiary distilled water was added on top of it. 50 µl of each mouse hemolysis sample was put into the well (duplication) and 200 µl of the reagent was added on top of it. The 96 well plate was lightly tapped so that the inputted substances could be mixed well, and the absorbance was measured at 400 nm using a Multimode plate reader.

The absorbance values were statistically compared with the blood loss values (Hb nmol)using the One way ANOVA with Dunnett's multiple comparison test of GraphPad Prism ver 5.01. A p-value greater than 0.05 was considered to be a statistically not significant difference, and a p-value less than 0.05 was considered to be a statistically significant difference.

Test results indicated that the groups administered with 40kDaPEG-G4scFVIII-B3 and Advate® 50, 100, and 200 IU/kg all showed statistically significant differences when compared with the blood loss value of the HA group. The HA mice were administered with 40kDa PEG-G4scFVIII-B3 and Advate®, and then acute tail-clipping test was conducted. As a result, it was found that both of the two test substances decreased the amount of blood losses in a concentration dependent way (see Fig. 8b).

In summary, in the present invention, in order to select the scFVIIIs which are excellent in expression, have maximum natural FVIII activities, and are stable such that its decomposition in the culture medium can be prevented, the scFVIIIs containing B domains of various lengths and sequences were prepared and expressed comparing their activities with each other and then, the scFVIIIs having useful properties as Hemophilia A type therapeutic agents were developed through confirming their stability. In addition, based on the scFVIIIs developed in the present invention, various pegylated conjugates were prepared, thereby developing FVIII pegylated conjugates with improved half-lives, and thus improved usefulness as therapeutic agents.

While the present invention has been described with respect to the above specific embodiments, it should be recognized that various modifications and changes using the basic concepts of the present application as defined in the claims which may be made by those skilled in the art also fall within the scope of the present invention.

All the technical terms that were used in the present invention, have the same meanings that are understood by a skilled person in this art, unless otherwise specified. The contents of all the publications disclosed in the present description as a reference document are incorporated in the present invention.

## Claims

1. A single-chain blood coagulation factor VIII (Factor VIII) comprising a heavy chain, a light chain and a partially-deleted B domain fragment of human Factor VIII, wherein the partially-deleted B domain fragment lacks at least 5 amino acids from residue 1648 of SEQ ID NO: 1 to the N-terminal direction and at least 5 amino acids from residue 1649 of SEQ ID NO: 1 to the C-terminal direction, so as not to comprise a cleavage site by furin protease, and contains 4 to 6 glycosylation sites.

2. The single-chain blood coagulation factor VIII of claim 1, wherein the partially-deleted B domain fragment comprises 15% to 40% of a wild type B domain sequence.

3. The single-chain blood coagulation factor VIII of claim 2, wherein the partially-deleted B domain fragment has the sequence of (i) amino acid residues 741 to 902 and 1654 to 1689; (ii) amino acid residues 741 to 965 and 1654 to 1689; or (iii) amino acid residues 741 to 902, 1637 to 1642, and 1654 to 1689, based on the sequence of SEQ ID NO: 1.

4. The single-chain blood coagulation factor VIII of claim 3, wherein the single-chain blood coagulation factor VIII has the sequence represented by SEQ ID NO: 4, SEQ ID NO: 5 or SEQ ID NO: 6, or the sequence having the sequence homology of 90% or more thereto.

5. The single-chain blood coagulation factor VIII of any one of claims 1 to 4, wherein the single-chain blood coagulation factor VIII has a specific activity of 90% or higher than that of a two-chain blood coagulation factor VIII when measured by a CS or APTT method.

6. The single-chain blood coagulation factor VIII of any one of claims 1 to 4, wherein the blood coagulation factor VIII is conjugated to a hydrophilic polymer at some amino acid residues of the A domain and/or B domain fragment;
the conjugation position of the B domain fragment is at least one selected from the group consisting of amino acid residues 754, 781,782, 788, 789, 825 and 897 based on the sequence of SEQ ID NO: 1 and the conjugation position of the A domain is at least one selected from the group consisting of amino acid residues 491, 495, 498 and 1806 based on the sequence of SEQ ID NO: 1; and
the residue of the conjugation position is substituted with cysteine for the conjugation with the hydrophilic polymer.

7. The single-chain blood coagulation factor VIII of claim 6, wherein the hydrophilic polymer comprises polyethylene glycol (PEG), polyethylene oxide, dextran, or polisialic, and the PEG is linked at the said position through acryloyl, sulfone or a maleimide.

8. The single-chain blood coagulation factor VIII of claim 5, wherein the PEG has an average molecular weight of 20 kDa or more.

9. The single-chain blood coagulation factor VIII of claim 1, which is represented by the amino acid sequence of any one of SEQ ID NOS: 2 to 7.

10. The single-chain blood coagulation factor VIII of claim 9, which has an amino acid sequence where the residue of the conjugation position in the SEQ ID NOS: 2 to 7 according to claim 6 is substituted with cysteine.

11. A nucleic acid molecule encoding the protein of claim 9 or 10.

12. The nucleic acid molecule of claim 11, wherein the nucleic acid molecule encoding the protein of claim 9 is any one of SEQ ID NOS: 10 to 15, and the nucleic acid molecule encoding the protein of claim 10 is any one of SEQ ID NOS: 17 to 32.

13. A vector comprising the nucleic acid molecule of claim 11 or 12.

14. A cell comprising the vector of claim 13.

15. A method for producing a single-chain blood coagulation factor VIII comprising:
transfecting the vector of claim 13 into a eukaryotic cell; or alternatively providing the cell of claim 14;
expressing a single-chain blood coagulation factor VIII where the free thiol group of the cysteine introduced thereinto is masked with a disulfide bond by cysteine or glutathione through culturing the cells in a culture solution;
collecting the single-chain FVIII expressed from the culture solution and treating the single-chain FVIII with a reducing agent to release the masked cysteine or glutathione; and
treating the treated culture solution with a pegylation buffer.

16. A single-chain blood coagulation factor VIII comprising a heavy chain, a light chain and a partially-deleted B domain fragment of human Factor VIII, wherein the partially-deleted B domain fragment does not comprise a cleavage site by furin protease, but comprises at least 4 glycosylation sites; and
wherein the partially-deleted B domain fragment has the sequence of (i) amino acid residues 741 to 902 and 1654 to 1689; (ii) amino acid residues 741 to 965 and 1654 to 1689; or (iii) amino acid residues 741 to 902, 1637 to 1642, and 1654 to 1689, based on the sequence of SEQ ID NO: 1.

17. A single-chain blood coagulation factor VIII comprising a heavy chain, a light chain and a partially-deleted B domain fragment of human Factor VIII, wherein the partially-deleted B domain fragment is does not comprise a cleavage site by furin protease, but comprises at least 4 glycosylation sites;
wherein the partially-deleted B domain fragment has the sequence of (i) amino acid residues 741 to 902 and 1654 to 1689; (ii) amino acid residues 741 to 965 and 1654 to 1689; or (iii) amino acid residues 741 to 902, 1637 to 1642, and 1654 to 1689, based on the sequence of SEQ ID NO: 1; and
wherein some residues of the A domain and/or B domain are pegylated; and
wherein the positions of the pegylated residues in the B domain are at least one selected from the group consisting of amino acid residues 754, 781,782, 788, 789, 825 and 897 based on the sequence of SEQ ID NO: 1; and the positions of the pegylated residues in the A domain are at least one selected from the group consisting of amino acid residues 491, 495, 498 and 1806 based on the sequence of SEQ ID NO: 1.

18. The single-chain blood coagulation factor VIII of claim 17, wherein the pegylated residue is substituted with cysteine.

19. A single-chain blood coagulation factor VIII comprising a heavy chain, a light chain and a partially-deleted B domain fragment of human Factor VIII, wherein the partially-deleted B domain fragment does not comprise a cleavage site by furin protease, but comprises at least 4 glycosylation sites;
wherein the partially-deleted B domain fragment has the sequence of (i) amino acid residues 741 to 902 and 1654 to 1689; (ii) amino acid residues 741 to 965 and 1654 to 1689; or (iii) amino acid residues 741 to 902, 1637 to 1642, and 1654 to 1689, based on the sequence of SEQ ID NO: 1;
wherein some residues of the A domain and/or B domain are pegylated;
wherein the positions of the pegylated residues in the B domain are at least one selected from the group consisting of amino acid residues 754, 781,782, 788, 789, 825 and 897 based on the sequence of SEQ ID NO: 1; and the positions of the pegylated residues in the A domain are at least one selected from the group consisting of amino acid residues 491, 495, 498 and 1806 based on the sequence of SEQ ID NO: 1; and
wherein the pegylated residue is substituted with cysteine.

20. A method for treating hemophilia comprising administering an effective amount of the single-chain blood coagulation factor VIII of any one of claims 1 to 10 or 16 to 19 to a subject in need thereof.

21. A method for blood coagulation comprising administering an effective amount of the single-chain blood coagulation factor VIII of any one of claims 1 to 10 or 16 to 19 to a subject in need thereof.

22. A composition for treating hemophilia comprising the single-chain blood coagulation factor VIII of any one of claims 1 to 10 or 16 to 19 and a pharmaceutically acceptable carrier.

23. A use of the single-chain blood coagulation factor VIII of any one of claims 1 to 10 or 16 to 19 for the treatment of hemophilia.
